(19) Europäisches Patentamt
European Patent Office
Office européen des brevets

(11) EP 0 782 989 A1

(12) **EUROPEAN PATENT APPLICATION**
published in accordance with Art. 158(3) EPC

(43) Date of publication:
**09.07.1997 Bulletin 1997/28**

(21) Application number: **96923101.8**

(22) Date of filing: **16.07.1996**

(51) Int. Cl.[6]: **C07D 209/42**, C07D 401/06, C07D 403/06, C07D 405/06, C07D 409/06, A61K 31/40, A61K 31/41, A61K 31/44, A61K 31/445, A61K 31/495, A61K 31/535

(86) International application number:
**PCT/JP96/01980**

(87) International publication number:
**WO 97/03965 (06.02.1997 Gazette 1997/07)**

(84) Designated Contracting States:
**AT BE CH DE DK ES FI FR GB GR IE IT LI LU MC NL PT SE**

(30) Priority: **18.07.1995 JP 181951/95**

(71) Applicant: **KYOWA HAKKO KOGYO CO., LTD.**
**Chiyoda-ku, Tokyo 100 (JP)**

(72) Inventors:
- **MACHII, Daisuke**
  **Shizuoka 411 (JP)**
- **TAKAI, Haruki**
  **Kanagawa 246 (JP)**
- **SUZUKI, Koji**
  **Shizuoka 411 (JP)**
- **KOSAKA, Nobuo**
  **Shizuoka 411 (JP)**
- **SATO, Soichiro**
  **Shizuoka 411 (JP)**

(74) Representative: **VOSSIUS & PARTNER**
**Siebertstrasse 4**
**81675 München (DE)**

(54) **INDOLE DERIVATIVES**

(57) The present invention relates to indole derivatives represented by formula (I):

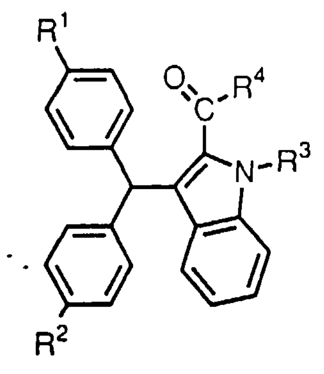

(I)

wherein $R^1$ represents hydrogen, lower alkyl, hydroxy, lower alkoxy, halogen, $-O-(CH_2)_n-OR^5$, or

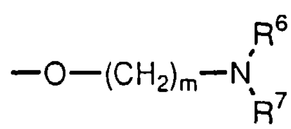

$R^2$ represents hydrogen, lower alkyl, or halogen, $R^3$ represents hydrogen, lower alkyl, or

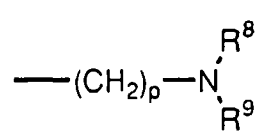

and $R^4$ represents hydroxy, lower alkoxy, substituted or unsubstituted aryloxy, or $-NR^{10}R^{11}$, with the proviso that when $R^3$ is hydrogen or lower alkyl, $R^4$ is $-NR^{10}R^{11}$, or pharmaceutically acceptable salts thereof.

EP 0 782 989 A1

## Description

Technical Field

The present invention relates to indole derivatives which are useful as therapeutic agents for osteoporosis.

Background Art

It is disclosed in Japanese Published Unexamined Patent Application No. 215461/91 that a triphenylmethane derivative having a substituent such as carbamoyl is useful as a therapeutic agent for osteoporosis. Further, it is disclosed in Japanese Published Unexamined Patent Application No. 211651/92 that an indole derivative having a phenyl group is useful as a therapeutic agent for osteoporosis. Further, an indole derivative having a carbamoyl group is disclosed in Japanese Published Unexamined Patent Application No. 76586/95. Further, an indole derivative having a benzhydryl group is disclosed in J. Med. Chem., 17, 1298 (1974).

Disclosure of the Invention

The present invention relates to indole derivatives represented by formula (I):

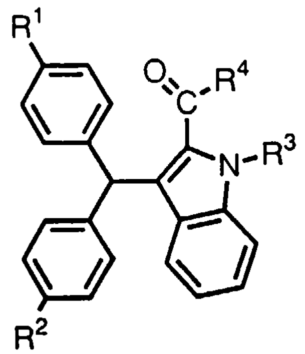

(I)

wherein $R^1$ represents hydrogen, lower alkyl, hydroxy, lower alkoxy, halogen, $-O-(CH_2)_n-OR^5$ (wherein $R^5$ represents hydrogen or lower alkyl, and n is an integer of 1 to 6), or

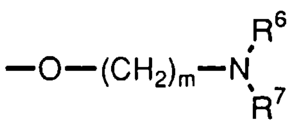

(wherein $R^6$ and $R^7$ independently represent hydrogen or lower alkyl, or $R^6$ and $R^7$ are combined together with the adjacent nitrogen atom to form a substituted or unsubstituted alicyclic heterocyclic group, and m represents an integer of 2 to 6), $R^2$ represents hydrogen, lower alkyl, or halogen, $R^3$ represents hydrogen, lower alkyl, or

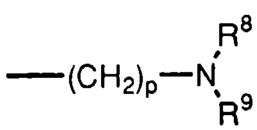

(wherein $R^8$ and $R^9$ independently represent hydrogen or lower alkyl, or $R^8$ and $R^9$ are combined together with the adjacent nitrogen atom to form a substituted or unsubstituted alicyclic heterocyclic group, and p represents an integer of 2 to 6), and $R^4$ represents hydroxy, lower alkoxy, substituted or unsubstituted aryloxy, or $-NR^{10}R^{11}$ {wherein $R^{10}$ and $R^{11}$ independently represent hydrogen, lower alkyl, alicyclic alkyl, lower alkenyl, substituted or unsubstituted aryl, a substituted or unsubstituted heterocyclic group,

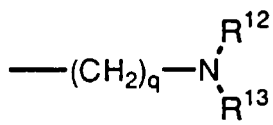

(wherein $R^{12}$ and $R^{13}$ independently represent hydrogen or lower alkyl, or $R^{12}$ and $R^{13}$ are combined together with the adjacent nitrogen atom to form a substituted or unsubstituted alicyclic heterocyclic group, and q represents an integer of 2 to 6), or $-(CH_2)_r-R^{14}$ (wherein $R^{14}$ represents substituted or unsubstituted aryl or a substituted or unsubstituted heterocyclic group, and r represents an integer of 1 to 6), or $R^{10}$ and $R^{11}$ are combined together with the adjacent nitrogen atom to form a substituted or unsubstituted alicyclic heterocyclic group), with the proviso that when $R^3$ is hydrogen or lower alkyl, $R^4$ is $-NR^{10}R^{11}$, or pharmaceutically acceptable salts thereof.

The compounds represented by formula (I) are hereinafter referred to as Compound (I). The same applies to the compounds of other formula numbers.

In the definitions of the groups in formula (I), the lower alkyl and the lower alkyl moiety of the lower alkoxy mean a straight or branched alkyl group having 1 to 8 carbon atoms such as methyl, ethyl, propyl, isopropyl, butyl, isobutyl, sec-butyl, tert-butyl, pentyl, neopentyl, hexyl, heptyl, and octyl. The alicyclic alkyl means an alicyclic alkyl group having 3 to 8 carbon atoms such as cyclopropyl, cyclobutyl, cyclopentyl, cyclohexyl, cycloheptyl, and cyclooctyl. The lower alkenyl means a straight or branched alkenyl group having 2 to 8 carbon atoms such as vinyl, allyl, methacryl, crotyl, 3-butenyl, 2-pentenyl, 4-pentenyl, 2-hexenyl, 5-hexenyl, 2-heptenyl, 6-heptenyl, 2-octenyl, and 7-octenyl. The halogen means fluorine, chlorine, bromine, and iodine. The aryl and the aryl moiety of the aryloxy mean phenyl and naphthyl. The alicyclic heterocyclic group means a group such as pyrrolidinyl, imidazolidinyl, pyrazolidinyl, piperidino, piperazinyl, homopiperazinyl, morpholino, and thiomorpholino. The heterocyclic group means a group such as an aromatic heterocyclic group (e.g., pyridyl, pyrazinyl, pyrimidinyl, pyridazinyl, quinolyl, isoquinolyl, phthalazinyl, naphthylidinyl, quinoxalinyl, thienyl, furyl, pyrrolyl, imidazolyl, pyrazolyl, triazolyl, tetrazolyl, thiazolyl, oxazolyl, indolyl, indazolyl, benzimidazolyl, purinyl), pyranyl, piperidyl, and tetrahydrofuranyl, in addition to the above-mentioned alicyclic heterocyclic groups.

The substituted alicyclic heterocyclic group, the substituted heterocyclic group, the substituted aryloxy, and the substituted aryl each has 1 to 3 independently selected substituents. Examples of the substituents are lower alkyl, hydroxy, lower alkoxy, lower alkylthio, aralkyl, carboxy, lower alkoxycarbonyl, lower alkanoyl, aroyl, halogen, nitro, amino, mono- or di(lower alkyl)amino, trifluoromethyl, oxo, substituted or unsubstituted phenyl, pyridyl, and pyrimidinyl.

In the definitions of the substituents, the lower alkyl and the lower alkyl moiety of the lower alkoxy, lower alkylthio, lower alkoxycarbonyl, and mono- or di(lower alkyl)amino have the same meaning as defined for the above-mentioned lower alkyl. The aralkyl means an aralkyl group having 7 to 13 carbon atoms such as benzyl, phenethyl, benzhydryl, and naphthylmethyl. The lower alkanoyl means an alkanoyl group having 1 to 7 carbon atoms such as formyl, acetyl, propionyl, butyryl, isobutyryl, valeryl, pivaloyl, hexanoyl, and heptanoyl. The aryl moiety of the aroyl has the same meaning as defined for the above-mentioned aryl. The halogen has the same meaning as defined for the above-mentioned halogen. The substituted phenyl has 1 to 3 independently selected substituents such as lower alkyl, hydroxy, lower alkoxy, lower alkylthio, aralkyl, carboxy, lower alkoxycarbonyl, lower alkanoyl, aroyl, halogen, nitro, amino, mono- or di(lower alkyl)amino, and trifluoromethyl. The lower alkyl, lower alkoxy, lower alkylthio, aralkyl, lower alkoxy-carbonyl, lower alkanoyl, aroyl, halogen, and mono- or di(lower alkyl)amino have the same definitions as defined above.

The pharmaceutically acceptable salts of Compound (I) include inorganic acid addition salts such as hydrochloride, sulfate, nitrate, and phosphate, organic acid addition salts such as acetate, maleate, fumarate, tartrate, citrate, lactate, glyoxylate, aspartate, methanesulfonate, ethanesulfonate, and benzenesulfonate, metal salts such as sodium salt, potassium salt, and calcium salt, ammonium salt, tetramethylammonium salt, and amine addition salts such as a salt with morpholine.

The processes for producing Compound (I) are described below.

Compound (I) can be prepared according to the following reaction step:

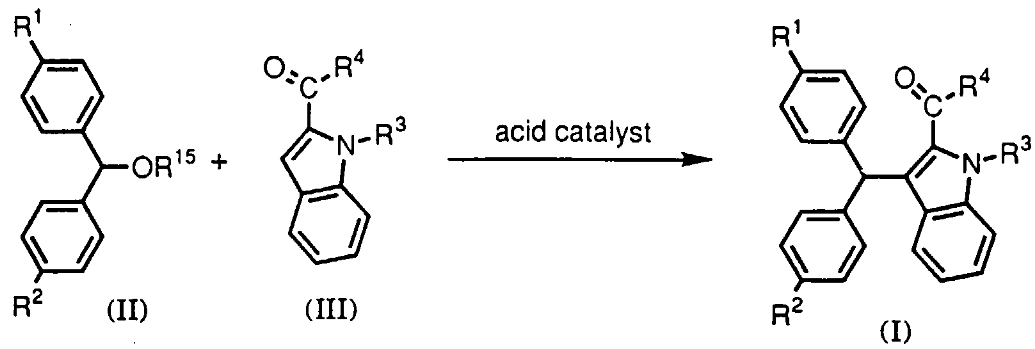


(II) (III) (I)

(In the formulae, $R^{15}$ represents hydrogen, lower alkyl, or lower alkanoyl, and $R^1$, $R^2$, $R^3$, and $R^4$ have the same meanings as defined above.)

The lower alkyl and lower alkanoyl in the definition of $R^{15}$ have the same meanings as defined above.

Compound (I) can be obtained by reacting Compound (II) with Compound (III) in the presence of 0.1 equivalence to an excess amount of an acid catalyst such as boron trifluoride-ether complex, methanesulfonic acid, paratoluenesulfonic acid, and trifluoroacetic acid, in a solvent such as methylene chloride, chloroform, ether, and tetrahydrofuran, at a temperature between -20°C and the boiling point of the employed solvent for 0.1 to 24 hours.

Compound (Ib), which is Compound (I) in which $R^1$ is a group other than hydroxy, $R^3$ is a group other than hydrogen, and $R^4$ is $-NR^{10}R^{11}$, can also be prepared from Compound (Ia), which is Compound (I) in which $R^1$ is a group other than hydroxy, $R^3$ is hydrogen, and $R^4$ is $-NR^{10}R^{11}$, according to the following reaction step:

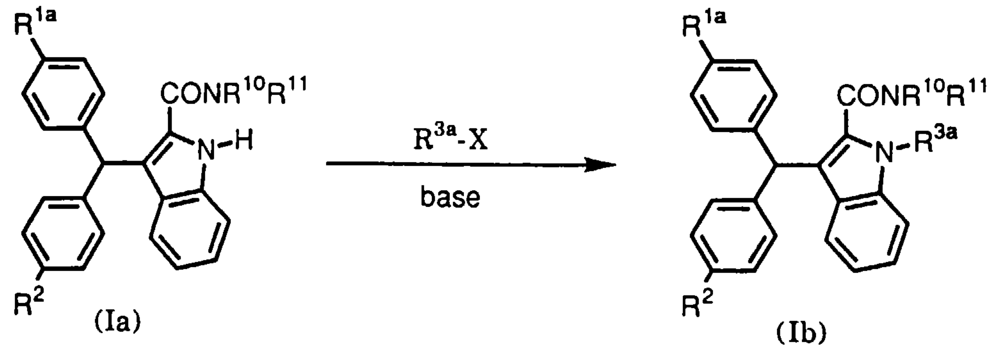


(Ia) (Ib)

(In the formulae, $R^{1a}$ represents a group other than hydroxy in the definition of $R^1$, $R^{3a}$ represents a group other than hydrogen in the definition of $R^3$, X represents chlorine, bromine, or iodine, and $R^2$, $R^{10}$, and $R^{11}$ have the same meanings as defined above.)

Compound (Ib) can be obtained by reacting Compound (Ia) with a halogenated alkyl or halogenated aminoalkyl in the presence of a base such as sodium hydride and potassium tert-butoxide, in a solvent such as a lower alcohol (e.g., methanol, ethanol), N,N-dimethylformamide, dimethyl sulfoxide, and tetrahydrofuran, at a temperature between 0°C and the boiling point of the employed solvent for 0.5 to 24 hours.

Compound (Ic), which is Compound (I) in which $R^1$ is a group other than hydroxy, $R^3$ is a group other than hydrogen and lower alkyl, and $R^4$ is lower alkoxy, and Compound (Id), which is Compound (I) in which $R^1$ is a group other than hydroxy, $R^3$ is a group other than hydrogen and lower alkyl, and $R^4$ is hydroxy, can also be prepared from Compound (IIa), which is Compound (II) in which $R^1$ is a group other than hydroxy, and Compound (IIIa), according to the following reaction steps:

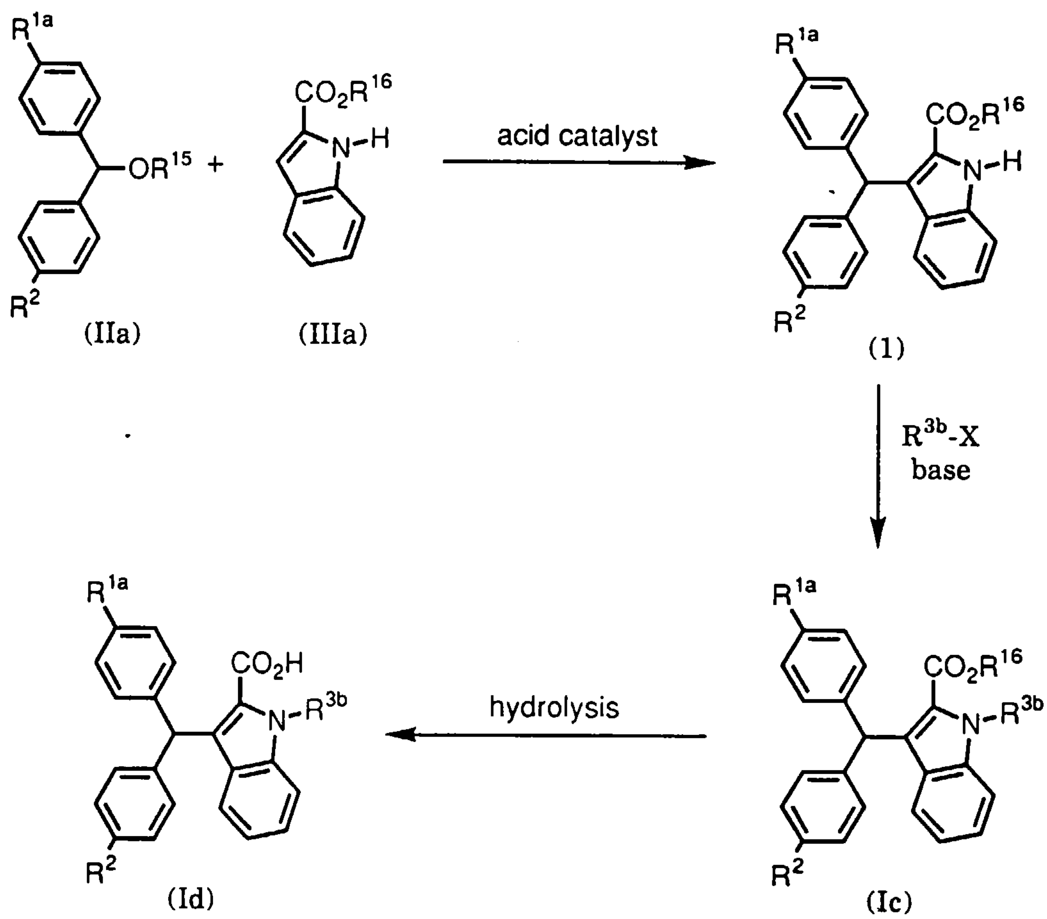


(IIa) (IIIa) (1) (Id) (Ic)

(In the formulae, $R^{3b}$ represents a group other than hydrogen and lower alkyl in the definition of $R^3$, $R^{16}$ represents lower alkyl, and X, $R^{1a}$, $R^2$, and $R^{15}$ have the same meanings as defined above.)

The lower alkyl in the definition of $R^{16}$ have the same meanings as defined above.

Compound (1) can be obtained from Compound (IIa) and Compound (IIIa) according to a method similar to that in producing Compound (I) from Compound (II) and Compound (III). Compound (Ic) can be obtained from Compound (1) according to a method similar to that in producing Compound (Ib) from Compound (Ia). Then, Compound (Id) can be obtained from Compound (Ic) under the normal hydrolytic condition, for example, treating Compound (Ic) in the presence of a base such as lithium hydroxide, sodium hydroxide, and potassium hydroxide, in a solvent such as a lower alcohol (e.g., methanol, ethanol), tetrahydrofuran, N,N-dimethylformamide, and dimethyl sulfoxide, if necessary, in the presence of water, at a temperature between room temperature and the boiling point of the employed solvent for 0.5 to 24 hours.

Compound (Ica), which is Compound (Ic) in which $R^3$ is

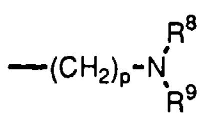

can also be prepared from Compound (1) according to the following reaction steps:

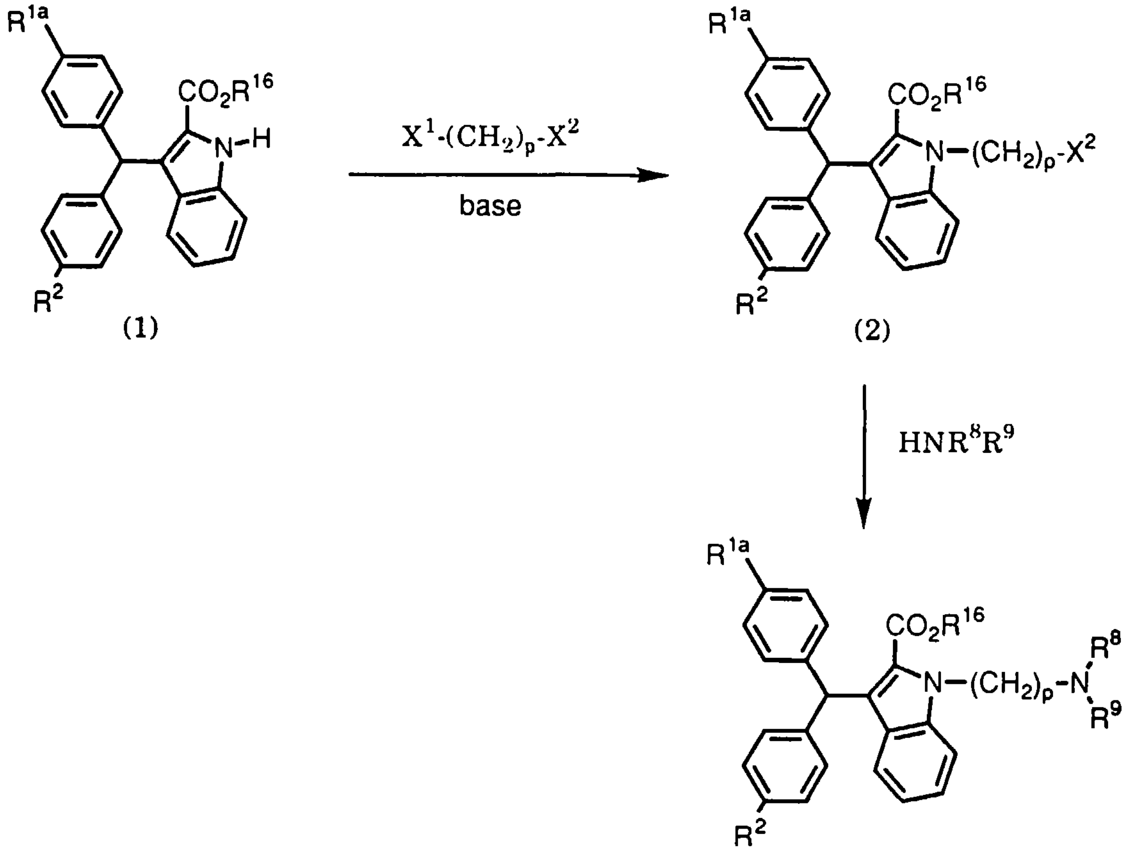


(1) (2) (Ica)

(In the formulae, $X^1$ and $X^2$ independently represent chlorine, bromine, or iodine, and $R^{1a}$, $R^2$, $R^8$, $R^9$, $R^{16}$, and p have the same meanings as defined above.)

Compound (2) can be obtained by reacting Compound (1) with α,ω-dihalogenated alkyl in the presence of a base such as sodium hydride and potassium tert-butoxide, in a solvent such as a lower alcohol (e.g., methanol, ethanol), N,N-dimethylformamide, dimethyl sulfoxide, and tetrahydrofuran, at a temperature between 0°C and the boiling point of the employed solvent for 0.5 to 24 hours. Then, Compound (Ica) can be obtained by reacting Compound (2) with one equivalence to an excess amount of ammonia, a primary amine, or a secondary amine, in a solvent such as a lower alcohol (e.g., methanol, ethanol), N,N-dimethylformamide, dimethyl sulfoxide, tetrahydrofuran, and water, or a mixed solvent thereof, if necessary, in the presence of an inorganic base such as potassium carbonate and sodium carbonate, at a temperature between 0°C and the boiling point of the employed solvent for one hour to 7 days.

Compound (Icb), which is Compound (Ic) in which $R^1$ is

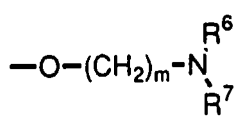

can also be prepared from Compound (3) and Compound (IIIb) according to the following reaction steps:

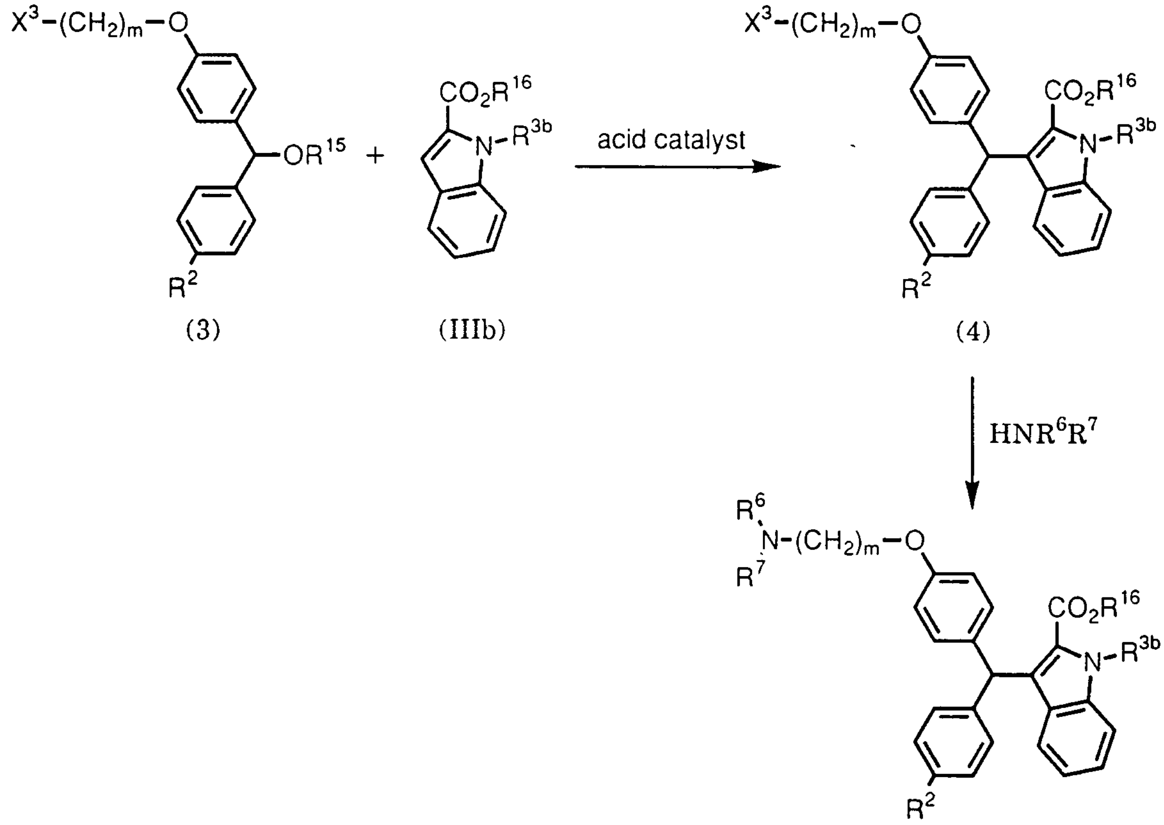


(3) (IIIb) (4) (Icb)

(In the formulae, $X^3$ represents chlorine, bromine, or iodine, and $R^2$, $R^{3b}$, $R^6$, $R^7$, $R^{15}$, $R^{16}$, and m have the same meanings as defined above.)

Compound (4) can be obtained from Compound (3) and Compound (IIIb) according to a method similar to that in producing Compound (I) from Compound (II) and Compound (III). Compound (Icb) can be obtained from Compound (4) according to a method similar to that in producing Compound (Ica) from Compound (2).

Compound (If), which is Compound (I) in which $R^4$ is $-NR^{10}R^{11}$, can also be prepared from Compound (Ie), which is Compound (I) in which $R^3$ is a group other than hydrogen and lower alkyl and $R^4$ is hydroxy, or Compound (IV), in which $R^3$ is hydrogen or lower alkyl and $R^4$ is hydroxy, according to the following reaction step:

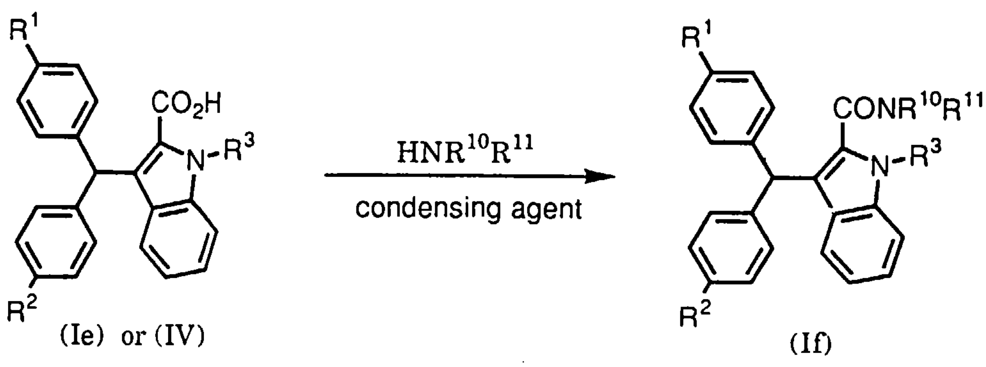


(Ie) or (IV) (If)

(In the formulae, $R^1$, $R^2$, $R^3$, $R^{10}$, and $R^{11}$ have the same meanings as defined above.)

Compound (If) can be obtained by reacting Compound (Ie) or Compound (IV) with ammonia, a primary amine, or a secondary amine in the presence of a condensing agent such as dicyclohexylcarbodiimide and 1-(3-dimethylaminopropyl)-3-ethylcarbodiimide hydrochloride, in a solvent such as methylene chloride, chloroform, and tetrahydrofuran, at

a temperature between room temperature and the boiling point of the employed solvent for 1 to 24 hours.

Compound (Ig), which is Compound (I) in which $R^1$ is hydroxy, can also be obtained by treating Compound (Ih), which is Compound (I) in which $R^1$ is methoxymethoxy, in the presence of an acid catalyst such as hydrochloric acid, sulfuric acid, and paratoluenesulfonic acid, in a solvent such as a lower alcohol (e.g., methanol, ethanol), N,N-dimethylformamide, dimethyl sulfoxide, tetrahydrofuran, and water, or a mixed solvent thereof, at a temperature between room temperature and the boiling point of the employed solvent for 0.5 to 24 hours.

Compound (Ig) can also be prepared from Compound (V) according to the following reaction step:

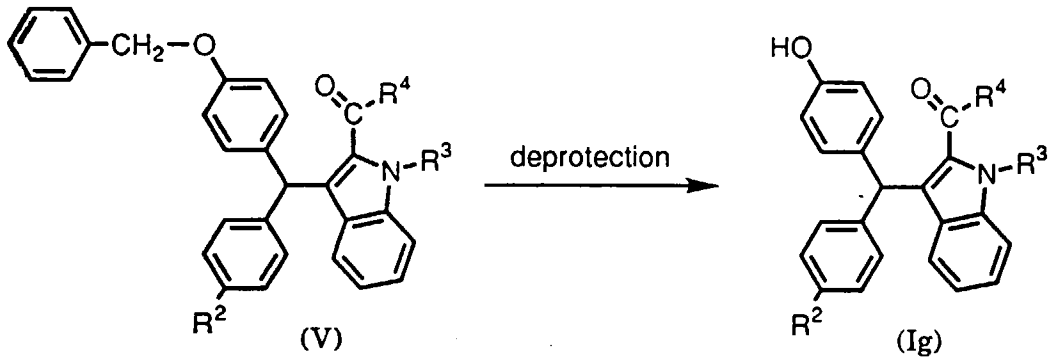


(V) (Ig)

(In the formulae, $R^2$, $R^3$, and $R^4$ have the same meanings as defined above.)

Compound (Ig) can be obtained by hydrogenating Compound (V) in the presence of a catalyst such as palladium on carbon, in a solvent such as a lower alcohol (e.g., methanol, ethanol), N,N-dimethylformamide, dimethyl sulfoxide, and tetrahydrofuran, or a mixed solvent thereof, under a hydrogen atmosphere, at a temperature between room temperature and the boiling point of the employed solvent for 0.5 to 24 hours.

Compound (IVb), which is Compound (IV) in which $R^3$ is lower alkyl, can be prepared by N-alkylating Compound (1) according to the method of producing Compound (Ib) from Compound (Ia) or a similar method thereto, followed by hydrolyzing the ester moiety according to the method of producing Compound (Id) from Compound (Ic) or a similar method thereto.

Compound (IVa), which is Compound (IV) in which $R^3$ is hydrogen, can be prepared by hydrolyzing the ester moiety according to the method of producing Compound (Id) from Compound (Ic) or a similar method thereto.

Compound (V) can be obtained from Compound (5), which is a compound in which the part of $R^1$ is replaced by a benzyloxy group in Compound (II), and Compound (III) according to the method of producing Compound (I) from Compound (II) and Compound (III) or a similar method thereto.

Compound (Iba), which is Compound (Ib) in which $R^3$ is

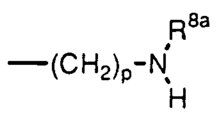

(In the formulae, $R^{8a}$ represents lower alkyl, and p has the same meaning as defined above.), can also be prepared from Compound (VI) according to the following reaction step:

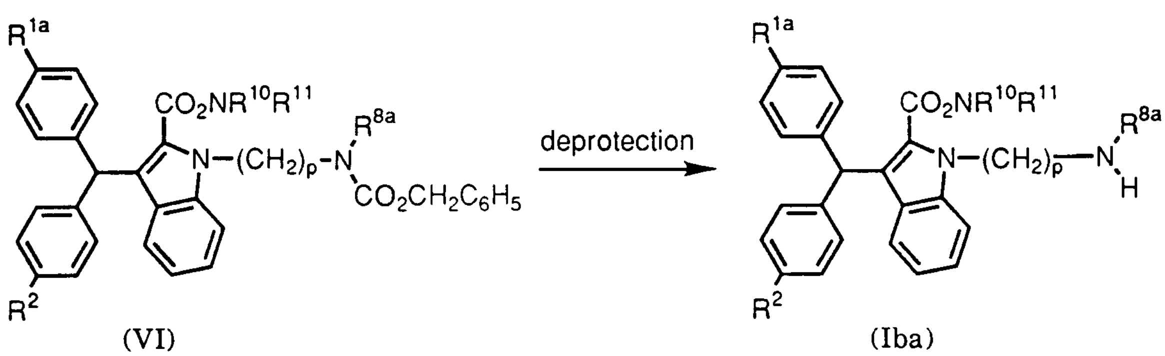


(VI) (Iba)

(In the formulae, $R^{1a}$, $R^2$, $R^{8a}$, $R^{10}$, $R^{11}$, and p have the same meanings as defined above.)

Compound (Iba) can be obtained by deprotecting a carboxybenzyl group from Compound (VI) according to a method similar to that in producing Compound (Ig) from Compound (V).

Compound (Ibb), which is Compound (Ib) in which $R^{3a}$ is

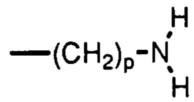

(In the formulae, p has the same meaning as defined above.), can also be prepared from Compound (VII) according to the following reaction step:

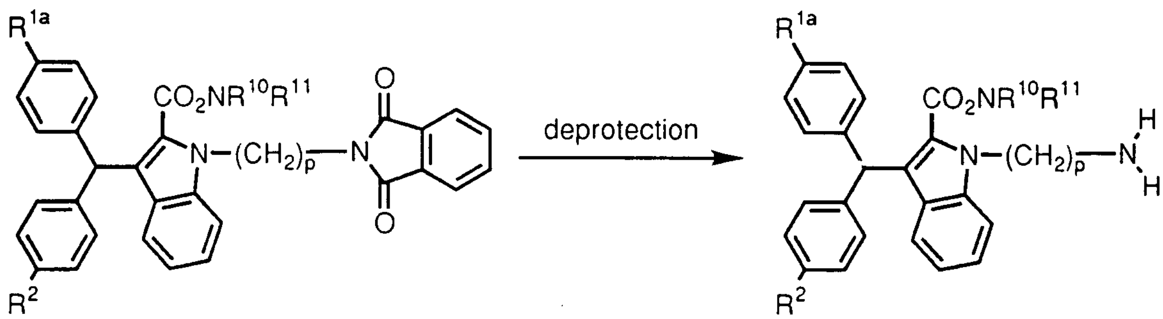


(VII) (Ibb)

(In the formulae, $R^{1a}$, $R^2$, $R^{10}$, $R^{11}$, and p have the same meanings as defined above.)

Compound (Ibb) can be obtained by deprotecting Compound (VII) by treating it with 1 equivalence to an excess amount of hydrazine hydrate in a solvent such as a lower alcohol (e.g., methanol, ethanol), N,N-dimethylformamide, dimethyl sulfoxide, and tetrahydrofuran, at a temperature between room temperature and the boiling point of the employed solvent for 0.5 to 24 hours.

Compound (VI) can be prepared from Compound (1) according to the following reaction steps:

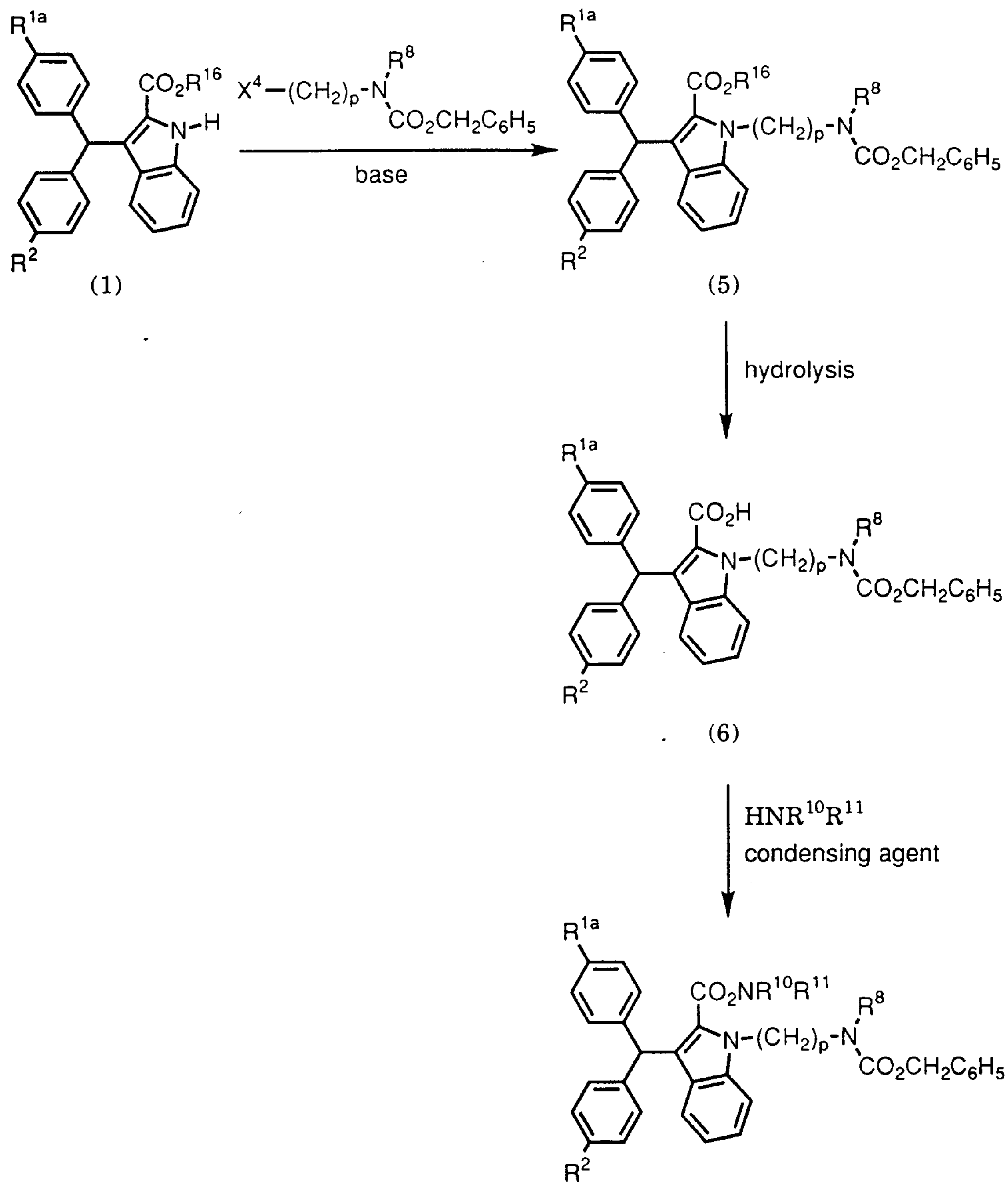


(1) (5) (6) (VI)

(In the formulae, $X^4$ represents chlorine, bromine, or iodine, and $R^{1a}$, $R^2$, $R^8$, $R^{10}$, $R^{11}$, $R^{16}$, and p have the same meanings as defined above.)

Compound (5) can be obtained from Compound (1) and a halogenated aminoalkyl protected by a carboxybenzyl group by N-alkylation according to the method of producing Compound (Ic) from Compound (1) or a similar method thereto. Compound (6) can be obtained from Compound (5) by hydrolysis of the ester moiety according to the method of producing Compound (Id) from Compound (Ic) or a similar method thereto. Then, Compound (VI) can be obtained from Compound (6) by amidation according to the method of producing Compound (If) from Compound (Ie) or Compound (IV) or a similar method thereto.

Compound (VII) can be prepared from Compound (1) and a halogenated alkylphthalimide according to the method of producing Compound (VI) from Compound (1) or a similar method thereto.

Compound (VIIa), which is Compound (VII) in which p is 2, can also be prepared from Compound (1) according to the following reaction steps:

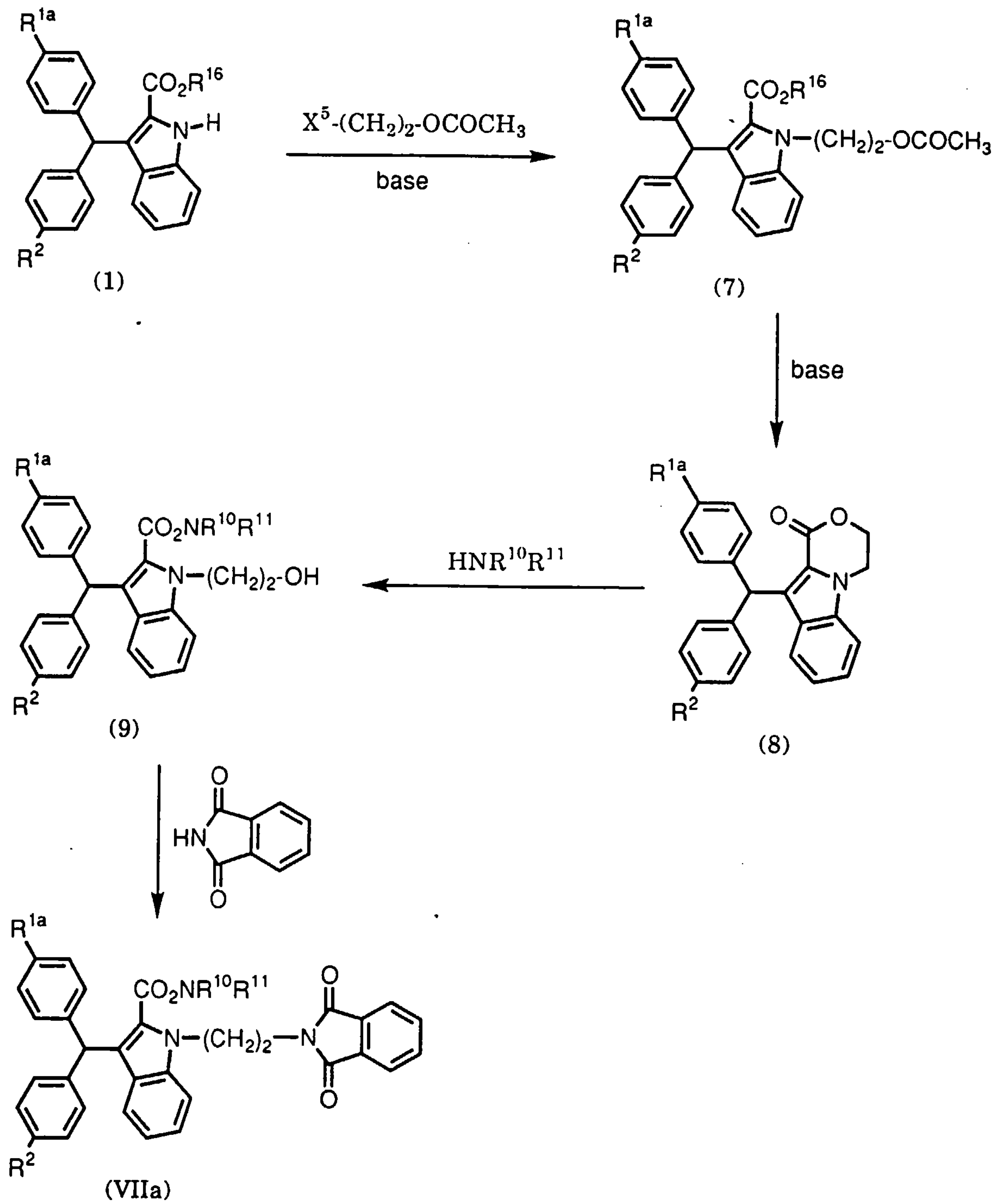


(1) (7) (8) (9) (VIIa)

(In the formulae, $X^5$ represents chlorine, bromine, or iodine, and $R^{1a}$, $R^2$, $R^{10}$, $R^{11}$, and $R^{16}$ have the same meanings as defined above.)

Compound (7) can be obtained from Compound (1) and a halogenated ethyl acetate by N-alkylation according to the method of producing Compound (Ic) from Compound (1) or a similar method thereto. Compound (8) can be obtained by treating Compound (7) with an alkyl lower alkoxide such as sodium methoxide, sodium ethoxide, and potassium tert-butoxide in a solvent such as a lower alcohol (e.g., methanol, ethanol), N,N-dimethylformamide, dimethyl sulfoxide, and tetrahydrofuran, at a temperature between room temperature and the boiling point of the employed solvent for 0.5 to 24 hours. Compound (9) can be obtained by reacting Compound (8) with ammonia, a primary amine, or a secondary amine in a solvent such as a lower alcohol (e.g., methanol, ethanol), N,N-dimethylformamide, dimethyl sulfoxide, and tetrahydrofuran, at a temperature between 50°C and the boiling point of the employed solvent for 0.5 to 24

hours, or without a solvent at a temperature between 50°C and 200°C for 0.5 to 24 hours. Then, Compound (VIIa) can be obtained by reacting Compound (9) with phthalimide in the presence of triphenylphosphine and the like, and diethyl azodicarboxylate and the like, in a solvent such as tetrahydrofuran, at a temperature between 0°C and the boiling point of the employed solvent for 0.5 to 48 hours.

As for the starting compounds, Compound (II) can be obtained according to the method described in Tetrahedron Lett., 28, 5651 (1987) or a similar method thereto, and Compound (III) can be obtained according to the method described in J. Am. Chem. Soc., 67, 423 (1945), J. Med. Chem., 32, 1681 (1989), or Chem. Pharm. Bull., 21, 1481 (1973), or a similar method thereto.

The intermediates and the desired compounds in the processes described above can be isolated and purified by purification methods conventionally used in organic synthetic chemistry, for example, filtration, extraction, washing, drying, concentration, recrystallization, and various kinds of chromatography. The intermediates may also be subjected to the subsequent reaction without purification.

In the case where a salt of Compound (I) is desired and it is produced in the form of the desired salt, it can be subjected to purification as such. In the case where Compound (I) is produced in the free state and its salt is desired, Compound (I) is dissolved or suspended in a suitable solvent, followed by addition of an appropriate acid or base to form a salt, and then the salt can be isolated.

Compounds (I) and pharmaceutically acceptable salts thereof may be in the form of adducts with water or various solvents, which are also within the scope of the present invention.

Examples of Compounds (I) obtained in the above processes are shown in Table 1.

Table 1-1

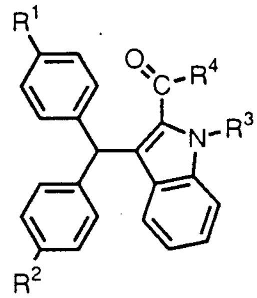

| Compd. No. | $R^1$ | $R^2$ | $R^3$ | $R^4$ |
|---|---|---|---|---|
| 1 | H | H | H | 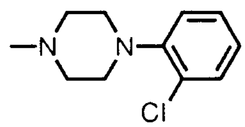 |
| 2 | H | H | 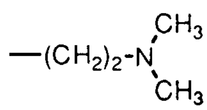 | 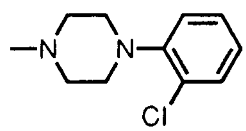 |
| 3 | H | H | 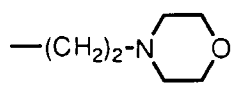 | 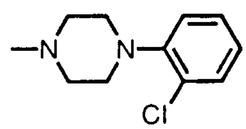 |
| 4 | H | H | 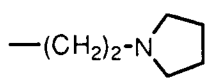 | 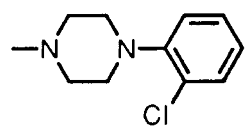 |
| 5 | H | H | 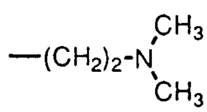 | $OCH_2CH_3$ |
| 6 | H | H | 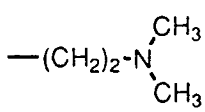 | OH |
| 7 | H | H | 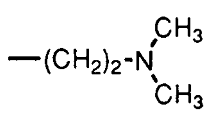 | $-\underset{H}{N}-(CH_2)_2CH_3$ |

Table 1-2

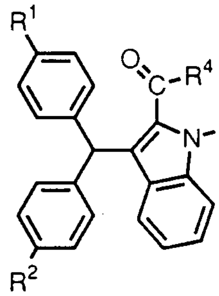

| Compd. No. | $R^1$ | $R^2$ | $R^3$ | $R^4$ |
|---|---|---|---|---|
| 8 | H | H | 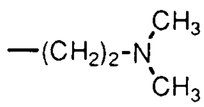 | 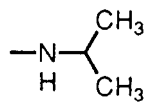 |
| 9 | H | H | 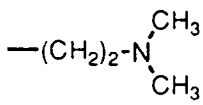 | 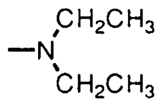 |
| 10 | H | H | 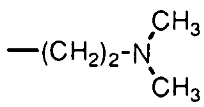 | 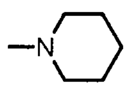 |
| 11 | H | H | 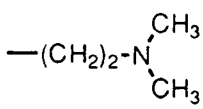 | 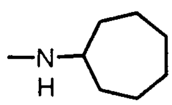 |
| 12 | H | H | 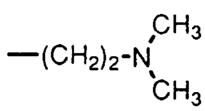 | 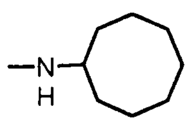 |
| 13 | H | H | 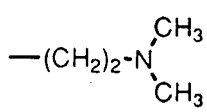 | 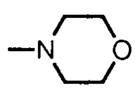 |
| 14 | H | H | 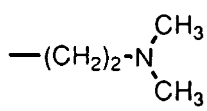 | 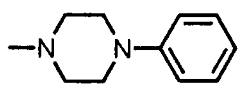 |

Table 1–3

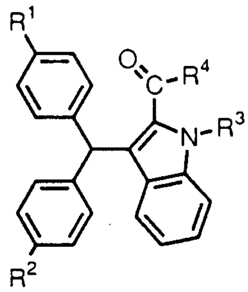

| Compd. No. | $R^1$ | $R^2$ | $R^3$ | $R^4$ |
|---|---|---|---|---|
| 15 | H | H | 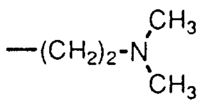 | 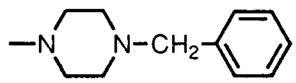 |
| 16 | H | H | 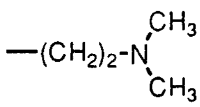 | 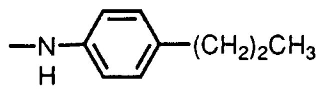 |
| 17 | H | H | 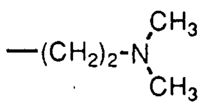 | 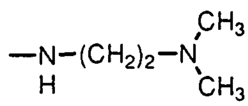 |
| 18 | H | H | 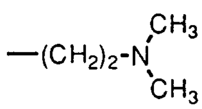 | 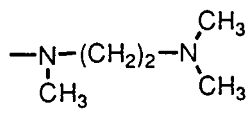 |
| 19 | H | H | 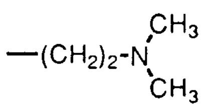 | 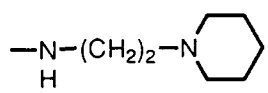 |
| 20 | H | H | 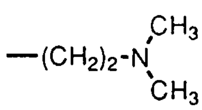 | 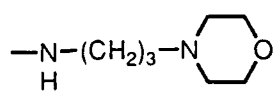 |
| 21 | H | H | 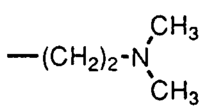 | 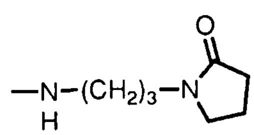 |

Table 1-4

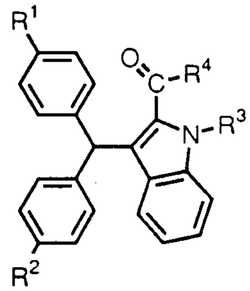

| Compd. No. | $R^1$ | $R^2$ | $R^3$ | $R^4$ |
|---|---|---|---|---|
| 22 | H | H | 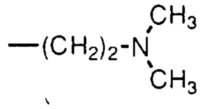 | 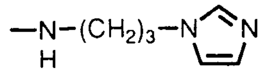 |
| 23 | H | H | 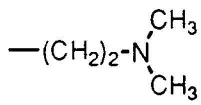 | 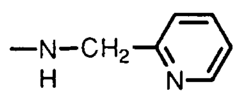 |
| 24 | H | H | 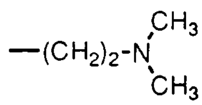 | 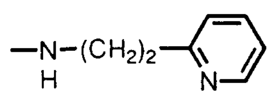 |
| 25 | H | H | 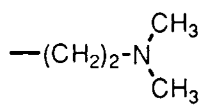 | 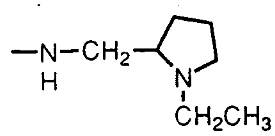 |
| 26 | H | H | 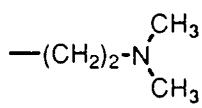 | 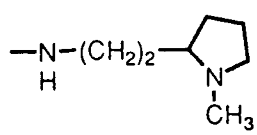 |
| 27 | H | H | 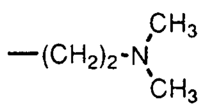 | 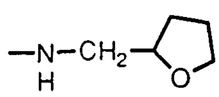 |
| 28 | H | H | 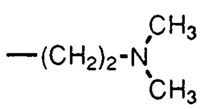 | 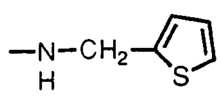 |

Table 1-5

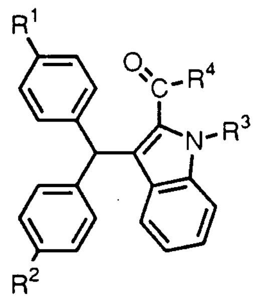

| Compd. No. | $R^1$ | $R^2$ | $R^3$ | $R^4$ |
|---|---|---|---|---|
| 29 | H | H | 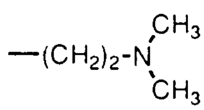 | 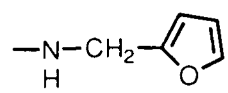 |
| 30 | H | H | 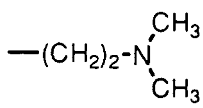 | 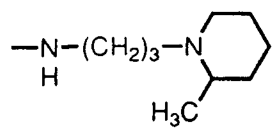 |
| 31 | H | H | 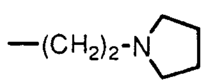 | OH |
| 32 | H | H | 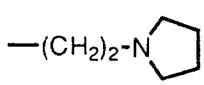 | 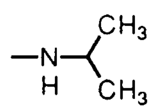 |
| 33 | H | H | 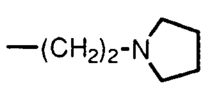 | 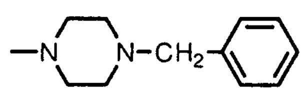 |
| 34 | H | H | 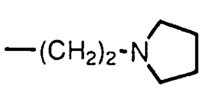 | 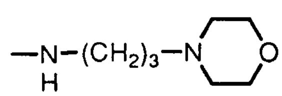 |
| 35 | H | H | 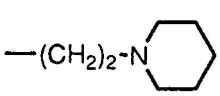 | OH |

Table 1-6

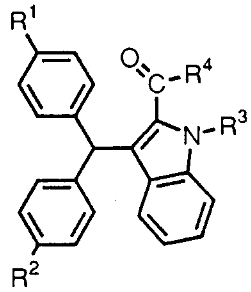

| Compd. No. | $R^1$ | $R^2$ | $R^3$ | $R^4$ |
|---|---|---|---|---|
| 36 | H | H | 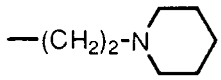 | 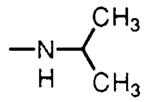 |
| 37 | H | H | 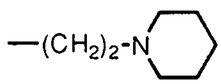 | 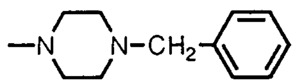 |
| 38 | H | H | 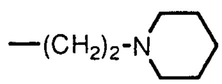 | 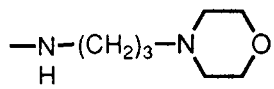 |
| 39 | H | H | 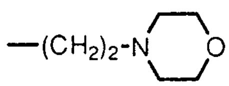 | OH |
| 40 | H | H | 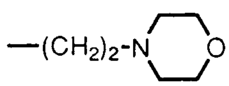 | 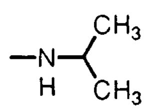 |
| 41 | H | H | 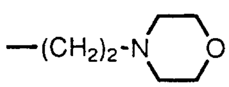 | 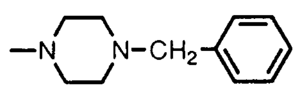 |
| 42 | H | H | 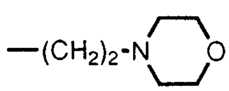 | 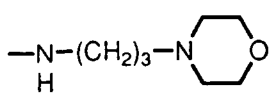 |

Table 1–7

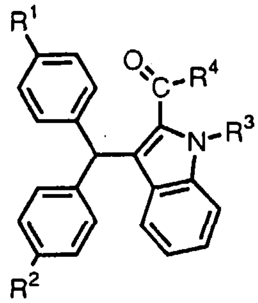

| Compd. No. | $R^1$ | $R^2$ | $R^3$ | $R^4$ |
|---|---|---|---|---|
| 43 | F | F | 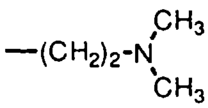 | $OCH_2CH_3$ |
| 44 | F | F | 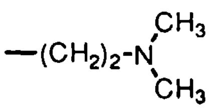 | OH |
| 45 | F | F | 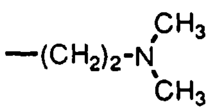 | 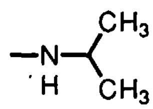 |
| 46 | F | F | 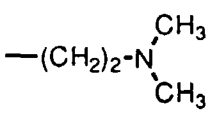 | 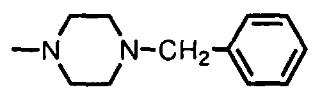 |
| 47 | F | F | 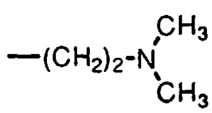 | 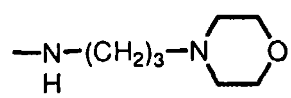 |

Table 1-8

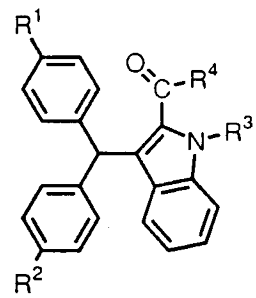

| Compd. No. | $R^1$ | $R^2$ | $R^3$ | $R^4$ |
|---|---|---|---|---|
| 48 | H | H | 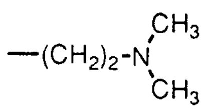 | 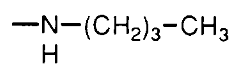 |
| 49 | H | H | 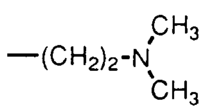 | 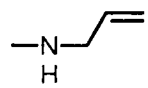 |
| 50 | H | H | 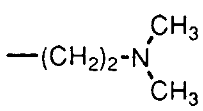 | 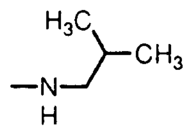 |
| 51 | H | H | 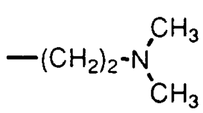 | 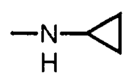 |
| 52 | H | H | 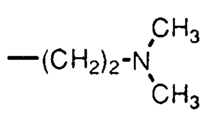 | 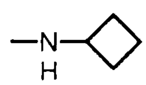 |
| 53 | H | H | 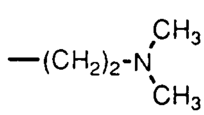 | 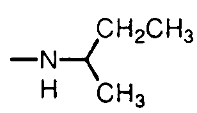 |
| 54 | H | H | 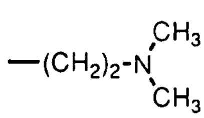 | 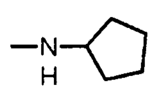 |

Table 1-9

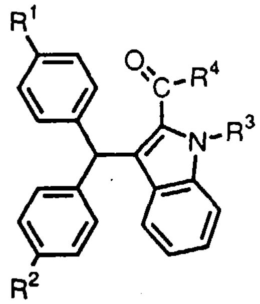

| Compd. No. | $R^1$ | $R^2$ | $R^3$ | $R^4$ |
|---|---|---|---|---|
| 55 | H | H | 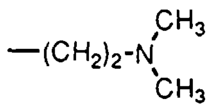 | 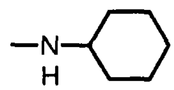 |
| 56 | H | H | 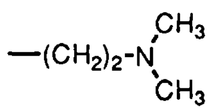 | 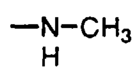 |
| 57 | H | H | 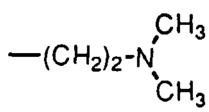 | 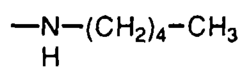 |
| 58 | H | H | 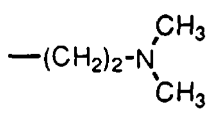 | 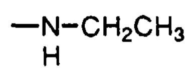 |
| 59 | H | H | 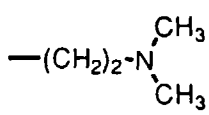 | 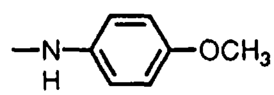 |
| 60 | H | H | 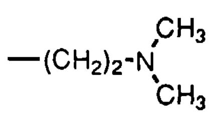 | 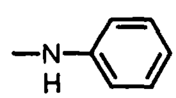 |

Table 1-10

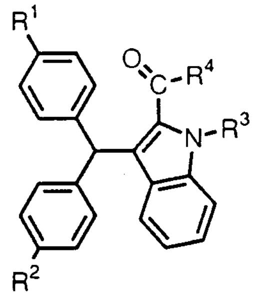

| Compd. No. | $R^1$ | $R^2$ | $R^3$ | $R^4$ |
|---|---|---|---|---|
| 61 | H | H | 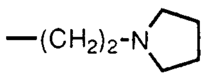 | 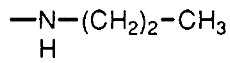 |
| 62 | H | H | 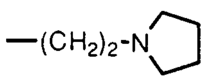 | 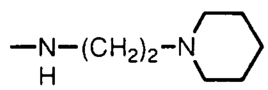 |
| 63 | H | H | 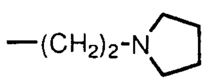 | 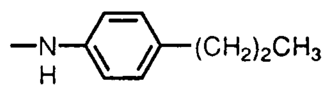 |
| 64 | H | H | 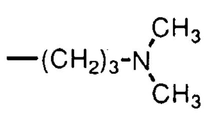 | $OCH_2CH_3$ |
| 65 | H | H | 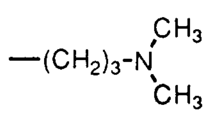 | OH |
| 66 | H | H | 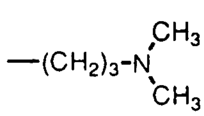 | 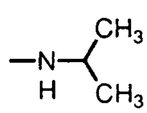 |
| 67 | H | H | 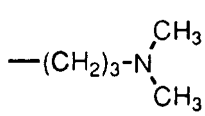 | 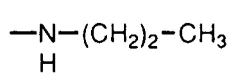 |

Table 1-11

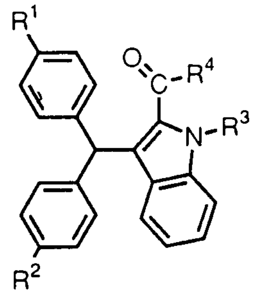

| Compd. No. | $R^1$ | $R^2$ | $R^3$ | $R^4$ |
|---|---|---|---|---|
| 68 | H | H | 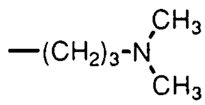 | 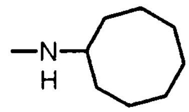 |
| 69 | H | H | 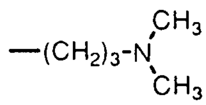 | 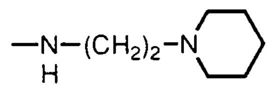 |
| 70 | H | H | 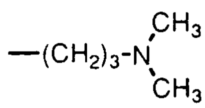 | 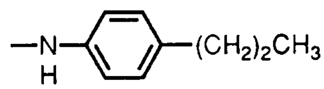 |
| 71 | H | H | 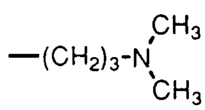 | 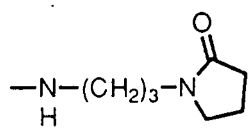 |
| 72 | H | H | 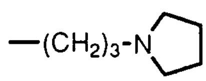 | $OCH_2CH_3$ |
| 73 | H | H | 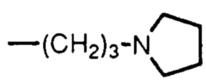 | OH |
| 74 | H | H | 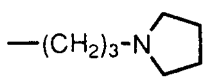 | 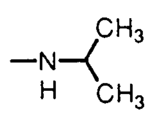 |

Table 1-12

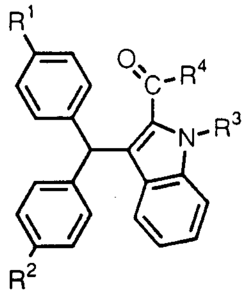

| Compd. No. | $R^1$ | $R^2$ | $R^3$ | $R^4$ |
|---|---|---|---|---|
| 75 | H | H | 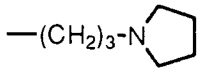 | 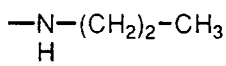 |
| 76 | H | H | 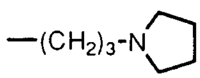 | 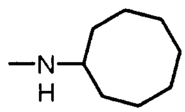 |
| 77 | H | H | 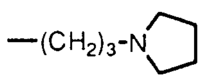 | 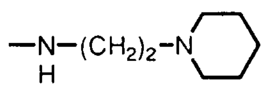 |
| 78 | H | H | 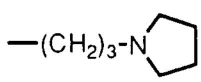 | 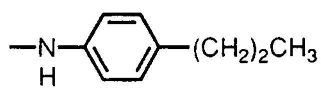 |
| 79 | H | H | 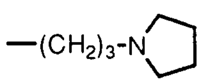 | 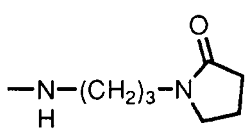 |
| 80 | H | H | 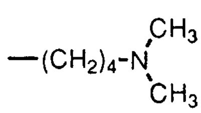 | $OCH_2CH_3$ |
| 81 | H | H | 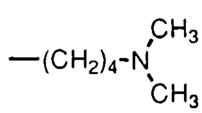 | OH |

Table 1-13

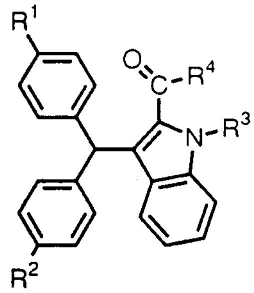

| Compd. No. | $R^1$ | $R^2$ | $R^3$ | $R^4$ |
|---|---|---|---|---|
| 82 | H | H | 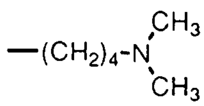 | 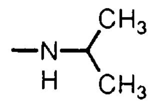 |
| 83 | H | H | 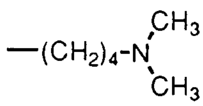 | 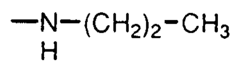 |
| 84 | H | H | 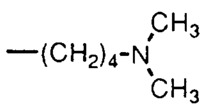 | 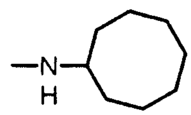 |
| 85 | H | H | 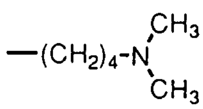 | 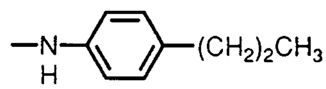 |
| 86 | H | H | 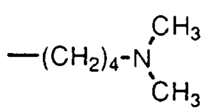 | 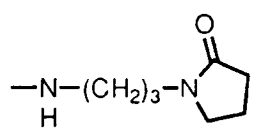 |
| 87 | H | H | 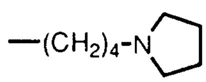 | $OCH_2CH_3$ |
| 88 | H | H | 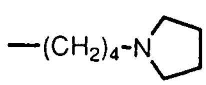 | OH |

Table 1-14

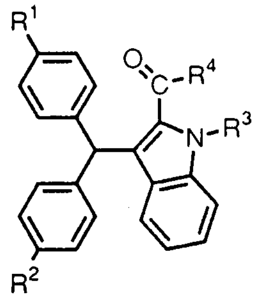

| Compd. No. | $R^1$ | $R^2$ | $R^3$ | $R^4$ |
|---|---|---|---|---|
| 89 | H | H | 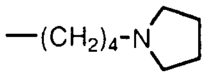 | 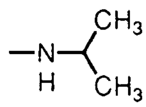 |
| 90 | H | H | 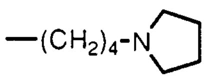 | 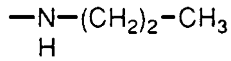 |
| 91 | H | H | 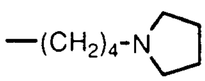 | 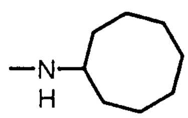 |
| 92 | H | H | 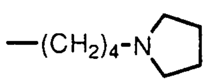 | 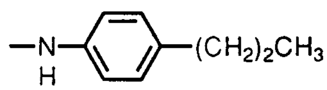 |
| 93 | H | H | 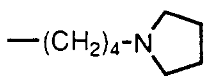 | 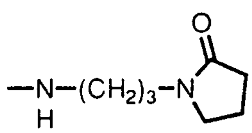 |
| 94 | Cl | Cl | 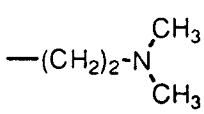 | $OCH_2CH_3$ |
| 95 | Cl | Cl | 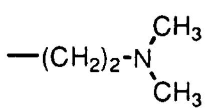 | OH |

Table 1–15

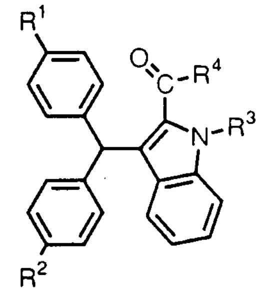

| Compd. No. | $R^1$ | $R^2$ | $R^3$ | $R^4$ |
|---|---|---|---|---|
| 96 | Cl | Cl | 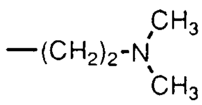 | 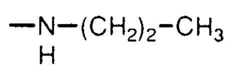 |
| 97 | Cl | Cl | 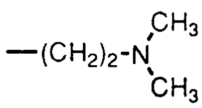 | 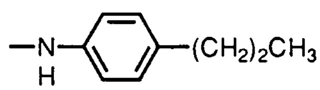 |
| 98 | $CH_3$ | $CH_3$ | 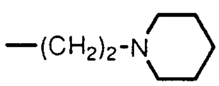 | $OCH_2CH_3$ |
| 99 | $CH_3$ | $CH_3$ | 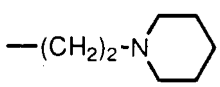 | OH |
| 100 | $CH_3$ | $CH_3$ | 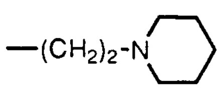 | 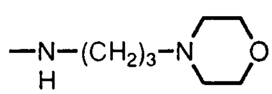 |
| 101 | $CH_3$ | $CH_3$ | 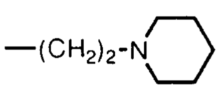 | 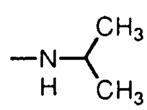 |
| 102 | $CH_3$ | $CH_3$ | 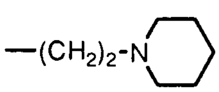 | 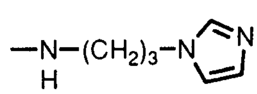 |

Table 1-16

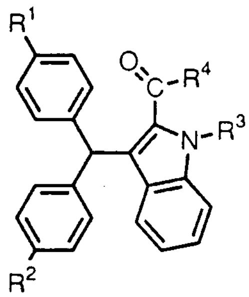

| Compd. No. | $R^1$ | $R^2$ | $R^3$ | $R^4$ |
|---|---|---|---|---|
| 103 | $CH_3$ | $CH_3$ | —$(CH_2)_2$-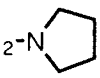 | $OCH_2CH_3$ |
| 104 | $CH_3$ | $CH_3$ | —$(CH_2)_2$-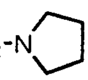 | OH |
| 105 | $CH_3$ | $CH_3$ | —$(CH_2)_2$-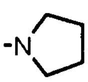 | —N(H)—$(CH_2)_3$-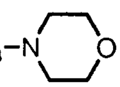 |
| 106 | $CH_3$ | $CH_3$ | —$(CH_2)_2$-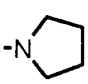 | 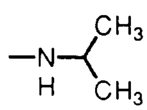 |
| 107 | $CH_3$ | $CH_3$ | —$(CH_2)_2$-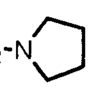 | —N(H)—$(CH_2)_3$-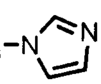 |
| 108 | $CH_3$ | $CH_3$ | —$(CH_2)_2$-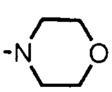 | $OCH_2CH_3$ |
| 109 | $CH_3$ | $CH_3$ | —$(CH_2)_2$-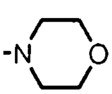 | OH |

Table 1-17

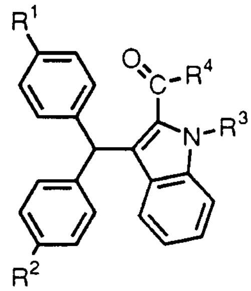

| Compd. No. | $R^1$ | $R^2$ | $R^3$ | $R^4$ |
|---|---|---|---|---|
| 110 | $CH_3$ | $CH_3$ | 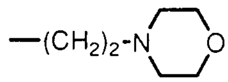 | 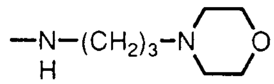 |
| 111 | $CH_3$ | $CH_3$ | 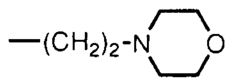 | 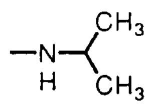 |
| 112 | $CH_3$ | $CH_3$ | 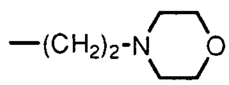 | 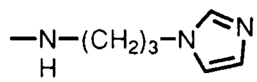 |
| 113 | $CH_3$ | $CH_3$ | 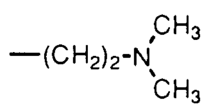 | $OCH_2CH_3$ |
| 114 | $CH_3$ | $CH_3$ | 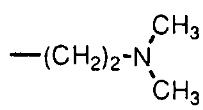 | OH |
| 115 | $CH_3$ | $CH_3$ | 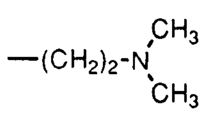 | 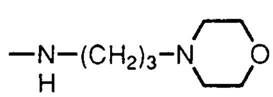 |
| 116 | $CH_3$ | $CH_3$ | 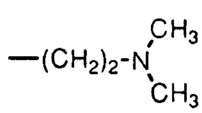 | 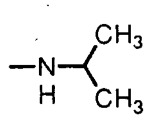 |

Table 1-18

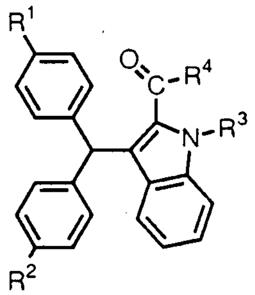

| Compd. No. | $R^1$ | $R^2$ | $R^3$ | $R^4$ |
|---|---|---|---|---|
| 117 | $CH_3$ | $CH_3$ | 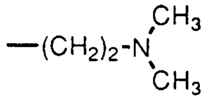 | 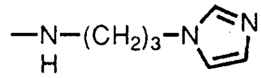 |
| 118 | OH | H | 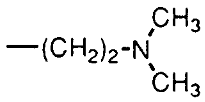 | 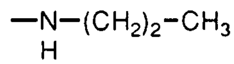 |
| 119 | OH | H | 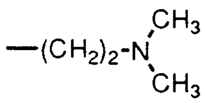 | 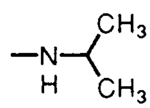 |
| 120 | OH | H | 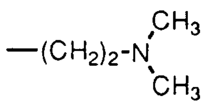 | 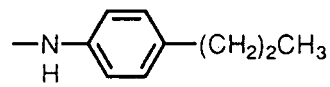 |
| 121 | OH | H | 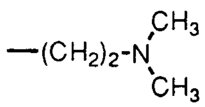 | 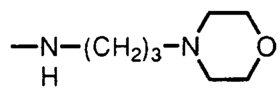 |
| 122 | OH | H | 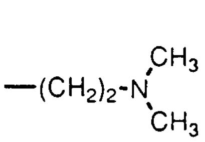 | 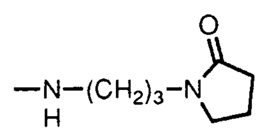 |
| 123 | OH | H | 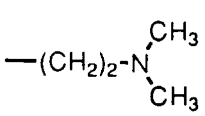 | 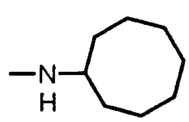 |

Table 1−19

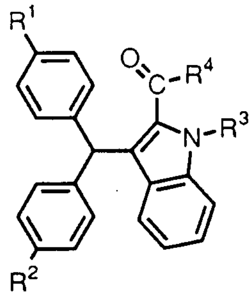

| Compd. No. | $R^1$ | $R^2$ | $R^3$ | $R^4$ |
|---|---|---|---|---|
| 124 | OH | H | 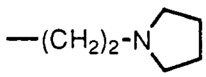 | $-\underset{H}{N}-(CH_2)_2-CH_3$ |
| 125 | OH | H | 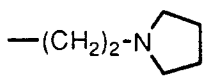 | 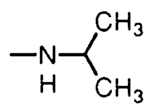 |
| 126 | OH | H | 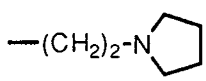 | 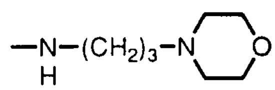 |
| 127 | OH | H | 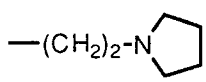 | 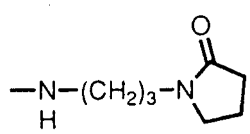 |
| 128 | 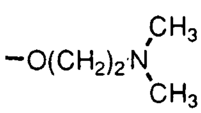 | H | H | 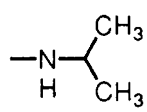 |
| 129 | 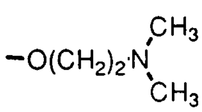 | H | H | $-\underset{H}{N}-(CH_2)_2-CH_3$ |
| 130 | 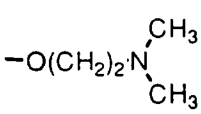 | H | H | 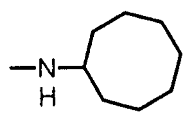 |

Table 1-20

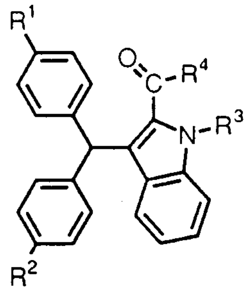

| Compd. No. | $R^1$ | $R^2$ | $R^3$ | $R^4$ |
|---|---|---|---|---|
| 131 | 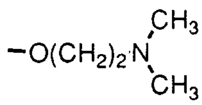 | H | H | 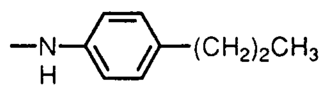 |
| 132 | 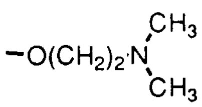 | H | H | 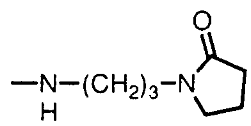 |
| 133 | 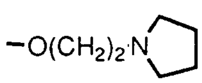 | H | H | 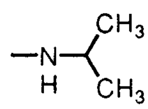 |
| 134 | 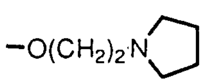 | H | H | 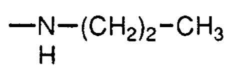 |
| 135 | 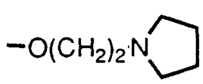 | H | H | 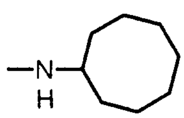 |
| 136 | 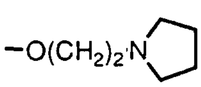 | H | H | 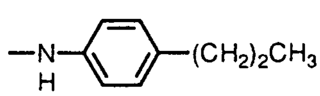 |
| 137 | 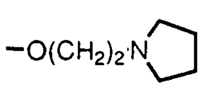 | H | H | 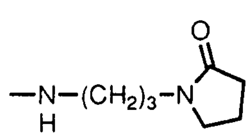 |

Table 1-21

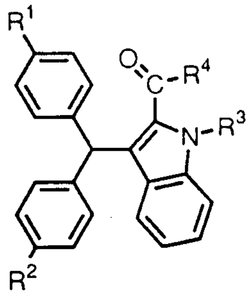

| Compd. No. | $R^1$ | $R^2$ | $R^3$ | $R^4$ |
|---|---|---|---|---|
| 138 | 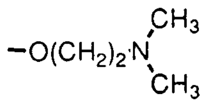 | H | 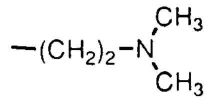 | $OCH_2CH_3$ |
| 139 | 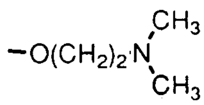 | H | 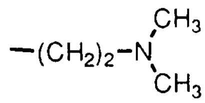 | OH |
| 140 | 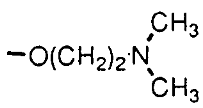 | H | 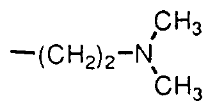 | 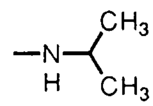 |
| 141 | 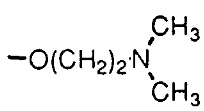 | H | 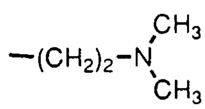 | 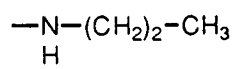 |
| 142 | 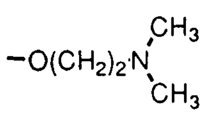 | H | 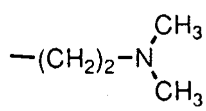 | 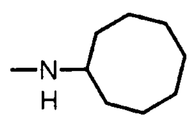 |
| 143 | 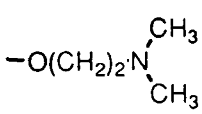 | H | 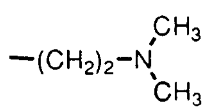 | 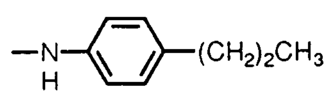 |
| 144 | 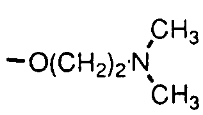 | H | 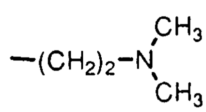 | 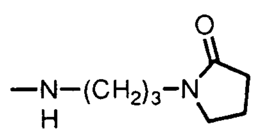 |

Table 1-22

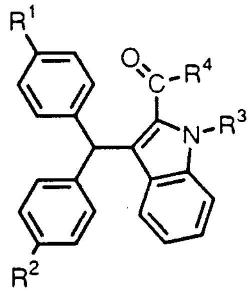

| Compd. No. | $R^1$ | $R^2$ | $R^3$ | $R^4$ |
|---|---|---|---|---|
| 145 | 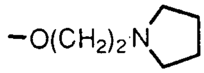 | H | 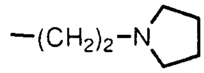 | $OCH_2CH_3$ |
| 146 | 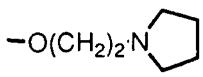 | H | 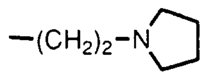 | OH |
| 147 | 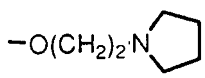 | H | 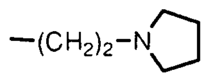 | 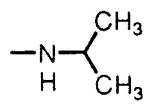 |
| 148 | 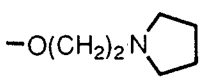 | H | 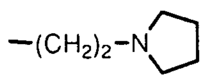 | $-\underset{H}{N}-(CH_2)_2-CH_3$ |
| 149 | 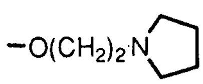 | H | 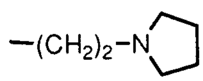 | 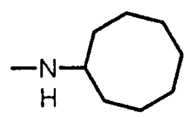 |
| 150 | 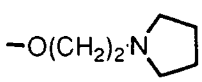 | H | 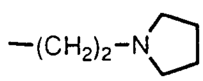 | 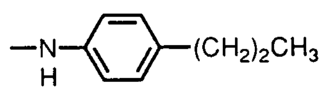 |
| 151 | 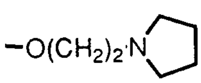 | H | 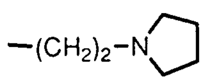 | 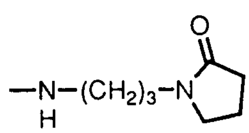 |

Table 1-23

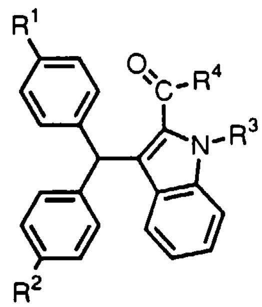

| Compd. No. | $R^1$ | $R^2$ | $R^3$ | $R^4$ |
|---|---|---|---|---|
| 152 | H | H | 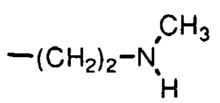 | 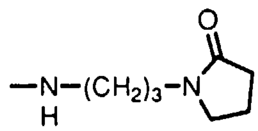 |
| 153 | H | H | 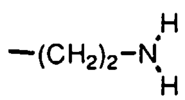 | 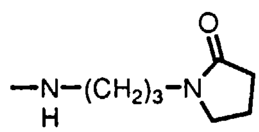 |

The inhibitory effect of the compounds of the present invention on bone absorption is shown below by test examples.

Test Example 1: Inhibitory effect on bone absorption

The calvaria was excised from a newborn dd mouse (5 to 6 days old) under sterile conditions. The calvaria was washed with a modified Dulbecco phosphate buffer physiological saline solution containing neither calcium nor magnesium (a product of Gibco Oriental), and divided into two parts along the center suture. A half of the calvaria was cultivated in a modified Dulbecco Eagle medium (1.5 ml) (a product of Gibco Oriental) containing 2.5% fetal calf serum and 15% equine serum which had been inactivated by heating at 56°C for 20 minutes. The test compound was dissolved in dimethyl sulfoxide and 10 μl of the resulting solution was added to the culture medium to give the final concentrations of $3 \times 10^{-6}$M, $1 \times 10^{-5}$M, and $3 \times 10^{-5}$M. PTH (parathyroid hormone) was dissolved in a 0.15 M saline solution (pH 3) and 3 μl of the resulting solution was added to the culture medium to give the final concentration of $1 \times 10^{-8}$M. The culturing was carried out under the condition of 95% air and 5% carbon dioxide and at a temperature of 37°C for 96 hours. At 48 hours after the start of the culturing, the culture medium was renewed and the test compound and PTH treated as defined above were added thereto. For examining the effect of the test compound on calcium liberation (bone absorption) from the PTH enhanced bone, a control group, a group using PTH ($1 \times 10^{-8}$M), and a group using both the test compound ($3 \times 10^{-6}$M, $1 \times 10^{-5}$M, $3 \times 10^{-5}$M) and PTH were prepared. The amount of the bone absorption was determined by measuring the amount of calcium accumulated in the culture as collected after 96 hours of culturing. The total calcium concentration in the culture was measured with Calcium C Test Wako. The inhibition rate was calculated using the following equation and the result was shown in terms of 50% inhibitory concentration ($IC_{50}$). The results are shown in Table 2.

Inhibition of bone absorption from PTH enhanced bone Inhibition rate (%) = $[(C_P - C_D)/(C_P - C_0)] \times 100$

$C_0$ : The total calcium concentration in the culture containing neither the test compound nor PTH.
$C_P$ : The total calcium concentration in the culture treated with only PTH.
$C_D$ : The total calcium concentration in the culture treated with both the test compound and PTH.

Table 2

| Compound No. | Inhibitory effect on bone absorption ($IC_{50}$;μM) |
|---|---|
| 8 | 14.3 |
| 10 | 9.1 |
| 11 | 17.5 |
| 13 | 11.2 |
| 14 | 10.9 |
| 15 | 14.3 |
| 20 | 3.7 |
| 21* | 14.3 |
| 22 | 13.7 |
| 56 | 13.0 |
| 62 | 18.1 |
| 64 | 16.7 |
| 97 | 14.3 |
| 119 | 10.8 |
| 120 | 10.5 |

*: free base

The inhibitory effect of the compounds of the present application on bone resorption was also determined by their inhibitory effect on the excretion of urinary hydroxyproline which is elevated by ovariectomy of the animals tested, according to the report of Kalu et al. [Bone and Mineral., 14, 175 (1991)].

Test Example 2: Inhibitory effect on the increase of urinary hydroxyproline excretion by ovariectomy

12-weeks-old female SD strain rats (Japan Clea Co., Ltd.) were acclimated for 1 week during which they were given tap water and a standard diet (F2; Funabashi Farmas) ad libitum. The experiment was carried out using the rats weighing 270-310 g. After the animals were subjected to sham operation or bilateral ovariectomy, they were allowed free access to water passed through ion exchange resin instead of tap water, and were housed in individual cages. The test compound (10 mg/kg) was suspended in 0.3% Tween or 0.5% methylcellulose, and the obtained suspension was orally administered to the ovariectomized rats once a day for two weeks starting from the day following ovariectomy (0.5 ml/100 g body weight). 0.3% Tween or 0.5% methylcellulose was similarly administered to sham-operated rats and ovariectomized control rats. After the final administration, the rats were placed in individual metabolic cages and fasted during 24 hours, and urine was collected. The volume of the collected urine was measured and the urine was centrifuged at 3,000 rpm for 15 min at 4°C. The concentration of hydroxyproline in the supernatant was measured by the method of Ikeda et al. [Ann. Rep. Tokyo Metr. Res. Lab. P. H., 36, 277-282 (1985)] and the concentration of creatinine in the supernatant was measured with creatinine test WAKO (Wako Pure Chem.) The amount of urinary hydroxyproline excretion was expressed by the molar ratio of the amount of hydroxyproline to the amount of creatinine. The rate of inhibition on increase of the amount of urinary hydroxyproline excretion in ovariectomized rats treated with the test compound compared to that of the control group was calculated using the following equation. The results are shown in Table 3.

$$\text{Inhibition rate (\%)} = [(P1 - P2)/(P1 - P3)] \times 100$$

P1 : The amount of urinary hydroxyproline excretion in ovariectomized rats treated with 0.3% Tween (μmol/mmol).

P2 : The amount of urinary hydroxyproline excretion in ovariectomized rats treated with the test compound (μmol/mmol).

P3 : The amount of urinary hydroxyproline in sham-operated rats treated with 0.3% Tween (μmol/mmol).

Table 3

| Compd. No. | Inhibition rate of increase of the amount of urinary hydroxyproline excretion |
|---|---|
| 4 | 43 |
| 6 | 100 |
| 8 | 100 |
| 11 | 100 |
| 15 | 100 |
| 20 | 100 |
| 21** | 100 |
| 22 | 87 |
| 51 | 70 |
| 56 | 57 |
| 62 | 53 |
| 64 | 77 |
| 97 | 40 |
| 98 | 47 |
| 121 | 59 |

**: hydrochloride

Test Example 3: Inhibitory effect on decrease of bone density by ovariectomy

The experiment was carried out using 13-weeks-old female SD strain rats (Japan Crea Co. Ltd.) The animals were subjected to sham operation or bilateral ovariectomy under Nembutal anesthesia. The test compound (10 mg/kg) was dissolved or suspended in 0.5% methylcellulose (Shin-Etsu Chemical Co., Ltd.) and administered orally once a day from the first day after the operation for 6 weeks. For the sham-operated group and the ovariectomized control group, 0.5% methylcellulose (Shin-Etsu Chemical Co., Ltd.) was administered orally. The rat was slaughtered by femoral artery bleeding, the right hindlimb was ablated, and then the bone mineral content (BMC) and bone density (BD) of the tibia was measured according to a DEXA (DXA) method [Japan Clinic, 52 (9), 2329-2334 (1994)] using bone mineral measuring apparatus DCS-600 (Aloka Co., Ltd.). Tibial BMC and BD in the sham-operated, ovariectomized control, and test compound-treated ovariectomized group are shown in Table 4.

Table 4

| Compound No. | BMC (mg) | BD ($mg/cm^2$) |
|---|---|---|
| sham-operated group | 257.76 ± 7.52 | 125.37 ± 1.42 |
| ovariectomized control group | 233.48 ± 4.29 | 110.43 ± 1.38 |
| Compound 21**-treated ovariectomized group | 268.81 ± 7.38 | 126.95 ± 1.78 |

**: hydrochloride

Compounds (I) and pharmaceutically acceptable salts thereof can be formulated into generally employed dose forms such as tablets, capsules, and syrups, and administered orally or parenterally through intramuscular injection, intravenous injection, drip infusion, or rectal administration using suppositories. For preparing these dose forms for oral or parenteral administration, generally known techniques are applied. For example, the preparations may contain various excipients, lubricants, binders, disintegrating agents, isotonizing agents, emulsifiers, and the like.

Examples of the carriers which can be used are water, injectable distilled water, physiological saline, glucose, fructose, sucrose, mannitol, lactose, starch, cellulose, methyl cellulose, carboxymethyl cellulose, hydroxypropyl cellulose, alginic acid, talc, sodium citrate, calcium carbonate, calcium hydrogenphosphate, magnesium stearate, urea, silicone resins, sorbitan fatty acid esters, and glycerin fatty acid esters.

The effective dose and administration schedule of Compound (I) or a pharmaceutically acceptable salt thereof varies depending upon the mode of administration, the age, body weight and conditions of a patient, etc. However, generally, Compound (I) or a pharmaceutically acceptable salt thereof is administered in a dose of 0.1 to 10 mg/kg/day in 1 to 4 parts.

Certain embodiments of the present invention are illustrated in the following Examples and Reference Examples.

## Best Mode for Carrying out the Invention

### Example 1

2-[4-(2-Chlorophenyl)piperazinylcarbonyl]-3-(diphenylmethyl)indole (Compound 1)

Benzhydryl acetate (2.2 g, 9.71 mmol) and 2-[4-(2-chlorophenyl)piperazinylcarbonyl]indole (3.0 g, 8.83 mmol) were dissolved in a mixed solvent of methylene chloride (50 ml) and chloroform (50 ml), and methanesulfonic acid (1.72 ml, 26.5 mmol) was added thereto, followed by stirring at room temperature for 3 hours. A saturated aqueous solution of sodium bicarbonate was added to the reaction solution for neutralization followed by extraction with chloroform. The resulting organic layer was washed with a saturated aqueous solution of sodium bicarbonate and a saturated aqueous solution of sodium chloride, and dried over magnesium sulfate, and the solvent was distilled off under reduced pressure to give 6.5 g of a crude product. The obtained crude product was washed with ethanol (60 ml) under heating to give 4.0 g (yield: 89%) of the title compound.

$^1$H-NMR($CDCl_3$) δ(ppm): 2.65-2.8 (4H, m), 3.5-3.65 (4H, m), 5.90 (1H, s), 6.8-6.9 (1H, m), 6.9-7.05 (2H, m), 7.1-7.4 (15H, m), 8.50 (1H, s).

### Example 2

2-[4-(2-Chlorophenyl)piperazinylcarbonyl]-1-(2-dimethylaminoethyl)-3-(diphenylmethyl)indole (Compound 2)

To a solution of Compound 1 (1.0 g, 1.98 mmol) obtained in Example 1 in 10 ml of N,N-dimethylformamide was portionwise added sodium hydride (60% in oil, 166 mg, 4.15 mmol) with stirring at room temperature, and 2-dimethylaminoethylchloride hydrochloride (298 mg, 2.07 mmol) was added thereto, followed by heating to 80°C and then stirring for one hour. A saturated aqueous solution of ammonium chloride was added to the reaction solution for neutralization, water was added thereto, and the resulting crystals were collected by filtration to give 1.1 g of a crude product. The obtained crude product was recrystallized from ethanol (65 ml) to give 0.67 g (yield: 59%) of the title compound.

$^1$H-NMR($CDCl_3$) δ(ppm): 2.1-2.25 (1H, m), 2.37 (6H, s), 2.45-2.55 (1H, m), 2.65-2.9 (3H, m), 3.0-3.15 (2H, m), 3.15-3.25 (1H, m), 3.65-3.8 (1H, m), 3.9-4.0 (1H, m), 4.05-4.2 (1H, m), 4.35-4.5 (1H, m), 5.77 (1H, s), 6.76 (1H, dd, J=7.9, 1.5Hz), 6.9-7.0 (2H, m), 7.05-7.4 (15H, m).

Example 3

2-[4-(2-Chlorophenyl)piperazinylcarbonyl]-3-(diphenylmethyl)-1-(2-morpholinoethyl)indole (Compound 3)

Substantially the same procedure as in Example 2 was repeated using Compound 1 (1.0 g, 1.98 mmol) obtained in Example 1 and 2-morpholinoethylchloride hydrochloride (385 mg, 2.07 mmol) to give 0.91 g (yield: 74%) of the title compound.

$^1$H-NMR($CDCl_3$) δ(ppm): 2.0-2.15 (1H, m), 2.45-2.65 (5H, m), 2.65-2.9 (3H, m), 2.9-3.15 (2H, m), 3.15-3.3 (1H, m), 3.6-3.8 (5H, m), 3.9-4.2 (2H, m), 4.3-4.5 (1H, m), 5.78 (1H, s), 6.76 (1H, dd, J=7.9, 1.5Hz), 6.9-7.0 (2H, m), 7.05-7.4 (15H, m).

Example 4

2-[4-(2-Chlorophenyl)piperazinylcarbonyl]-3-(diphenylmethyl)-1-(2-pyrrolidinylethyl)indole (Compound 4)

Substantially the same procedure as in Example 2 was repeated using Compound 1 (0.95 g, 1.88 mmol) obtained in Example 1 and 2-pyrrolidinylethylchloride hydrochloride (335 mg, 1.97 mmol) to give 0.61 g (yield: 54%) of the title compound.

$^1$H-NMR($CDCl_3$) δ(ppm): 1.58 (2H, br s), 1.7-1.85 (3H, m), 2.0-2.1 (1H, m), 2.45-2.55 (1H, m), 2.61 (4H, br s), 2.75-2.85 (2H, m), 2.85-3.3 (4H, m), 3.65-3.8 (1H, m), 3.95-4.2 (2H, m), 4.3-4.5 (1H, m), 5.79 (1H, s), 6.7-6.8 (1H, m), 6.9-7.05 (2H, m), 7.10 (1H, d, J=7.9Hz), 7.15-7.4 (14H, m).

Example 5

Ethyl 1-(2-dimethylaminoethyl)-3-(diphenylmethyl)indole-2-carboxylate (Compound 5)

Substantially the same procedure as in Example 2 was repeated using ethyl 3-(diphenylmethyl)indole-2-carboxylate (8.5 g, 23.9 mmol) obtained in Reference Example 1 and 2-dimethylaminoethylchloride hydrochloride (3.8 g, 26.3 mmol) to give 7.0 g (yield: 69%) of the title compound.

$^1$H-NMR($CDCl_3$) δ(ppm): 1.35 (3H, t, J=7.2Hz), 2.33 (6H, s), 2.6-2.7 (2H, m), 4.34 (2H, q, J=7.2Hz), 4.55-4.65 (2H, m), 6.57 (1H, s), 6.8-6.95 (2H, m), 7.1-7.3 (11H, m), 7.39 (1H, d, J=8.3Hz).

Example 6

1-(2-Dimethylaminoethyl)-3-(diphenylmethyl)indole-2-carboxylic acid (Compound 6)

Compound 5 (7.0 g, 16.5 mmol) obtained in Example 5 was dissolved in ethanol (50 ml), and a 2N aqueous sodium hydroxide solution (25 ml) was added thereto, followed by heating under reflux. After 4 hours, a 2N aqueous sodium hydroxide solution (10 ml) was added thereto, followed by heating under reflux for one hour. The reaction solution was concentrated under reduced pressure, water was added, and the pH of the solution was adjusted to 5. A little amount of chloroform was added to the mixture followed by stirring. The precipitated crystals were collected by filtration, washed with water, and dried under reduced pressure to give 5.7 g (yield: 87%) of the title compound.

$^1$H-NMR($CDCl_3$) δ(ppm): 2.45 (6H, s), 3.27 (2H, t, J=5.9Hz), 4.77 (2H, t, J=5.9Hz), 6.81 (1H, s), 6.89 (1H, t, J=7.6Hz), 7.05-7.2 (12H, m), 7.33 (1H, d, J=8.3Hz).

Example 7

N-Propyl-1-(2-dimethylaminoethyl)-3-(diphenylmethyl)indole-2-carboxamide (Compound 7)

1-(3-Dimethylaminopropyl)-3-ethylcarbodiimide hydrochloride (1.45 g, 7.53 mmol) was added to a solution of Compound 6 (1.5 g, 3.76 mmol) obtained in Example 6 and propylamine (0.47 ml, 5.65 mmol) in 50 ml of methylene chloride, followed by stirring at room temperature for 4.5 hours. Water was added to the reaction solution followed by extraction with chloroform. The resulting organic layer was washed successively with water and a saturated aqueous solution of sodium chloride, and dried over magnesium sulfate, and the solvent was distilled off under reduced pressure to give a crude product. The obtained crude product was purified with silica gel column chromatography (chloroform/methanol = 40/1) followed by recrystallization from diisopropyl ether to give 0.98 g (yield: 59%) of the title compound.

$^1$H-NMR($CDCl_3$) δ(ppm): 0.85 (3H, t, J=7.4Hz), 1.4-1.55 (2H, m), 2.19 (6H, s), 2.76 (2H, t, J=6.6Hz), 3.25-3.35 (2H, m), 4.42 (2H, t, J=6.6Hz), 6.14 (1H, s), 6.85-6.95 (2H, m), 7.15-7.35 (13H, m).

In the following Examples 8 to 29, substantially the same procedure as in Example 7 was repeated using corresponding amines in place of propylamine to give the desired compounds.

Example 8

N-Isopropyl-1-(2-dimethylaminoethyl)-3-(diphenylmethyl)indole-2-carboxamide (Compound 8)

$^1$H-NMR($CDCl_3$) δ(ppm): 1.10 (6H, d, J=6.6Hz), 2.23 (6H, s), 2.78 (2H, t, J=6.9Hz), 4.15-4.3 (1H, m), 4.46 (2H, t, J=6.9Hz), 6.06 (1H, s), 6.67 (1H, br d, J=7.3Hz), 6.8-6.9 (2H, m), 7.1-7.3 (11H, m), 7.35 (1H, d, J=8.6Hz).

Example 9

N,N-Diethyl-1-(2-dimethylaminoethyl)-3-(diphenylmethyl)indole-2-carboxamide (Compound 9)

$^1$H-NMR($CDCl_3$) δ(ppm): 0.73 (3H, t, J=7.3Hz), 1.22 (3H, t, J=7.3Hz), 2.31 (6H, s), 2.5-2.8 (3H, m), 3.0-3.15 (1H, m), 3.4-3.5 (2H, m), 4.0-4.1 (1H, m), 4.15-4.25 (1H, m), 5.65 (1H, s), 6.9-7.0 (1H, m), 7.1-7.35 (13H, m).

Example 10

1-(2-Dimethylaminoethyl)-3-(diphenylmethyl)-2-(piperidinocarbonyl)indole (Compound 10)

$^1$H-NMR($CDCl_3$) δ(ppm): 0.8-1.0 (1H, m), 1.0-1.2 (1H, m), 1.4-1.6 (4H, m), 2.30 (6H, s), 2.5-2.8 (3H, m), 3.0-3.1 (1H, m), 3.4-3.5 (1H, m), 3.65-3.75 (1H, m), 4.0-4.15 (1H, m), 4.25-4.4 (1H, m), 5.70 (1H, s), 6.92 (1H, t, J=7.4Hz), 7.1-7.3 (13H, m).

Example 11

N-Cycloheptyl-1-(2-dimethylaminoethyl)-3-(diphenylmethyl)indole-2-carboxamide (Compound 11)

$^1$H-NMR($CDCl_3$) δ(ppm): 1.2-1.35 (2H, m), 1.35-1.65 (8H, m), 1.85-2.0 (2H, m), 2.23 (6H, s), 2.78 (2H, t, J=6.9Hz), 4.0-4.15 (1H, m), 4.46 (2H, t, J=6.9Hz), 6.06 (1H, s), 6.69 (1H, br d, J=7.6Hz), 6.85-6.9 (2H, m), 7.15-7.3 (11H, m), 7.35 (1H, d, J=8.2Hz).

Example 12

N-Cyclooctyl-1-(2-dimethylaminoethyl)-3-(diphenylmethyl)indole-2-carboxamide (Compound 12)

$^1$H-NMR($CDCl_3$) δ(ppm): 1.2-1.35 (2H, m), 1.35-1.6 (10H, m), 1.7-1.85 (2H, m), 2.20 (6H, s), 2.74 (2H, t, J=6.9Hz), 4.05-4.2 (1H, m), 4.45 (2H, t, J=6.9Hz), 6.07 (1H, s), 6.55-6.65 (1H, m), 6.8-6.85 (2H, m), 7.1-7.35 (12H, m).

Example 13

1-(2-Dimethylaminoethyl)-3-(diphenylmethyl)-2-(morpholinocarbonyl)indole (Compound 13)

$^1$H-NMR($CDCl_3$) δ(ppm): 2.28 (6H, s), 2.5-2.7 (2H, m), 2.7-2.9 (2H, m), 2.9-3.1 (1H, m), 3.1-3.3 (1H, m), 3.5-3.75 (4H, m), 3.95-4.15 (1H, m), 4.2-4.4 (1H, m), 5.73 (1H, s), 6.93 (1H, t, J=7.4Hz), 7.05-7.4 (13H, m).

Example 14

1-(2-Dimethylaminoethyl)-3-(diphenylmethyl)-2-(4-phenylpiperazinylcarbonyl)indole (Compound 14)

$^1$H-NMR($CDCl_3$) δ(ppm): 2.28 (6H, s), 2.3-2.4 (1H, m), 2.5-2.75 (3H, m), 2.9-3.25 (4H, m), 3.7-3.85 (2H, m), 4.0-4.15 (1H, m), 4.25-4.4 (1H, m), 5.75 (1H, s), 6.77 (2H, d, J=8.6Hz), 6.85-6.95 (2H, m), 7.1-7.3 (15H, m).

Example 15

2-(4-Benzylpiperazinylcarbonyl)-1-(2-dimethylaminoethyl)-3-(diphenylmethyl)indole (Compound 15)

$^1$H-NMR($CDCl_3$) δ(ppm): 1.6-1.7 (1H, m), 1.95-2.05 (1H, m), 2.3-2.45 (2H, m), 2.33 (6H, s), 2.55-2.8 (2H, m), 2.8-2.95 (1H, m), 3.05-3.15 (1H, m), 3.3-3.45 (2H, m), 3.6-3.7 (2H, m), 4.0-4.15 (1H, m), 4.3-4.45 (1H, m), 5.70 (1H, s),

7.13 (1H, t, J=6.9Hz), 7.05-7.4 (18H, m).

Example 16

N-(4-Propylphenyl)-1-(2-dimethylaminoethyl)-3-(diphenylmethyl)indole-2-carboxamide (Compound 16)

$^1$H-NMR($CDCl_3$) δ(ppm): 0.93 (3H, t, J=7.4Hz), 1.55-1.7 (2H, m), 2.16 (6H, s), 2.55 (2H, t, J=7.6Hz), 2.82 (2H, t, J=6.2Hz), 4.48 (2H, t, J=6.2Hz), 6.25 (1H, s), 6.85-7.0 (2H, m), 7.13 (2H, d, J=8.6Hz), 7.2-7.4 (14H, m), 9.47 (1H, br s).

Example 17

N-(2-Dimethylaminoethyl)-1-(2-dimethylaminoethyl)-3-(diphenylmethyl)indole-2-carboxamide (Compound 17)

$^1$H-NMR($CDCl_3$) δ(ppm): 2.16 (6H, s), 2.27 (6H, s), 2.41 (2H, t, J=6.1Hz), 2.75-2.85 (2H, m), 3.47 (2H, q, J=6.0Hz), 4.46 (2H, t, J=6.7Hz), 6.13 (1H,s), 6.8-6.9 (1H, m), 6.96 (1H, d, J=7.9Hz), 7.15-7.3 (11H, m), 7.36 (1H, d, J=8.6Hz).

Example 18

N-(2-Dimethylaminoethyl)-N-methyl-1-(2-dimethylaminoethyl)-3-(diphenylmethyl)indole-2-carboxamide (Compound 18)

$^1$H-NMR($CDCl_3$) δ(ppm): 2.27 (12H, s), 2.4-2.5 (2H, m), 2.51 (3H, s), 2.5-2.7 (2H, m), 3.45-3.55 (2H, m), 4.05-4.2 (1H, m), 4.2-4.35 (1H, m), 5.76 (1H, s), 6.9-7.0 (1H, m), 7.1-7.3 (13H, m).

Example 19

N-(2-Piperidinoethyl)-1-(2-dimethylaminoethyl)-3-(diphenylmethyl)indole-2-carboxamlde (Compound 19)

$^1$H-NMR($CDCl_3$) δ(ppm): 1.3-1.5 (6H, m), 2.20 (6H, s), 2.25-2.35 (4H, m), 2.41 (2H, t, J=6.3Hz), 2.74 (2H, t, J=6.7Hz), 3.4-3.5 (2H, m), 4.44 (2H, t, J=6.7Hz), 6.14 (1H, s), 6.8-6.9 (1H, m), 6.99 (1H, d, J=7.9Hz), 7.15-7.3 (12H, m), 7.33 (1H, d, J=8.6Hz).

Example 20

N-(3-Morpholinopropyl)-1-(2-dimethylaminoethyl)-3-(diphenylmethyl)indole-2-carboxamide (Compound 20)

$^1$H-NMR($CDCl_3$) δ(ppm): 1.55-1.7 (2H, m), 2.2-2.35 (6H, m), 2.26 (6H, s), 2.75-2.9 (2H, br), 3.41 (2H, q, J=6.6Hz), 3.55-3.65 (4H, m), 4.44 (2H, t, J=6.6Hz), 6.12 (1H, s), 6.8-6.95 (2H, m), 7.1-7.3 (11H, m), 7.3-7.4 (2H, m).

Example 21

N-[3-(2-Oxopyrrolidinyl)propyl]-1-(2-dimethylaminoethyl)-3-(diphenylmethyl)indole-2-carboxamide (Compound 21)

$^1$H-NMR($CDCl_3$) δ(ppm): 1.65-1.8 (2H, m), 1.9-2.1 (2H, m), 2.18 (6H, s), 2.36 (2H, t, J=8.1Hz), 2.79 (2H, t, J=6.4Hz), 3.24 (2H, t, J=7.1Hz), 3.3-3.4 (4H, m), 4.40 (2H, t, J=6.4Hz), 6.19 (1H, s), 6.85-6.9 (1H, m), 6.95 (1H, d, J=7.6Hz), 7.15-7.3 (11H, m), 7.31 (1H, d, J=8.2Hz), 7.9-8.0 (1H, m).

Example 22

N-[3-(1-Imidazolyl)propyl]-1-(2-dimethylaminoethyl)-3-(diphenylmethyl)indole-2-carboxamide (Compound 22)

$^1$H-NMR($CDCl_3$) δ(ppm): 1.85-2.0 (2H, m), 2.19 (6H, s), 2.8-2.9 (2H, m), 3.33 (2H, q, J=6.6Hz), 3.80 (2H, t, J=6.9Hz), 4.40 (2H, t, J=5.7Hz), 6.23 (1H, s), 6.84 (1H, s), 6.89 (2H, d, J=3.6Hz), 7.05 (1H, s), 7.15-7.4 (13H, m), 7.85-8.0 (1H, br).

Example 23

N-(2-Pyridylmethyl)-1-(2-dimethylaminoethyl)-3-(diphenylmethyl)indole-2-carboxamide (Compound 23)

$^1$H-NMR($CDCl_3$) δ(ppm): 2.15 (6H, s), 2.82 (2H, t, J=6.5Hz), 4.48 (2H, t, J=6.5Hz), 4.67 (2H, d, J=5.4Hz), 6.28 (1H, s), 6.89 (1H, t, J=8.4Hz), 6.98 (1H, d, J=8.4Hz), 7.1-7.3 (13H, m), 7.34 (1H, d, J=8.4Hz), 7.60 (1H, td, J=7.9, 1.5Hz), 8.43 (1H, d, J=4.4Hz), 8.4-8.55 (1H, br).

Example 24

N-[2-(2-Pyridyl)ethyl]-1-(2-dimethylaminoethyl)-3-(diphenylmethyl)indole-2-carboxamide (Compound 24)

$^1$H-NMR($CDCl_3$) δ(ppm): 2.10 (6H, s), 2.7-2.8 (2H, m), 3.02 (2H, t, J=6.4Hz), 3.79 (2H, q, J=6.4Hz), 4.38 (2H, t, J=6.4Hz), 6.16 (1H, s), 6.8-7.0 (3H, m), 7.0-7.1 (1H, m), 7.1-7.3 (11H, m), 7.32 (1H, d, J=8.4Hz), 7.42 (1H, td, J=7.4, 2.0Hz), 7.9-8.1 (1H, br), 8.35 (1H, d, J=3.0Hz).

Example 25

N-[(1-Ethyl-2-pyrrolidinyl)methyl]-1-(2-dimethylaminoethyl)-3-(diphenylmethyl)indole-2-carboxamide (Compound 25)

$^1$H-NMR($CDCl_3$) δ(ppm): 0.95 (3H, t, J=7.3Hz), 1.35-1.6 (2H, m), 1.6-1.8 (2H, m), 2.0-2.2 (2H, m), 2.23 (6H, s), 2.4-2.5 (1H, m), 2.55-2.65 (3H, m), 2.9-3.0 (1H, m), 3.05-3.15 (1H, m), 3.65-3.75 (1H, m), 4.43 (2H, t, J=6.7Hz), 6.13 (1H, s), 6.8-6.9 (1H, m), 6.97 (1H, d, J=7.9Hz), 7.15-7.3 (12H, m), 7.33 (1H, d, J=8.6Hz).

Example 26

N-[2-(1-Methyl-2-pyrrolidinyl)ethyl]-1-(2-dimethylaminoethyl)-3-(diphenylmethyl)indole-2-carboxamide (Compound 26)

$^1$H-NMR($CDCl_3$) δ(ppm): 1.4-1.6 (2H, m), 1.6-2.0 (4H, m), 2.05-2.2 (2H, m), 2.21 (6H, s), 2.24 (3H, s), 2.78 (2H, d, J=6.4Hz), 3.0-3.1 (1H, m), 3.3-3.6 (2H, m), 4.42 (2H, t, J=6.4Hz), 6.18 (1H, s), 6.8-6.9 (1H, m), 6.95 (1H, d, J=7.9Hz), 7.15-7.3 (11H, m), 7.32 (1H, d, J=8.6Hz), 7.7-7.8 (1H, m).

Example 27

N-[(2-Tetrahydrofuranyl)methyl]-1-(2-dimethylaminoethyl)-3-(diphenylmethyl)indole-2-carboxamide (Compound 27)

$^1$H-NMR($CDCl_3$) δ(ppm): 1.4-1.55 (1H, m), 1.7-1.95 (3H, m), 2.19 (6H, s), 2.7-2.9 (2H, m), 3.35-3.45 (1H, m), 3.55-3.7 (3H, m), 3.9-4.0 (1H, m), 4.35-4.5 (2H, m), 6.22 (1H, s), 6.85-6.95 (1H, m), 6.97 (1H, d, J=7.9Hz), 7.15-7.3 (11H, m), 7.32 (1H, d, J=8.3Hz), 8.05-8.1 (1H, m).

Example 28

N-(2-Thienylmethyl)-1-(2-dimethylaminoethyl)-3-(diphenylmethyl)indole-2-carboxamide (Compound 28)

$^1$H-NMR($CDCl_3$) δ(ppm): 2.02 (6H, s), 2.75 (2H, t, J=6.3Hz), 4.41 (2H, t, J=6.3Hz), 4.73 (2H, d, J=5.3Hz), 6.23 (1H, s), 6.8-7.0 (4H, m), 7.15-7.35 (13H, m), 8.6-8.7 (1H, m).

Example 29

N-(2-Furylmethyl)-1-(2-dimethylaminoethyl)-3-(diphenylmethyl)indole-2-carboxamide (Compound 29)

$^1$H-NMR($CDCl_3$) δ(ppm): 2.06 (6H, s), 2.76 (2H, t, J=6.1Hz), 4.40 (2H, t, J=6.1Hz), 4.56 (2H, d, J=5.3Hz), 6.21 (1H, s), 6.2-6.25 (1H, m), 6.3-6.35 (1H, m), 6.85-6.95 (1H, m), 6.99 (1H, d, J=7.6Hz), 7.15-7.35 (12H, m), 7.19 (1H, br s), 8.5-8.6 (1H, m).

Example 30

N-[3-(2-Methylpiperidino)propyl]-1-(2-dimethylaminoethyl)-3-(diphenylmethyl)indole-2-carboxamide (Compound 30)

$^1$H-NMR($CDCl_3$) δ(ppm): 1.03 (3H, d, J=6.3Hz), 1.2-1.35 (2H, m), 1.5-1.8 (6H, m), 2.15-2.25 (1H, m), 2.21 (6H, s), 2.3-2.45 (2H, m), 2.7-2.9 (2H, m), 2.79 (2H, t, J=6.4Hz), 3.38 (2H, q, J=6.4Hz), 4.41 (2H, t, J=6.4Hz), 6.17 (1H, s), 6.85-6.95 (1H, m), 6.97 (1H, d, J=7.6Hz), 7.15-7.3 (11H, m), 7.31 (1H, d, J=8.3Hz), 7.85-8.05 (1H, br).

Example 31

3-(Diphenylmethyl)-1-(2-pyrrolidinylethyl)indole-2-carboxylic acid (Compound 31)

Substantially the same procedure as in Example 2 was repeated using ethyl 3-(diphenylmethyl)indole-2-carboxylate (5.0 g, 14.1 mmol) obtained in Reference Example 1 and 2-pyrrolidinylethylchloride hydrochloride (2.63 g, 15.5 mmol) to give 7.05 g of ethyl 3-(diphenylmethyl)-1-(2-pyrrolidinylethyl)indole-2-carboxylate. Then, substantially the same procedure as in Example 6 was repeated using the obtained compound to give 4.7 g (yield: 79%, 2 steps) of the title compound.

$^1$H-NMR(DMSO-$d_6$) δ(ppm): 1.7-1.85 (4H, br), 2.7-2.9 (4H, br), 3.48 (2H, t, J=5.7Hz), 4.77 (2H, t, J=5.7Hz), 6.77 (1H, s), 6.85-6.95 (1H, m), 7.05-7.3 (12H, m), 7.35 (1H, d, J=8.3Hz), 7.85 (1H, s).

In the following Examples 32 to 34, substantially the same procedure as in Example 7 was repeated using Compound 31 in place of Compound 6 and using corresponding amines in place of propylamine to give the desired compounds.

Example 32

N-Isopropyl-3-(diphenylmethyl)-1-(2-pyrrolidinylethyl)indole-2-carboxamide (Compound 32)

$^1$H-NMR($CDCl_3$) δ(ppm): 1.11 (6H, d, J=6.6Hz), 1.6-1.75 (4H, br), 2.25-2.4 (4H, br), 2.97 (2H, t, J=6.6Hz), 4.15-4.3 (1H, m), 4.47 (2H, t, J=6.6Hz), 6.15 (1H, s), 6.8-6.95 (2H, m), 7.15-7.3 (11H, m), 7.35 (1H, d, J=8.3Hz), 7.55-7.7 (1H, br).

Example 33

2-(4-Benzylpiperazinylcarbonyl)-3-(diphenylmethyl)-1-(2-pyrrolidinylethyl)indole (Compound 33)

$^1$H-NMR($CDCl_3$) δ(ppm): 1.6-1.7 (1H, m), 1.75-1.85 (4H, m), 1.95-2.05 (1H, m), 2.25-2.5 (2H, m), 2.55-2.65 (4H, m), 2.7-3.0 (3H, m), 3.05-3.15 (1H, m), 3.3-3.45 (2H, m), 3.65-3.75 (2H, m), 4.05-4.2 (1H, m), 4.25-4.4 (1H, m), 5.72 (1H, s), 6.85-6.95 (1H, m), 7.08 (1H, d, J=8.3Hz), 7.1-7.35 (16H, m), 7.35 (1H, d, J=8.3Hz).

Example 34

N-(3-Morpholinopropyl)-3-(diphenylmethyl)-1-(2-pyrrolidinylethyl)indole-2-carboxamide (Compound 34)

$^1$H-NMR($CDCl_3$) δ(ppm): 1.6-1.75 (6H, m), 2.25-2.5 (10H, m), 2.99 (2H, t, J=6.6Hz), 3.39 (2H, td, J=6.9, 5.9Hz), 3.55-3.65 (4H, m), 4.45 (2H, t, J=6.6Hz), 6.17 (1H, s), 6.85-6.95 (1H, m), 6.97 (1H, d, J=7.6Hz), 7.15-7.3 (11H, m), 7.34 (1H, d, J=8.3Hz), 7.95-8.05 (1H, br).

Example 35

3-(Diphenylmethyl)-1-(2-piperidinoethyl)indole-2-carboxylic acid (Compound 35)

Substantially the same procedure as in Example 2 was repeated using ethyl 3-(diphenylmethyl)indole-2-carboxylate (5.0 g, 14.1 mmol) obtained in Reference Example 1 and 2-piperidinoethylchloride hydrochloride (2.85 g, 15.5 mmol) to give 7.20 g of ethyl 3-(diphenylmethyl)-1-(2-piperidinoethyl)indole-2-carboxylate. Then, substantially the same procedure as in Example 6 was repeated using the obtained compound to give 5.3 g (yield: 85%, 2 steps) of the title compound.

$^1$H-NMR($CDCl_3$) δ(ppm): 1.4-1.65 (6H, m), 2.3-2.6 (4H, m), 3.12 (2H, t, J=5.4Hz), 4.72 (2H, t, J=5.4Hz), 6.83 (1H, s), 6.85-7.0 (1H, m), 7.1-7.35 (13H, m).

In the following Examples 36 to 38, substantially the same procedure as in Example 7 was repeated using Compound 35 in place of Compound 6 and using corresponding amines in place of propylamine to give the desired compounds.

Example 36

N-Isopropyl-3-(diphenylmethyl)-1-(2-piperidinoethyl)indole-2-carboxamide (Compound 36)

$^1$H-NMR($CDCl_3$) δ(ppm): 1.12 (6H, d, J=6.6Hz), 1.3-1.45 (2H, m), 1.45-1.55 (4H, m), 2.25-2.35 (4H, m), 2.74 (2H, t, J=6.9Hz), 4.15-4.3 (1H, m), 4.49 (2H, t, J=6.9Hz), 6.08 (1H, s), 6.75-6.9 (3H, m), 7.15-7.3 (11H, m), 7.36 (1H, d, J=8.3Hz).

Example 37

2-(4-Benzylpiperazinylcarbonyl)-3-(diphenylmethyl)-1-(2-piperidinoethyl)indole (Compound 37)

$^1$H-NMR($CDCl_3$) δ(ppm): 1.4-1.5 (2H, m), 1.5-1.65 (4H, m), 1.65-1.8 (2H, m), 1.95-2.05 (1H, m), 2.25-2.4 (1H, m), 2.4-2.65 (5H, m), 2.7-2.9 (2H, m), 3.05-3.15 (1H, m), 3.25-3.35 (2H, m), 3.6-3.8 (2H, m), 4.05-4.2 (1H, m), 4.25-4.4 (1H, m), 5.71 (1H, s), 6.91 (1H, t, J=7.3Hz), 7.08 (1H, d, J=7.9Hz), 7.15-7.3 (16H, m), 7.35 (1H, d, J=8.3Hz).

Example 38

N-(3-Morpholinopropyl)-3-(diphenylmethyl)-1-(2-piperidinoethyl)indole-2-carboxamide (Compound 38)

$^1$H-NMR($CDCl_3$) δ(ppm): 1.3-1.55 (6H, m), 1.65-1.75 (2H, m), 2.2-2.4 (10H, m), 2.80 (2H, t, J=6.4Hz), 3.42 (2H, td, J=6.9, 6.3Hz), 3.6-3.7 (4H, m), 4.45 (2H, t, J=6.4Hz), 6.17 (1H, s), 6.85-6.95 (1H, m), 6.96 (1H, d, J=7.6Hz), 7.15-7.3 (11H, m), 7.34 (1H, d, J=8.6Hz), 8.05-8.15 (1H, br).

Example 39

3-(Diphenylmethyl)-1-(2-morpholinoethyl)indole-2-carboxylic acid (Compound 39)

Substantially the same procedure as in Example 2 was repeated using ethyl 3-(diphenylmethyl)indole-2-carboxylate (5.0 g, 14.1 mmol) obtained in Reference Example 1 and 2-morpholinoethylchloride hydrochloride (2.88 g, 15.5 mmol) to give 7.24 g of ethyl 3-(diphenylmethyl)-1-(2-morpholinoethyl)indole-2-carboxylate. Then, substantially the same procedure as in Example 6 was repeated using the obtained compound to give 5.6 g (yield: 88%, 2 steps) of the title compound.

$^1$H-NMR($CDCl_3$) δ(ppm): 2.3-2.45 (4H, br), 3.03 (2H, t, J=5.6Hz), 3.4-3.5 (4H, m), 4.66 (2H, t, J=5.6Hz), 6.68 (1H, s), 6.9-7.0 (1H, m), 7.06 (1H, d, J=8.2Hz), 7.15-7.3 (12H, m).

In the following Examples 40 to 42, substantially the same procedure as in Example 7 was repeated using Compound 39 in place of Compound 6 and using corresponding amines in place of propylamine to give the desired compounds.

Example 40

N-Isopropyl-3-(diphenylmethyl)-1-(2-morpholinoethyl)indole-2-carboxamide (Compound 40)

$^1$H-NMR($CDCl_3$) δ(ppm): 1.10 (6H, d, J=6.6Hz), 2.25-2.35 (4H, m), 2.76 (2H, t, J=7.1Hz), 3.55-3.65 (4H, m), 4.15-4.3 (1H, m), 4.51 (2H, t, J=7.1Hz), 6.00 (1H, s), 6.11 (1H, br d, J=7.6Hz), 6.75-6.9 (2H, m), 7.15-7.35 (11H, m), 7.36 (1H, d, J=8.2Hz).

Example 41

2-(4-Benzylpiperazinylcarbonyl)-3-(diphenylmethyl)-1-(2-morpholinoethyl)indole (Compound 41)

$^1$H-NMR($CDCl_3$) δ(ppm): 1.6-1.7 (1H, m), 1.9-2.0 (1H, m), 2.3-2.65 (7H, m), 2.75-2.9 (2H, m), 3.05-3.15 (1H, m), 3.25-3.45 (2H, m), 3.65-3.75 (6H, m), 4.0-4.15 (1H, m), 4.25-4.4 (1H, m), 5.71 (1H, s), 6.85-6.95 (1H, m), 7.05-7.4 (18H, m).

Example 42

N-(3-Morpholinopropyl)-3-(diphenylmethyl)-1-(2-morpholinoethyl)indole-2-carboxamide (Compound 42)

$^1$H-NMR($CDCl_3$) δ(ppm): 1.6-1.75 (2H, m), 2.25-2.45 (10H, m), 2.77 (2H, t, J=6.9Hz), 3.35-3.45 (2H, m), 3.55-3.65 (8H, m), 4.48 (2H, t, J=6.9Hz), 6.05 (1H, s), 6.8-6.95 (3H, m), 7.15-7.35 (11H, m), 7.35 (1H, d, J=8.3Hz).

Example 43

Ethyl 3-[bis(4-fluorophenyl)methyl]-1-(2-dimethylaminoethyl)indole-2-carboxylate (Compound 43)

Substantially the same procedure as in Example 2 was repeated using ethyl 3-[bis(4-fluorophenyl)methyl]indole-2-carboxylate (5.0 g, 12.8 mmol) obtained in Reference Example 2 and 2-dimethylaminoethylchloride hydrochloride (1.93 g, 13.4 mmol) to give 6.8 g (quantitative) of the title compound.

$^1$H-NMR($CDCl_3$) δ(ppm): 1.46 (3H, t, J=7.2Hz), 2.43 (6H, s), 2.7-2.8 (2H, m), 4.46 (2H, q, J=7.2Hz), 4.65-4.75 (2H, m), 6.59 (1H, s), 6.95-7.1 (6H, m), 7.15-7.25 (4H, m), 7.35-7.4 (1H, m), 7.50 (1H, d, J=8.5Hz).

Example 44

3-[Bis(4-fluorophenyl)methyl]-1-(2-dimethylaminoethyl)indole-2-carboxylic acid (Compound 44)

Substantially the same procedure as in Example 6 was repeated using Compound 43 (6.8 g, 12.8 mmol) obtained in Example 43 to give 3.6 g (yield: 65%) of the title compound.

$^1$H-NMR(DMSO-$d_6$) δ(ppm): 2.45 (6H, s), 3.28 (2H, t, J=5.6Hz), 4.73 (2H, t, J=5.6Hz), 6.74 (1H, s), 6.85-6.95 (1H, m), 6.97 (1H, d, J=7.9Hz), 7.0-7.2 (9H, m), 7.54 (1H, d, J=8.3Hz).

In the following Examples 45 to 47, substantially the same procedure as in Example 7 was repeated using Compound 44 in place of Compound 6 and using corresponding amines in place of propylamine to give the desired compounds.

Example 45

N-Isopropyl-3-[bis(4-fluorophenyl)methyl]-1-(2-dimethylaminoethyl)indole-2-carboxamide (Compound 45)

$^1$H-NMR($CDCl_3$) δ(ppm): 1.15 (6H, d, J=6.6Hz), 2.12 (6H, s), 2.78 (2H, t, J=6.4Hz), 4.15-4.3 (1H, m), 4.42 (2H, t, J=6.4Hz), 6.10 (1H, s), 6.9-7.0 (6H, m), 7.15-7.25 (5H, m), 7.33 (1H, d, J=8.3Hz), 7.59 (1H, br d, J=7.3Hz).

Example 46

2-(4-Benzylpiperazinylcarbonyl)-3-[bis(4-fluorophenyl)methyl]-1-(2-dimethylaminoethyl)indole (Compound 46)

$^1$H-NMR($CDCl_3$) δ(ppm): 1.65-1.75 (1H, m), 2.0-2.1 (1H, m), 2.28 (6H, s), 2.3-2.4 (1H, m), 2.45-2.55 (1H, m), 2.55-2.75 (2H, m), 2.85-2.95 (1H, m), 3.05-3.15 (1H, m), 3.3-3.45 (2H, m), 3.55-3.65 (2H, m), 4.0-4.15 (1H, m), 4.25-4.4 (1H, m), 5.66 (1H, s), 6.85-7.15 (8H, m), 7.2-7.4 (9H, m).

Example 47

N-(3-Morpholinopropyl)-3-[bis(4-fluorophenyl)methyl]-1-(2-dimethylaminoethyl)indole-2-carboxamide (Compound 47)

$^1$H-NMR($CDCl_3$) δ(ppm): 1.65-1.85 (2H, m), 2.17 (6H, s), 2.25-2.4 (6H, m), 2.81 (2H, t, J=5.9Hz), 3.42 (2H, q, J=6.9Hz), 3.6-3.7 (4H, m), 4.38 (2H, t, J=5.9Hz), 6.17 (1H, s), 6.85-7.0 (6H, m), 7.1-7.25 (5H, m), 7.31 (1H, d, J=8.3Hz), 8.25-8.35 (1H, m).

Example 48

N-[3-(2-Oxopyrrolidinyl)propyl]-1-(2-dimethylaminoethyl)-3-(diphenylmethyl)indole-2-carboxamide hydrochloride (Compound 21 hydrochloride)

A solution of hydrogen chloride in 10 ml of ethyl acetate was added to a suspension of Compound 21 (12.85 g) obtained in Example 21 in 70 ml of ethanol, followed by stirring. Then, ethanol (45 ml) was added to the reaction solution followed by recrystallization to give 12.2 g (yield: 89%) of the title compound.

$^1$H-NMR($CDCl_3$) δ(ppm): 1.55-1.65 (2H, m), 1.95-2.05 (2H, m), 2.33 (2H, t, J=8.1Hz), 2.88 (3H, s), 2.90 (3H, s), 3.18 (2H, t, J=6.9Hz), 3.25-3.35 (4H, m), 3.45-3.55 (2H, m), 4.85-4.95 (2H, m), 5.97 (1H, s), 6.05 (1H, t, J=5.6Hz), 6.79 (1H, d, J=7.9Hz), 6.89 (1H, t, J=7.1Hz), 7.1-7.2 (4H, m), 7.2-7.35 (7H, m), 7.78 (1H, d, J=8.3Hz), 12.93 (1H, br).

In the following Examples 49 to 61, substantially the same procedure as in Example 7, and then substantially the same procedure as in Example 48 were repeated using corresponding amines in place of propylamine to give the desired compounds.

Example 49

N-Butyl-1-(2-dimethylaminoethyl)-3-(diphenylmethyl)indole-2-carboxamide hydrochloride (Compound 48 hydrochloride)

$^1$H-NMR($CDCl_3$) δ(ppm): 0.88 (3H, t, J=7.3Hz), 1.15-1.3 (2H, m), 1.3-1.45 (2H, m), 2.91 (6H, s), 3.29 (2H, dt, J=5.6, 6.9Hz), 3.55-3.65 (2H, m), 4.8-4.9 (2H, m), 5.76 (1H, t, J=5.6Hz), 5.95 (1H, s), 6.77 (1H, d, J=8.2Hz), 6.88 (1H, t, J=6.5Hz), 7.1-7.2 (4H, m), 7.2-7.35 (7H, m), 7.79 (1H, d, J=8.6Hz), 13.0 (1H, br).

Example 50

N-Allyl-1-(2-dimethylaminoethyl)-3-(diphenylmethyl)indole-2-carboxamide hydrochloride (Compound 49 hydrochloride)

$^1$H-NMR($CDCl_3$) δ(ppm): 2.90 (3H, s), 2.92 (3H, s), 3.55-3.65 (2H, m), 3.92 (2H, t, J=5.7Hz), 4.8-4.9 (2H, m), 5.10 (1H, d, J=12.5Hz), 5.15 (1H, d, J=5.3Hz), 5.7-5.85 (1H, m), 5.85-5.9 (1H, m), 5.96 (1H, s), 6.80 (1H, d, J=7.9Hz), 6.90 (1H, t, J=7.5Hz), 7.1-7.2 (4H, m), 7.2-7.4 (7H, m), 7.80 (1H, d, J=8.6Hz), 13.04 (1H, br).

Example 51

N-Isobutyl-1-(2-dimethylaminoethyl)-3-(diphenylmethyl)indole-2-carboxamide hydrochloride (Compound 50 hydrochloride)

$^1$H-NMR($CDCl_3$) δ(ppm): 0.78 (6H, d, J=6.6Hz), 1.5-1.7 (1H, m), 2.90 (3H, s), 2.92 (3H, s), 3.13 (2H, t, J=6.4Hz), 3.55-3.65 (2H, m), 4.8-4.9 (2H, m), 5.75-5.85 (1H, m), 5.97 (1H, s), 6.78 (1H, d, J=8.2Hz), 6.88 (1H, t, J=7.6Hz), 7.05-7.2 (4H, m), 7.2-7.35 (7H, m), 7.79 (1H, d, J=8.6Hz), 13.03 (1H, br).

Example 52

N-Cyclopropyl-1-(2-dimethylaminoethyl)-3-(diphenylmethyl)indole-2-carboxamide hydrochloride (Compound 51 hydrochloride)

$^1$H-NMR($CDCl_3$) δ(ppm): 0.35-0.45 (2H, m), 0.75-0.85 (2H, m), 2.75-2.85 (1H, m), 2.91 (3H, s), 2.93 (3H, s), 3.55-3.65 (2H, m), 4.85-4.95 (2H, m), 5.86 (2H, s), 6.74 (1H, d, J=7.9Hz), 6.88 (1H, t, J=7.1Hz), 7.05-7.15 (4H, m), 7.2-7.4 (7H, m), 7.82 (1H, d, J=7.9Hz), 13.06 (1H, br).

Example 53

N-Cyclobutyl-1-(2-dimethylaminoethyl)-3-(diphenylmethyl)indole-2-carboxamide hydrochloride (Compound 52 hydrochloride)

$^1$H-NMR($CDCl_3$) δ(ppm): 1.55-1.75 (4H, m), 2.25-2.35 (2H, m), 2.90 (3H, s), 2.91 (3H, s), 3.55-3.65 (2H, m), 4.35-4.45 (1H, m), 4.75-4.85 (2H, m), 5.95-6.0 (1H, m), 5.96 (1H, s), 6.75 (1H, d, J=7.9Hz), 6.88 (1H, t, J=7.4Hz), 7.1-7.2 (4H, m), 7.25-7.4 (7H, m), 7.79 (1H, d, J=8.6Hz), 13.02 (1H, br).

Example 54

N-(sec-Butyl)-1-(2-dimethylaminoethyl)-3-(diphenylmethyl)indole-2-carboxamide hydrochloride (Compound 53 hydrochloride)

$^1$H-NMR($CDCl_3$) δ(ppm): 0.82 (3H, t, J=6.9Hz), 1.06 (3H, d, J=6.6Hz), 1.25-1.4 (2H, m), 2.91 (6H, s), 3.55-3.65 (2H, m), 3.95-4.05 (1H, m), 4.85-4.95 (2H, m), 5.63 (1H, d, J=7.9Hz), 5.95 (1H, s), 6.73 (1H, d, J=8.3Hz), 6.87 (1H, t,

J=7.6Hz), 7.05-7.15 (4H, m), 7.2-7.4 (7H, m), 7.79 (1H, d, J=7.8Hz), 13.05 (1H, br).

Example 55

N-Cyclopentyl-1-(2-dimethylaminoethyl)-3-(diphenylmethyl)indole-2-carboxamide hydrochloride (Compound 54 hydrochloride)

$^1$H-NMR($CDCl_3$) δ(ppm): 1.1-1.25 (2H, m), 1.5-1.75 (4H, m), 1.85-2.0 (2H, m), 2.91 (6H, s), 3.55 -3.65 (2H, m), 4.2-4.3 (1H, m), 4.8-4.9 (2H, m), 5.78 (1H, d, J=7.3Hz), 5.94 (1H, s), 6.73 (1H, d, J=7.2Hz), 6.87 (1H, t, J=6.7Hz), 7.05-7.15 (4H, m), 7.25-7.4 (7H, m), 7.78 (1H, d, J=8.6Hz), 13.0 (1H, br).

Example 56

N-Cyclohexyl-1-(2-dimethylaminoethyl)-3-(diphenylmethyl)indole-2-carboxamide hydrochloride (Compound 55 hydrochloride)

$^1$H-NMR($CDCl_3$) δ(ppm): 0.9-1.05 (2H, m), 1.05-1.2 (1H, m), 1.2-1.45 (3H, m), 1.55-1.7 (2H, m), 1.85-1.95 (2H, m), 2.90 (3H, s), 2.92 (3H, s), 3.55-3.65 (2H, m), 3.75 (1H, m), 4.75-4.85 (2H, m), 5.69 (1H, d, J=7.9Hz), 5.93 (1H, s), 6.75 (1H, d, J=7.9Hz), 6.87 (1H, t, J=7.6Hz), 7.05-7.15 (4H, m), 7.2-7.35 (7H, m), 7.78 (1H, d, J=8.2Hz), 13.03 (1H, br).

Example 57

N-Methyl-1-(2-dimethylaminoethyl)-3-(diphenylmethyl)indole-2-carboxamide hydrochloride (Compound 56 hydrochloride)

$^1$H-NMR($CDCl_3$) δ(ppm): 2.83 (3H, d, J=5.0Hz), 2.90 (3H, s), 2.92 (3H, s), 3.5-3.6 (2H, m), 4.75-4.85 (2H, m), 5.7-5.8 (1H, m), 5.92 (1H, s), 6.83 (1H, d, J=7.9Hz), 6.91 (1H, t, J=7.3Hz), 7.05-7.15 (4H, m), 7.2-7.35 (7H, m), 7.79 (1H, d, J=8.6Hz), 13.03 (1H, br).

Example 58

N-Pentyl-1-(2-dimethylaminoethyl)-3-(diphenylmethyl)indole-2-carboxamide hydrochloride (Compound 57 hydrochloride)

$^1$H-NMR($CDCl_3$) δ(ppm): 0.86 (3H, t, J=7.3Hz), 1.05-1.45 (6H, m), 2.90 (3H, s), 2.92 (3H, s), 3.29 (2H, dt, J=5.6, 7.0Hz), 3.55-3.65 (2H, m), 4.8-4.9 (2H, m), 5.76 (1H, t, J=5.6Hz), 5.95 (1H, s), 6.78 (1H, d, J=8.2Hz), 6.89 (1H, t, J=7.1Hz), 7.1-7.2 (4H, m), 7.2-7.35 (7H, m), 7.79 (1H, d, J=8.6Hz), 13.04 (1H, br).

Example 59

N-Ethyl-1-(2-dimethylaminoethyl)-3-(diphenylmethyl)indole-2-carboxamide hydrochloride (Compound 58 hydrochloride)

$^1$H-NMR($CDCl_3$) δ(ppm): 1.07 (3H, t, J=7.3Hz), 2.90 (3H, s), 2.92 (3H, s), 3.33 (2H, dq, J=5.3, 7.3Hz), 3.55-3.65 (2H, m), 4.75-4.9 (2H, m), 5.75 (1H, t, J=5.3Hz), 5.94 (1H, s), 6.78 (1H, d, J=8.2Hz), 6.89 (1H, t, J=7.6Hz), 7.05-7.15 (4H, m), 7.2-7.35 (7H, m), 7.79 (1H, d, J=7.9Hz), 13.0 (1H, br).

Example 60

N-(4-Methoxyphenyl)-1-(2-dimethylaminoethyl)-3-(diphenylmethyl)indole-2-carboxamide hydrochloride (Compound 59 hydrochloride)

$^1$H-NMR($CDCl_3$) δ(ppm): 2.90 (3H, s), 2.91 (3H, s), 3.55-3.65 (2H, m), 3.80 (3H, s), 4.85-4.95 (2H, m), 6.07 (1H, s), 6.75-6.95 (4H, m), 7.1-7.25 (6H, m), 7.25-7.4 (7H, m), 7.46 (1H, s), 7.85 (1H, d, J=8.6Hz), 13.09 (1H, br).

Example 61

N-Phenyl-1-(2-dimethylaminoethyl)-3-(diphenylmethyl)indole-2-carboxamide hydrochloride (Compound 60 hydrochloride)

$^1$H-NMR($CDCl_3$) δ(ppm): 2.89 (6H, s), 3.55-3.65 (2H, m), 4.8-4.9 (2H, m), 6.06 (1H, s), 6.8-6.95 (2H, m), 7.1-7.4 (16H, m), 7.81 (1H, d, J=8.6Hz), 8.42 (1H, s), 12.47 (1H, br).

In the following Examples 62 to 64, substantially the same procedure as in Example 7, and then substantially the same procedure as in Example 48 were repeated using Compound 31 in place of Compound 6 and using corresponding amines in place of propylamine to give the desired compounds.

Example 62

N-Propyl-3-(diphenylmethyl)-1-(2-pyrrolidinylethyl)indole-2-carboxamide hydrochloride (Compound 61 hydrochloride)

$^1$H-NMR($CDCl_3$) δ(ppm): 0.83 (3H, d, J=7.4Hz), 1.3-1.5 (2H, m), 2.0-2.3 (4H, m), 2.9-3.05 (2H, m), 3.2-3.3 (2H, m), 3.6-3.9 (4H, m), 4.75-4.85 (2H, m), 5.77 (1H, t, J=5.3Hz), 5.95 (1H, s), 6.78 (1H, d, J=8.3Hz), 6.88 (1H, t, J=7.6Hz), 7.05-7.2 (4H, m), 7.2-7.4 (7H, m), 7.81 (1H, d, J=8.6Hz), 12.86 (1H, br).

Example 63

N-(2-Piperidinoethyl)-3-(diphenylmethyl)-1-(2-pyrrolidinylethyl)indole-2-carboxamide 2 hydrochloride (Compound 62 2 hydrochloride)

$^1$H-NMR($CDCl_3$) δ(ppm): 1.75-1.9 (4H, m), 2.0-2.3 (6H, m), 2.55-2.9 (4H, m), 3.2-3.3 (2H, m), 3.55-3.8 (8H, m), 4.84 (2H, t, J=7.4Hz), 6.04 (1H, s), 6.9-6.95 (2H, m), 7.15-7.35 (11H, m), 7.46 (1H, d, J=8.6Hz), 7.81 (1H, t, J=5.9Hz), 11.54 (1H, br), 12.50 (1H, br).

Example 64

N-(4-Propylphenyl)-3-(diphenylmethyl)-1-(2-pyrrolidinylethyl)indole-2-carboxamide hydrochloride (Compound 63 hydrochloride)

$^1$H-NMR($CDCl_3$) δ(ppm): 0.93 (3H, t, J=7.4Hz), 1.5-1.65 (2H, m), 2.05-2.35 (4H, m), 2.56 (2H, t, J=7.6Hz), 2.9-3.05 (2H, m), 3.6-3.85 (4H, m), 4.85-4.95 (2H, m), 6.07 (1H, s), 6.79 (1H, d, J=8.2Hz), 6.90 (1H, t, J=7.4Hz), 7.05-7.2 (6H, m), 7.2-7.4 (9H, m), 7.48 (1H, s), 7.87 (1H, t, J=8.6Hz), 12.91 (1H, br).

Example 65

Ethyl 1-(3-dimethylaminopropyl)-3-(diphenylmethyl)indole-2-carboxylate (Compound 64)

Substantially the same procedure as in Example 2 was repeated using ethyl 3-(diphenylmethyl)indole-2-carboxylate (40.0 g, 113 mmol) obtained in Reference Example 1 and 3-dimethylaminopropylchloride hydrochloride (19.6 g, 124 mmol) to give 55.4 g (quantitative) of the title compound.

$^1$H-NMR($CDCl_3$) δ(ppm): 1.35 (3H, t, J=7.3Hz), 2.05-2.25 (2H, m), 2.39 (6H, s), 2.40-2.65 (2H, br), 4.33 (2H, q, J=7.3Hz), 4.56 (2H, t, J=6.9Hz), 6.57 (1H, s), 6.83-6.94 (2H, m), 7.16-7.43 (12H, m).

Example 66

1-(3-Dimethylaminopropyl)-3-(diphenylmethyl)indole-2-carboxylic acid (Compound 65)

Substantially the same procedure as in Example 6 was repeated using Compound 64 (55.4 g) obtained in Example 65 to give 48.3 g (yield: 94%) of the title compound.

$^1$H-NMR(DMSO-$d_6$) δ(ppm): 2.28 (2H, t, J=6.3Hz), 2.55 (6H, s), 2.68 (2H, t, J=5.9Hz), 4.59 (2H, t, J=6.3Hz), 6.74 (1H, s), 6.86 (1H, t, J=7.6Hz), 7.02 (1H, d, J=7.9Hz), 7.12-7.31 (11H, m), 7.52 (1H, d, J=8.3Hz).

In the following Examples 67 to 72, substantially the same procedure as in Example 7 was repeated using Compound 65 in place of Compound 6 and using corresponding amines in place of propylamine to give the desired compounds.

Example 67

N-Isopropyl-1-(3-dimethylaminopropyl)-3-(diphenylmethyl)indole-2-carboxamide (Compound 66)

$^1$H-NMR($CDCl_3$) δ(ppm): 1.07 (6H, d, J=6.6Hz), 1.94-2.05 (2H, m), 2.16 (6H, s), 2.23 (2H, t, J=6.9Hz), 4.14-4.26 (1H, m), 4.41 (2H, t, J=7.3Hz), 5.93 (1H, s), 5.96 (1H, s), 6.77-6.87 (2H, m), 7.15-7.38 (12H, m).

Example 68

N-Propyl-1-(3-dimethylaminopropyl)-3-(diphenylmethyl)indole-2-carboxamide (Compound 67)

$^1$H-NMR($CDCl_3$) δ(ppm): 0.83 (3H, t, J=7.3Hz), 1.38-1.51 (2H, m), 1.96-2.06 (2H, m), 2.15 (6H, s), 2.23 (2H, t, J=6.9Hz), 3.23 (2H, dt, J=6.9, 7.3Hz), 4.40 (2H, t, J=6.9Hz), 5.99 (1H, s), 6.37 (1H, t, J=5.6Hz), 6.84-6.86 (1H, m), 7.15-7.39 (12H, m).

Example 69

N-Cyclooctyl-1-(3-dimethylaminopropyl)-3-(diphenylmethyl)indole-2-carboxamide (Compound 68)

$^1$H-NMR($CDCl_3$) δ(ppm): 1.25-1.60 (14H, m), 1.94-2.04 (2H, m), 2.17 (6H, s), 2.24 (2H, t, J=6.9Hz), 4.06-4.19 (1H, m), 4.41 (2H, t, J=6.9Hz), 5.93 (1H, s), 5.97 (1H, s), 6.77-6.83 (2H, m), 7.15-7.39 (12H, m).

Example 70

N-(2-piperidinoethyl)-1-(3-dimethylaminopropyl)-3-(diphenylmethyl)indole-2-carboxamide (Compound 69)

$^1$H-NMR($CDCl_3$) δ(ppm): 1.30-1.45 (6H, m), 1.95-2.05 (2H, m), 2.16 (6H, s), 2.24 (2H, t, J=7.3Hz), 2.20-2.30 (4H, m), 2.38 (2H, t, J=6.3Hz), 3.44 (2H, dt, J=6.3, 5.9Hz), 4.39 (2H, t, J=6.9Hz), 6.05 (1H, s), 6.66 (1H, t, J=5.0Hz), 6.83-6.98 (2H, m), 7.15-7.39 (12H, m).

Example 71

N-(4-Propylphenyl)-1-(3-dimethylaminopropyl)-3-(diphenylmethyl)indole-2-carboxamide (Compound 70)

$^1$H-NMR($CDCl_3$) δ(ppm): 0.93 (3H, t, J=7.3Hz), 1.55-1.69 (2H, m), 1.99-2.09 (2H, m), 2.13 (6H, s), 2.22 (2H, t, J=6.9Hz), 2.55 (2H, t, J=7.6Hz), 4.47 (2H, t, J=7.3Hz), 6.09 (1H, s), 6.87 (2H, d, J=3.6Hz), 7.09-7.43 (17H, m), 7.99 (1H, s).

Example 72

N-[3-(2-Oxopyrrolidinyl)propyl]-1-(3-dimethylaminopropyl)-3-(diphenylmethyl)indole-2-carboxamide (Compound 71)

$^1$H-NMR($CDCl_3$) δ(ppm): 1.63-1.73 (2H, m), 1.95-2.15 (4H, m), 2.21 (6H, s), 2.25-2.40 (2H, br), 2.36 (2H, t, J=7.9Hz), 3.20-3.32 (4H, m), 3.36 (2H, t, J=6.9Hz), 4.38 (2H, t, J=6.9Hz), 6.02 (1H, s), 6.83-6.91 (2H, m), 7.16-7.38 (12H, m).

Example 73

Ethyl 3-(diphenylmethyl)-1-(3-pyrrolidinylpropyl)indole-2-carboxylate (Compound 72)

To a solution of ethyl 3-(diphenylmethyl)-1-(3-chloropropyl)indole-2-carboxylate (39.64 g, 91.8 mmol) obtained in Reference Example 3 in 400 ml of ethanol was added pyrrolidine (41.4 ml, 496 mmol) with stirring at room temperature, followed by heating under reflux for 20 hours. Water and a saturated aqueous solution of sodium bicarbonate were added to the reaction solution followed by extraction with ethyl acetate. The resulting organic layer was washed successively with water and a saturated aqueous solution of sodium chloride, and dried over magnesium sulfate, and the solvent was distilled off under reduced pressure to give 46.5 g (quantitative) of the title compound.

$^1$H-NMR($CDCl_3$) δ(ppm): 1.35 (3H, t, J=7.3Hz), 1.70-1.95 (4H, m), 1.74-1.79 (2H, m), 2.35-2.55 (6H, m), 4.33 (2H, q, J=6.9Hz), 4.56 (2H, t, J=6.9Hz), 6.58 (1H, s), 6.81-6.93 (2H, m), 7.16-7.28 (11H, m), 7.42 (1H, d, J=8.3Hz).

Example 74

3-(Diphenylmethyl)-1-(3-pyrrolidinylpropyl)indole-2-carboxylic acid (Compound 73)

Substantially the same procedure as in Example 6 was repeated using Compound 72 (46.5 g) obtained in Example 73 to give 34.1 9 (yield: 78%) of the title compound.

$^1$H-NMR($CDCl_3$) δ(ppm): 1.60-1.75 (4H, m), 2.25-2.40 (2H, m), 2.45-2.70 (4H, br), 2.70 (2H, t, J=5.6Hz), 4.62 (2H, t, J=5.6Hz), 6.77 (1H, s), 6.88-6.94 (1H, m), 7.09-7.32 (13H, m).

In the following Examples 75 to 80, substantially the same procedure as in Example 7 was repeated using Compound 73 in place of Compound 6 and using corresponding amines in place of propylamine to give the desired compounds.

Example 75

N-Isopropyl-3-(diphenylmethyl)-1-(3-pyrrolidinylpropyl)indole-2-carboxamide (Compound 74)

$^1$H-NMR($CDCl_3$) δ(ppm): 1.07 (6H, d, J=6.6Hz), 1.70-1.80 (4H, m), 2.01-2.12 (2H, m), 2.45-2.55 (6H, m), 4.13-4.26 (1H, m), 4.43 (2H, t, J=6.9Hz), 5.72 (1H, d, J=7.9Hz), 5.94 (1H, s), 6.76-6.86 (2H, m), 7.16-7.40 (12H, m).

Example 76

N-Propyl-3-(diphenylmethyl)-1-(3-pyrrolidinylpropyl)indole-2-carboxamide (Compound 75)

$^1$H-NMR($CDCl_3$) δ(ppm): 0.83 (3H, t, J=7.3Hz), 1.38-1.52 (2H, m), 1.65-1.75 (4H, m), 2.00-2.11 (2H, m), 2.35-2.45 (6H, m), 3.27 (2H, dt, J=6.9, 5.9Hz), 4.42 (2H, t, J=6.9Hz), 5.98 (1H, s), 6.38 (1H, t, J=5.3Hz), 6.81-6.88 (2H, m), 7.15-7.39 (12H, m).

Example 77

N-Cyclooctyl-3-(diphenylmethyl)-1-(3-pyrrolidinylpropyl)indole-2-carboxamide (Compound 76)

$^1$H-NMR($CDCl_3$) δ(ppm): 1.25-1.85 (18H, m), 1.99-2.10 (2H, m), 2.40-2.55 (6H, m), 4.07-4.17 (1H, m), 4.44 (2H, t, J=6.9Hz), 5.79 (1H, d, J=7.9Hz), 5.95 (1H, s), 6.76-6.85 (2H, m), 7.14-7.40 (12H, m).

Example 78

N-(2-Piperidinoethyl)-3-(diphenylmethyl)-1-(3-pyrrolidinylpropyl)indole-2-carboxamide (Compound 77)

$^1$H-NMR($CDCl_3$) δ(ppm): 1.30-1.45 (6H, m), 1.70-1.80 (4H, m), 2.04-2.12 (2H, m), 2.25-2.35 (4H, m), 2.39 (2H, t, J=6.3Hz), 2.40-2.50 (6H, m), 3.43 (2H, dt, J=6.3, 5.9Hz), 4.42 (2H, t, J=6.9Hz), 6.04 (1H, s), 6.71 (1H, t, J=5.3Hz), 6.83-6.98 (2H, m), 7.15-7.39 (12H, m).

Example 79

N-(4-Propylphenyl)-3-(diphenylmethyl)-1-(3-pyrrolidinylpropyl)indole-2-carboxamide (Compound 78)

$^1$H-NMR($CDCl_3$) δ(ppm): 0.93 (3H, t, J=7.3Hz), 1.55-1.75 (6H, m), 2.03-2.14 (2H, m), 2.35-2.50 (6H, m), 2.55 (2H, t, J=7.3Hz), 4.49 (2H, t, J=7.3Hz), 6.08 (1H, s), 6.84-6.90 (2H, m), 7.08-7.43 (16H, m), 7.88 (1H, s).

Example 80

N-[3-(2-Oxopyrrolidinyl)propyl]-3-(diphenylmethyl)-1-(3-pyrrolidinylpropyl)indole-2-carboxamide (Compound 79)

$^1$H-NMR($CDCl_3$) δ(ppm): 1.50-1.90 (8H, m), 1.95-2.25 (4H, m), 2.30-2.65 (4H, br), 2.36 (2H, t, J=7.9Hz), 3.20-3.31 (4H, m), 3.37 (2H, t, J=6.9Hz), 4.40 (2H, t, J=6.9Hz), 6.01 (1H, s), 6.83-6.92 (2H, m), 7.16-7.38 (12H, m).

Example 81

Ethyl 1-(4-dimethylaminobutyl)-3-(diphenylmethyl)indole-2-carboxylate (Compound 80)

Substantially the same procedure as in Example 73 was repeated using ethyl 1-(4-chlorobutyl)-3-(diphenylmethyl)indole-2-carboxylate (39.84 g, 89.3 mmol) obtained in Reference Example 4 and an aqueous solution of dimethylamine (50%, 90 ml, 893 mmol) to give 29.2 g (yield: 72%) of the title compound.

$^1$H-NMR($CDCl_3$) δ(ppm): 1.35 (3H, t, J=6.9Hz), 1.40-1.60 (2H, m), 1.70-1.90 (2H, m), 2.18 (6H, s), 2.26 (2H, t, J=7.6Hz), 4.34 (2H, q, J=7.3Hz), 4.50 (2H, t, J=7.6Hz), 6.57 (1H, s), 6.81-6.93 (2H, m), 7.16-7.38 (12H, m).

Example 82

1-(4-Dimethylaminobutyl)-3-(diphenylmethyl)indole-2-carboxylic acid (Compound 81)

Substantially the same procedure as in Example 6 was repeated using Compound 80 (29.2 g) obtained in Example 81 to give 22.8 g (yield: 83%) of the title compound.

$^1$H-NMR($CDCl_3$) δ(ppm): 1.50-1.70 (2H, m), 1.75-1.95 (2H, m), 2.42 (6H, s), 2.74 (2H, t, J=7.6Hz), 4.69 (2H, t, J=6.3Hz), 6.79 (1H, s), 6.85 (1H, t, J=6.9Hz), 7.01 (1H, d, J=8.3Hz), 7.10-7.28 (12H, m).

In the following Examples 83 to 87, substantially the same procedure as in Example 7 was repeated using Compound 81 in place of Compound 6 and using corresponding amines in place of propylamine to give the desired compounds.

Example 83

N-Isopropyl-1-(4-dimethylaminobutyl)-3-(diphenylmethyl)indole-2-carboxamide (Compound 82)

$^1$H-NMR($CDCl_3$) δ(ppm): 1.69 (6H, d, J=6.6Hz), 1.42-1.53 (2H, m), 1.76-1.88 (2H, m), 2.17 (6H, s), 2.25 (2H, t, J=7.3Hz), 4.13-4.26 (1H, m), 4.38 (2H, t, J=7.6Hz), 5.57 (1H, d, J=7.9Hz), 5.92 (1H, s), 6.76-6.86 (2H, m), 7.15-7.36 (12H, m).

Example 84

N-Propyl-1-(4-dimethylaminobutyl)-3-(diphenylmethyl)indole-2-carboxamide (Compound 83)

$^1$H-NMR($CDCl_3$) δ(ppm): 0.83 (3H, t, J=7.3Hz), 1.37-1.52 (4H, m), 1.76-1.87 (2H, m), 2.16 (6H, s), 2.23 (2H, t, J=7.6Hz), 3.28 (2H, dt, J=6.9, 5.9Hz), 4.39 (2H, t, J=7.3Hz), 5.73 (1H, t, J=5.6Hz), 5.94 (1H, s), 6.83-6.84 (2H, m), 7.16-7.36 (12H, m).

Example 85

N-Cyclooctyl-1-(4-dimethylaminobutyl)-3-(diphenylmethyl)indole-2-carboxamide (Compound 84)

$^1$H-NMR($CDCl_3$) δ(ppm): 1.30-1.90 (18H, m), 2.16 (6H, s), 2.25 (2H, t, J=7.3Hz), 4.11-4.16 (1H, m), 4.39 (2H, t, J=7.3Hz), 5.68 (1H, d, J=7.9Hz), 5.94 (1H, s), 6.76-6.85 (2H, m), 7.15-7.35 (12H, m).

Example 86

N-(4-Propylphenyl)-1-(4-dimethylaminobutyl)-3-(diphenylmethyl)indole-2-carboxamide (Compound 85)

$^1$H-NMR($CDCl_3$) δ(ppm): 0.93 (3H, t, J=7.3Hz), 1.44-1.55 (2H, m), 1.55-1.68 (2H, m), 1.75-1.95 (2H, m), 2.14 (6H, s), 2.25 (2H, t, J=7.3Hz), 2.55 (2H, t, J=7.6Hz), 4.43 (2H, t, J=7.3Hz), 6.04 (1H, s), 6.81-6.89 (2H, m), 7.08-7.39 (16H, m), 7.44 (1H, s).

Example 87

N-[3-(2-Oxopyrrolidinyl)propyl]-1-(4-dimethylaminobutyl)-3-(diphenylmethyl)indole-2-carboxamide (Compound 86)

$^1$H-NMR($CDCl_3$) δ(ppm): 1.43-1.54 (2H, m), 1.62-1.72 (2H, m), 1.76-1.87 (2H, m), 1.96-2.06 (2H, m), 2.18 (6H, s), 2.26 (2H, t, J=7.6Hz), 2.36 (2H, t, J=7.6Hz), 3.19-3.32 (4H, m), 3.35 (2H, t, J=6.9Hz), 4.35 (2H, t, J=7.6Hz), 5.95

(1H, s), 6.14 (1H, t, J=5.9Hz), 6.85 (2H, d, J=4.0Hz), 7.16-7.35 (12H, m).

Example 88

Ethyl 3-(diphenylmethyl)-1-(4-pyrrolidinylbutyl)indole-2-carboxylate (Compound 87)

Substantially the same procedure as in Example 73 was repeated using ethyl 1-(4-chlorobutyl)-3-(diphenylmethyl)indole-2-carboxylate (40.0 g, 89.7 mmol) obtained in Reference Example 4 and pyrrolidine (40.4 ml, 484 mmol) to give 43.0 g (quantitative) of the title compound.

$^1$H-NMR($CDCl_3$) δ(ppm): 1.45-1.65 (2H, m), 1.65-1.90 (6H, m), 2.35-2.55 (6H, m), 4.33 (2H, q, J=7.3Hz), 4.50 (2H, t, J=7.3Hz), 6.57 (1H, s), 6.81-6.93 (2H, m), 7.16-7.38 (12H, m).

Example 89

3-(Diphenylmethyl)-1-(4-pyrrolidinylbutyl)indole-2-carboxylic acid (Compound 88)

Substantially the same procedure as in Example 6 was repeated using Compound 87 (43.0 g) obtained in Example 88 to give 25.5 g (yield: 63%) of the title compound.

$^1$H-NMR($CDCl_3$) δ(ppm): 1.60-1.95 (8H, m), 2.76-2.82 (2H, t, J=7.9Hz), 2.70-3.15 (4H, br), 4.66 (2H, t, J=6.3Hz), 6.77 (1H, s), 6.83 (1H, t, J=6.9Hz), 7.36 (1H, d, J=7.9Hz), 7.10-7.28 (12H, m).

In the following Examples 90 to 94, substantially the same procedure as in Example 7 was repeated using Compound 88 in place of Compound 6 and using corresponding amines in place of propylamine to give the desired compounds.

Example 90

N-Isopropyl-3-(diphenylmethyl)-1-(4-pyrrolidinylbutyl)indole-2-carboxamide (Compound 89)

$^1$H-NMR($CDCl_3$) δ(ppm): 1.07 (6H, d, J=6.6Hz), 1.50-1.70 (2H, m), 1.70-1.95 (6H, m), 2.45-2.65 (6H, m), 4.13-4.25 (1H, m), 4.37 (2H, t, J=7.3Hz), 5.57 (1H, d, J=7.9Hz), 5.92 (1H, s), 6.76-6.86 (2H, m), 7.15-7.36 (12H, m).

Example 91

N-Propyl-3-(diphenylmethyl)-1-(4-pyrrolidinylbutyl)indole-2-carboxamide (Compound 90)

$^1$H-NMR($CDCl_3$) δ(ppm): 0.83 (3H, t, J=7.6Hz), 1.35-1.60 (4H, m), 1.70-1.95 (6H, m), 2.35-2.55 (6H, m), 3.28 (2H, dt, J=6.6, 7.3Hz), 4.38 (2H, t, J=7.3Hz), 5.74 (1H, t, J=5.6Hz), 5.93 (1H, s), 6.83-6.88 (2H, m), 7.16-7.36 (12H, m).

Example 92

N-Cyclooctyl-3-(diphenylmethyl)-1-(4-pyrrolidinylbutyl)indole-2-carboxamide (Compound 91)

$^1$H-NMR($CDCl_3$) δ(ppm): 1.25-1.95 (22H, m), 2.35-2.60 (6H, m), 4.05-4.20 (1H, m), 4.38 (2H, t, J=7.3Hz), 5.67 (1H, d, J=7.9Hz), 5.94 (1H, s), 6.76-6.85 (2H, m), 7.15-7.36 (12H, m).

Example 93

N-(4-Propylphenyl)-3-(diphenylmethyl)-1-(4-pyrrolidinylbutyl)indole-2-carboxamide (Compound 92)

$^1$H-NMR($CDCl_3$) δ(ppm): 0.93 (3H, t, J=7.3Hz), 1.50-1.80 (10H, m), 1.80-1.95 (2H, m), 2.40-2.60 (6H, m), 4.43 (2H, t, J=7.3Hz), 6.04 (1H, s), 6.81-6.90 (2H, m), 7.08-7.39 (16H, m), 7.45 (1H, m).

Example 94

N-[3-(2-Oxopyrrolidinyl)propyl]-3-(diphenylmethyl)-1-(4-pyrrolidinylbutyl)indole-2-carboxamide (Compound 93)

$^1$H-NMR($CDCl_3$) δ(ppm): 1.50-1.95 (12H, m), 2.02 (2H, m), 2.36 (2H, t, J=7.6Hz), 2.35-2.70 (4H, br), 3.19-3.32 (4H, m), 3.36 (2H, t, J=6.9Hz), 4.34 (2H, t, J=7.3Hz), 5.96 (1H, s), 6.24 (1H, br), 6.85-6.87 (2H, m), 7.17-7.35 (12H, m).

Example 95

Ethyl 3-[bis(4-chlorophenyl)methyl]-1-(2-dimethylaminoethyl)indole-2-carboxylate (Compound 94)

Substantially the same procedure as in Example 2 was repeated using ethyl 3-[bis(4-chlorophenyl)methyl]indole-2-carboxylate (5.0 g, 11.8 mmol) obtained in Reference Example 5 and 2-dimethylaminoethylchloride hydrochloride (1.78 g, 12.4 mmol) to give 6.3 g (quantitative) of the title compound.

$^1$H-NMR($CDCl_3$) δ(ppm): 1.36 (3H, t, J=7.1Hz), 2.33 (6H, s), 2.66 (2H, dd, J=7.6, 7.9Hz), 4.36 (2H, q, J=7.1Hz), 4.59 (2H, dd, J=7.6, 7.9Hz), 6.49 (1H, s), 6.85-6.95 (2H, m), 7.15 (4H, d, J=7.7Hz), 7.2-7.35 (5H, m), 7.41 (1H, d, J=8.6Hz).

Example 96

3-[Bis(4-chlorophenyl)methyl]-1-(2-dimethylaminoethyl)indole-2-carboxylic acid (Compound 95)

Substantially the same procedure as in Example 6 was repeated using Compound 94 (6.3 g) obtained in Example 95 to give 4.2 g (yield: 77%) of the title compound.

$^1$H-NMR($CDCl_3$) δ(ppm): 2.60 (6H, s), 3.34 (2H, t, J=6.4Hz), 4.87 (2H, t, J=6.4Hz), 6.77 (1H, s), 6.9-7.0 (1H, m), 7.04 (1H, d, J=7.9Hz), 7.1-7.2 (8H, m), 7.2-7.35 (2H, m).

In the following Examples 97 and 98, substantially the same procedure as in Example 7, and then substantially the same procedure as in Example 48 were repeated using Compound 95 in place of Compound 6 and using corresponding amines in place of propylamine to give the desired compounds.

Example 97

N-Propyl-3-[bis(4-chlorophenyl)methyl]-1-(2-dimethylaminoethyl)indole-2-carboxamide hydrochloride (Compound 96 hydrochloride)

$^1$H-NMR($CDCl_3$) δ(ppm): 0.88 (3H, t, J=7.3Hz), 1.4-1.55 (2H, m), 2.90 (3H, s), 2.91 (3H, s), 3.28 (2H, dt, J=5.9, 7.3Hz), 3.5-3.6 (2H, m), 4.8-4.9 (2H, m), 5.72 (1H, t, J=5.9Hz), 5.87 (1H, s), 6.80 (1H, d, J=7.9Hz), 6.91 (1H, t, J=7.3Hz), 7.06 (4H, d, J=8.2Hz), 7.25-7.4 (5H, m), 7.81 (1H, d, J=8.6Hz), 13.1 (1H, br).

Example 98

N-(4-propylphenyl)-3-[bis(4-chlorophenyl)methyl]-1-(2-dimethylaminoethyl)indole-2-carboxamide hydrochloride (Compound 97 hydrochloride)

$^1$H-NMR($CDCl_3$) δ(ppm): 0.94 (3H, t, J=7.3Hz), 1.55-1.7 (2H, m), 2.57 (2H, t, J=7.6Hz), 2.83 (3H, s), 2.85 (3H, s), 3.5-3.6 (2H, m), 4.65-4.75 (2H, m), 6.02 (1H, s), 6.78 (1H, d, J=8.3Hz), 6.94 (1H, t, J=7.5Hz), 7.05-7.4 (13H, m), 7.8-7.9 (2H, m), 12.9 (1H, br).

Example 99

Ethyl 3-[bis(4-methylphenyl)methyl]-1-(2-piperidinoethyl)indole-2-carboxylate (Compound 98)

Substantially the same procedure as in Example 2 was repeated using ethyl 3-[bis(4-methylphenyl)methyl]indole-2-carboxylate (5.0 g, 13.04 mmol) obtained in Reference Example 6 and 2-piperidinoethylchloride hydrochloride (2.64 g, 14.34 mmol) to give 6.8 g (quantitative) of the title compound.

$^1$H-NMR($CDCl_3$) δ(ppm): 1.37 (3H, t, J=7.3Hz), 1.4-1.5 (2H, m), 1.5-1.6 (4H, m), 2.30 (6H, s), 2.4-2.55 (4H, m), 2.6-2.7 (2H, m), 4.34 (2H, q, J=7.3Hz), 4.55-4.65 (2H, m), 6.49 (1H, s), 6.8-6.9 (1H, m), 6.95-7.1 (9H, m), 7.2-7.3 (1H, m), 7.40 (1H, d, J=8.6Hz).

Example 100

3-[Bis(4-methylphenyl)methyl]-1-(2-piperidinoethyl)indole-2-carboxylic acid (Compound 99)

Substantially the same procedure as in Example 6 was repeated using Compound 98 (6.8 g) obtained in Example 99 to give 5.1 g (yield: 84%) of the title compound.

$^1$H-NMR($CDCl_3$) δ(ppm): 1.4-1.7 (6H, m), 2.27 (6H, s), 2.3-2.6 (4H, m), 3.05-3.15 (2H, m), 4.65-4.75 (2H, m),

6.70 (1H, s), 6.85-6.95 (1H, m), 7.00 (4H, d, J=7.9Hz), 7.1-7.2 (7H, m).

In the following Examples 101 to 103, substantially the same procedure as in Example 7, and then, if necessary, substantially the same procedure as in Example 48 were repeated using Compound 99 in place of Compound 6 and using corresponding amines in place of propylamine to give the desired compounds.

Example 101

N-(3-Morpholinopropyl)-3-[bis(4-methylphenyl)methyl]-1-(2-piperidinoethyl)indole-2-carboxamide 2 hydrochloride (Compound 100 2 hydrochloride)

$^1$H-NMR($CDCl_3$) δ(ppm): 1.8-2.0 (6H, m), 2.1-2.25 (4H, m), 2.32 (6H, s), 2.75-3.1 (4H, m), 3.3-3.65 (8H, m), 3.9-4.0 (2H, m), 4.1-4.25 (2H, m), 4.9-5.0 (2H, m), 5.86 (1H, s), 6.71 (1H, br), 6.8-6.95 (2H, m), 7.03 (4H, d, J=8.1Hz), 7.09 (4H, d, J=8.1Hz), 7.2-7.3 (1H, m), 7.61 (1H, d, J=8.3Hz), 12.0 (1H, br), 12.5 (1H, br).

Example 102

N-Isopropyl-3-[bis(4-methylphenyl)methyl]-1-(2-piperidinoethyl)indole-2-carboxamide hydrochloride (Compound 101 hydrochloride)

$^1$H-NMR($CDCl_3$) δ(ppm): 1.07 (6H, d, J=6.6Hz), 1.85-2.0 (4H, m), 2.25-2.4 (2H, m), 2.33 (6H, s), 2.7-2.9 (2H, m), 3.45-3.65 (4H, m), 4.05-4.2 (1H, m), 4.85-4.95 (2H, m), 5.70 (1H, d, J=7.9Hz), 5.83 (1H, s), 6.78 (1H, d, J=7.9Hz), 6.88 (1H, t, J=7.3Hz), 7.01 (4H, d, J=8.1Hz), 7.10 (4H, d, J=8.1Hz), 7.25-7.3 (1H, m), 7.87 (1H, d, J=8.3Hz), 12.57 (1H, br).

Example 103

N-[3-(1-Imidazolyl)propyl]-3-[bis(4-methylphenyl)methyl]-1-(2-piperidinoethyl)indole-2-carboxamide (Compound 102)

$^1$H-NMR($CDCl_3$) δ(ppm): 1.3-1.45 (2H, m), 1.65-1.8 (4H, m), 1.9-2.0 (2H, m), 2.2-2.3 (4H, m), 2.30 (6H, s), 2.80 (2H, t, J=6.3Hz), 3.3-3.4 (2H, m), 3.89 (2H, t, J=7.2Hz), 4.42 (2H, t, J=6.3Hz), 6.14 (1H, s), 6.85-7.0 (3H, m), 7.0-7.1 (9H, m), 7.15-7.25 (1H, m), 7.33 (1H, d, J=8.2Hz), 7.40 (1H, s), 8.36 (1H, br).

Example 104

Ethyl 3-[bis(4-methylphenyl)methyl]-1-(2-pyrrolidinylethyl)indole-2-carboxylate (Compound 103)

Substantially the same procedure as in Example 2 was repeated using ethyl 3-[bis(4-methylphenyl)methyl]indole-2-carboxylate (5.0 g, 13.04 mmol) obtained in Reference Example 6 and 2-pyrrolidinylethylchloride hydrochloride (2.45 g, 14.34 mmol) to give 6.7 g (quantitative) of the title compound.

$^1$H-NMR($CDCl_3$) δ(ppm): 1.37 (3H, t, J=7.3Hz), 1.7-1.9 (4H, m), 2.31 (6H, s), 2.6-2.7 (4H, m), 2.8-2.9 (2H, m), 4.34 (2H, q, J=7.3Hz), 4.6-4.7 (2H, m), 6.49 (1H, s), 6.8-6.9 (1H, m), 6.95-7.15 (9H, m), 7.2-7.3 (1H, m), 7.41 (1H, d, J=8.6Hz).

Example 105

3-[Bis(4-methylphenyl)methyl]-1-(2-pyrrolidinylethyl)indole-2-carboxylic acid (Compound 104)

Substantially the same procedure as in Example 6 was repeated using Compound 103 (6.7 g) obtained in Example 104 to give 4.7 g (yield: 79%) of the title compound.

$^1$H-NMR(DMSO-$d_6$+$CD_3OD$) δ(ppm): 1.75-1.9 (4H, m), 2.33 (6H, s), 2.85-3.0 (4H, m), 3.55-3.7 (2H, m), 4.75-4.9 (2H, m), 6.71 (1H, s), 6.93 (1H, t, J=7.4Hz), 7.05-7.25 (10H, m), 7.54 (1H, d, J=7.3Hz).

In the following Examples 106 to 108, substantially the same procedure as in Example 7, and then, if necessary, substantially the same procedure as in Example 48 were repeated using Compound 104 in place of Compound 6 and using corresponding amines in place of propylamine to give the desired compounds.

Example 106

N-(3-Morpholinopropyl)-3-[bis(4-methylphenyl)methyl]-1-(2-pyrrolidinylethyl)indole-2-carboxamide 2 hydrochloride (Compound 105 2 hydrochloride)

$^1$H-NMR($CDCl_3$) δ(ppm): 2.05-2.2 (6H, m), 2.33 (6H, s), 2.8-3.0 (6H, m), 3.3-3.45 (4H, m), 3.55-3.65 (2H, m), 3.8-3.9 (2H, m), 3.9-4.0 (2H, m), 4.15-4.3 (2H, m), 4.8-4.95 (2H, m), 5.86 (1H, s),Å@6.48 (1H, br), 6.8-6.95 (2H, m), 7.02 (4H, d, J=8.1Hz), 7.10 (4H, d, J=8.1Hz), 7.25-7.3 (1H, m), 7.62 (1H, d, J=8.6Hz), 12.58 (1H, br), 12.72 (1H, br).

Example 107

N-Isopropyl-3-[bis(4-methylphenyl)methyl]-1-(2-pyrrolidinylethyl)indole-2-carboxamide hydrochloride (Compound 106 hydrochloride)

$^1$H-NMR($CDCl_3$) δ(ppm): 1.07 (6H, d, J=6.6Hz), 2.0-2.3 (4H, m), 2.33 (6H, s), 2.85-3.05 (2H, m), 3.55-3.7 (2H, m), 3.7-3.85 (2H, m), 4.05-4.2 (1H, m), 4.8-4.9 (2H, m), 5.71 (1H, d, J=7.9Hz), 5.84 (1H, s), 6.78 (1H, d, J=7.9Hz), 6.88 (1H, t, J=7.6Hz), 7.01 (4H, d, J=8.3Hz), 7.10 (4H, d, J=8.3Hz), 7.25-7.35 (1H, m), 7.79 (1H, d, J=8.6Hz), 12.86 (1H, br).

Example 108

N-[3-(1-Imidazolyl)propyl]-3-[bis(4-methylphenyl)methyl]-1-(2-pyrrolidinylethyl)indole-2-carboxamide (Compound 107)

$^1$H-NMR($CDCl_3$) δ(ppm): 1.55-1.7 (4H, m), 1.85-2.0 (2H, m), 2.29 (6H, s), 2.3-2.4 (4H, m), 3.00 (2H, t, J=6.3Hz), 3.33 (2H, dd, J=6.0, 6.9Hz), 3.83 (2H, t, J=7.3Hz), 4.43 (2H, t, J=6.3Hz), 6.15 (1H, s), 6.84 (1H, s), 6.8-7.0 (2H, m), 7.0-7.15 (9H, m), 7.15-7.25 (1H, m), 7.32 (1H, d, J=8.3Hz), 7.38 (1H, s), 8.25 (1H, br).

Example 109

Ethyl 3-[bis(4-methylphenyl)methyl]-1-(2-morpholinoethyl)indole-2-carboxylate (Compound 108)

Substantially the same procedure as in Example 2 was repeated using ethyl 3-[bis(4-methylphenyl)methyl]indole-2-carboxylate (5.0 g, 13.04 mmol) obtained in Reference Example 6 and 2-morpholinoethylchloride hydrochloride (2.67 g, 14.34 mmol) to give 6.8 g (quantitative) of the title compound.

$^1$H-NMR($CDCl_3$) δ(ppm): 1.37 (3H, t, J=7.3Hz), 2.31 (6H, s), 2.45-2.55 (4H, m), 2.65-2.75 (2H, m), 3.6-3.7 (4H, m), 4.34 (2H, q, J=7.3Hz), 4.6-4.7 (2H, m), 6.47 (1H, s), 6.8-6.9 (1H, m), 6.95-7.15 (9H, m), 7.2-7.3 (1H, m), 7.3-7.4 (1H, m).

Example 110

3-[Bis(4-methylphenyl)methyl]-1-(2-morpholinoethyl)indole-2-carboxylic acid (Compound 109)

Substantially the same procedure as in Example 6 was repeated using Compound 108 (6.8 g) obtained in Example 109 to give 4.5 g (yield: 74%) of the title compound.

$^1$H-NMR($CDCl_3$) δ(ppm): 2.29 (6H, s), 2.3-2.4 (4H, m), 3.02 (2H, t, J=5.6Hz), 3.45-3.55 (4H, m), 4.62 (2H, t, J=5.6Hz), 6.56 (1H, s), 6.9-7.0 (1H, m), 7.02 (4H, d, J=8.1Hz), 7.12 (4H, d, J=8.1Hz), 7.2-7.3 (3H, m).

In the following Examples 111 to 113, substantially the same procedure as in Example 7, and then, if necessary, substantially the same procedure as in Example 48 were repeated using Compound 109 in place of Compound 6 and using corresponding amines in place of propylamine to give the desired compounds.

Example 111

N-(3-Morpholinopropyl)-3-[bis(4-methylphenyl)methyl]-1-(2-morpholinoethyl)indole-2-carboxamide 2 hydrochloride (Compound 110 2 hydrochloride)

$^1$H-NMR($CDCl_3$) δ(ppm): 2.05-2.2 (2H, m), 2.32 (6H, s), 2.6-2.8 (2H, m), 2.9-3.3 (4H, m), 3.35-3.45 (4H, m), 3.45-3.6 (4H, m), 3.9-4.1 (4H, m), 4.1-4.3 (4H, m), 4.85-4.95 (2H, m), 5.88 (1H, s), 6.85-6.95 (2H, m), 7.0-7.15 (9H, m), 7.2-7.3 (1H, m), 7.68 (1H, d, J=8.2Hz), 12.32 (1H, br), 12.75 (1H, br).

Example 112

N-Isopropyl-3-[bis(4-methylphenyl)methyl]-1-(2-morpholinoethyl)indole-2-carboxamide hydrochloride (Compound 111 hydrochloride)

$^1$H-NMR($CDCl_3$) δ(ppm): 1.07 (6H, d, J=6.6Hz), 2.34 (6H, s), 2.95-3.15 (2H, m), 3.45-3.6 (4H, m), 3.95-4.05 (2H, m), 4.05-4.2 (1H, m), 4.30 (2H, t, J=11.9Hz), 4.9-5.0 (2H, m), 5.72 (1H, d, J=7.6Hz), 5.84 (1H, s), 6.79 (1H, d, J=8.2Hz), 6.89 (1H, t, J=7.6Hz), 7.01 (4H, d, J=8.2Hz), 7.10 (4H, d, J=8.2Hz), 7.25-7.3 (1H, m), 7.81 (1H, d, J=8.2Hz), 13.55 (1H, br).

Example 113

N-[3-(1-Imidazolyl)propyl]-3-[bis(4-methylphenyl)methyl]-1-(2-morpholinoethyl)indole-2-carboxamide (Compound 112)

$^1$H-NMR($CDCl_3$) δ(ppm): 1.85-2.0 (2H, m), 2.32 (6H, s), 2.35-2.45 (4H, m), 2.78 (2H, t, J=6.9Hz), 3.32 (2H, q, J=6.6Hz), 3.5-3.6 (4H, m), 3.89 (2H, t, J=7.1Hz), 4.48 (2H, t, J=6.9Hz), 5.97 (1H, s), 6.76 (1H, t, J=6.6Hz), 6.8-6.9 (3H, m), 7.0-7.15 (9H, m), 7.2-7.3 (1H, m), 7.35 (1H, d, J=8.3Hz), 7.40 (1H, s).

Example 114

Ethyl 3-[bis(4-methylphenyl)methyl]-1-(2-dimethylaminoethyl)indole-2-carboxylate (Compound 113)

Substantially the same procedure as in Example 2 was repeated using ethyl 3-[bis(4-methylphenyl)methyl]indole-2-carboxylate (4.55 g, 11.86 mmol) obtained in Reference Example 6 and 2-dimethylaminoethylchloride hydrochloride (1.88 g, 13.05 mmol) to give 5.7 g (quantitative) of the title compound.

$^1$H-NMR($CDCl_3$) δ(ppm): 1.37 (3H, t, J=6.9Hz), 2.31 (6H, s), 2.37 (6H, s), 2.65-2.75 (2H, m), 4.34 (2H, q, J=6.9Hz), 4.55-4.65 (2H, m), 6.49 (1H, s), 6.8-6.9 (1H, m), 6.9-7.15 (9H, m), 7.2-7.3 (1H, m), 7.40 (1H, d, J=8.3Hz).

Example 115

3-[Bis(4-methylphenyl)methyl]-1-(2-dimethylaminoethyl)indole-2-carboxylic acid (Compound 114)

Substantially the same procedure as in Example 6 was repeated using Compound 113 (5.7 g) obtained in Example 114 to give 3.5 g (yield: 68%) of the title compound.

$^1$H-NMR($CDCl_3$) δ(ppm): 2.28 (6H, s), 2.37 (6H, s), 3.16 (2H, t, J=5.7Hz), 4.71 (2H, t, J=5.7Hz), 6.70 (1H, s), 6.85-6.95 (1H, m), 7.01 (4H, d, J=6.9Hz), 7.15 (4H, d, J=6.9Hz), 7.15-7.25 (3H, m).

In the following Examples 116 to 118, substantially the same procedure as in Example 7, and then, if necessary, substantially the same procedure as in Example 48 were repeated using Compound 114 in place of Compound 6 and using corresponding amines in place of propylamine to give the desired compounds.

Example 116

N-(3-Morpholinopropyl)-3-[bis(4-methylphenyl)methyl]-1-(2-dimethylaminoethyl)indole-2-carboxamide 2 hydrochloride (Compound 115 2 hydrochloride)

$^1$H-NMR($CDCl_3$) δ(ppm): 2.05-2.2 (2H, m), 2.31 (6H, s), 2.85-3.1 (4H, m), 2.93 (6H, s), 3.3-3.45 (4H, m), 3.5-3.6 (2H, m), 3.9-4.05 (2H, m), 4.05-4.25 (2H, m), 4.8-4.9 (2H, m), 5.89 (1H, s), 6.85-6.9 (2H, m), 7.05-7.1 (8H, m), 7.2-7.35 (2H, m), 7.65 (1H, d, J=8.3Hz), 12.14 (2H, br).

Example 117

N-Isopropyl-3-[bis(4-methylphenyl)methyl]-1-(2-dimethylaminoethyl)indole-2-carboxamide hydrochloride (Compound 116 hydrochloride)

$^1$H-NMR($CDCl_3$) δ(ppm): 1.08 (6H, d, J=6.3Hz), 2.34 (6H, s), 2.90 (3H, s), 2.92 (3H, s), 3.5-3.65 (2H, m), 4.05-4.2 (1H, m), 4.8-4.9 (2H, m), 5.72 (1H, d, J=7.9Hz), 5.84 (1H, s), 6.79 (1H, d, J=7.3Hz), 6.89 (1H, t, J=7.6Hz), 7.00 (4H, d, J=7.9Hz), 7.10 (4H, d, J=7.9Hz), 7.25-7.3 (1H, m), 7.77 (1H, d, J=8.6Hz), 13.05 (1H, br).

Example 118

N-[3-(1-Imidazolyl)propyl)-3-[bis(4-methylphenyl)methyl]-1-(2-dimethylaminoethyl)indole-2-carboxamide (Compound 117)

$^1$H-NMR($CDCl_3$) δ(ppm): 1.9-2.0 (2H, m), 2.17 (6H, s), 2.30 (6H, s), 2.78 (2H, t, J=6.3Hz), 3.25-3.35 (2H, m), 3.82 (2H, t, J=7.1Hz), 4.39 (2H, t, J=6.3Hz), 6.11 (1H, s), 6.82 (1H, s), 6.85-6.95 (2H, m), 7.0-7.1 (9H, m), 7.2-7.35 (2H, m), 7.36 (1H, s), 7.6-7.7 (1H, m).

Example 119

N-Propyl-1-(2-dimethylaminoethyl)-3-(4-hydroxybenzhydryl)indole-2-carboxamide hydrochloride (Compound 118 hydrochloride)

To a solution of N-propyl-3-(4-benzyloxybenzhydryl)-1-(2-dimethylaminoethyl)indole-2-carboxamide (1.95 g, 3.57 mmol) obtained in Reference Example 10 in 30 ml of ethanol was added 10% palladium on carbon (0.39 g, 50% aqueous), followed by stirring under a hydrogen atmosphere at 70°C for 4.5 hours. The catalyst was filtered off and the solvent was distilled off under reduced pressure to give 1.65 g of a crude compound. The obtained crude product was crystallized from diethyl ether to give 1.42 g (yield :82%) of a free base of the title compound. Then, substantially the same procedure as in Example 61 was repeated using the free base (1.0 g) to give 0.92 g (yield: 86%) of the title compound.

$^1$H-NMR($CDCl_3$) δ(ppm): 0.83 (3H, t, J=7.4Hz), 1.35-1.5 (2H, m), 2.89 (6H, s), 3.2-3.35 (2H, m), 4.0-4.15 (2H, m), 4.7-4.85 (2H, m), 5.8-5.95 (2H, m), 6.38 (1H, s), 6.7-6.9 (4H, m), 6.96 (2H, d, J=8.6Hz), 7.05-7.15 (2H, m), 7.15-7.35 (4H, m), 7.74 (1H, d, J=8.3Hz), 12.73 (1H, br).

In the following Examples 120 to 128, substantially the same procedure as in Example 119 was repeated using Compounds obtained in Reference Examples 11 to 19 in place of N-propyl-3-(4-benzyloxybenzhydryl)-1-(2-dimethylaminoethyl)indole-2-carboxamide to give the desired compounds.

Example 120

N-Isopropyl-1-(2-dimethylaminoethyl)-3-(4-hydroxybenzhydryl)indole-2-carboxamide hydrochloride (Compound 119 hydrochloride)

$^1$H-NMR($CDCl_3$) δ(ppm): 1.06 (3H, d, J=6.3Hz), 1.07 (3H, d, J=6.3Hz), 2.89 (6H, s), 3.5-3.6 (2H, m), 4.0-4.15 (1H, m), 4.65-4.85 (2H, m), 5.74 (1H, d, J=7.6Hz), 5.83 (1H, s), 6.36 (1H, s), 6.2-6.4 (4H, m), 6.96 (2H, d, J=8.6Hz), 7.0-7.1 (2H, m), 7.1-7.35 (4H, m), 7.73 (1H, d, J=8.6Hz), 12.74 (1H, br).

Example 121

N-(4-Propylphenyl)-1-(2-dimethylaminoethyl)-3-(4-hydroxybenzhydryl)indole-2-carboxamide hydrochloride (Compound 120 hydrochloride)

$^1$H-NMR($CDCl_3$) δ(ppm): 0.93 (3H, t, J=7.3Hz), 1.5-1.65 (2H, m), 2.45-2.6 (2H, m), 2.90 (6H, s), 3.5-3.65 (2H, m), 4.3-4.45 (2H, m), 5.94 (1H, s), 5.98 (1H, s), 6.7-6.95 (4H, m), 7.00 (2H, d, J=8.3Hz), 7.05-7.2 (6H, m), 7.2-7.35 (4H, m), 7.54 (1H, s), 7.82 (1H, d, J=8.6Hz), 12.86 (1H, br).

Example 122

N-(3-Morpholinopropyl)-1-(2-dimethylaminoethyl)-3-(4-hydroxybenzhydryl)indole-2-carboxamide 2 hydrochloride (Compound 121 2 hydrochloride)

$^1$H-NMR(DMSO-$d_6$) δ(ppm): 1.9-2.1 (2H, m), 2.85 (6H, s), 2.95-3.2 (4H, m), 3.2-3.5 (6H, m), 3.75-4.0 (4H, m), 4.55-4.7 (2H, m), 5.86 (1H, s), 6.67 (2H, d, J=8.3Hz), 6.8-7.0 (4H, m), 7.1-7.3 (6H, m), 7.65 (1H, d, J=8.3Hz), 8.47 (1H, t, J=5.6Hz), 9.20 (1H, s), 10.81 (1H, br), 11.04 (1H, br).

Example 123

N-[3-(2-Oxopyrrolidinyl)propyl]-1-(2-dimethylaminoethyl)-3-(4-hydroxybenzhydryl)indole-2-carboxamide hydrochloride (Compound 122 hydrochloride)

$^1$H-NMR(DMSO-$d_6$) δ(ppm): 1.6-1.75 (2H, m), 1.9-2.05 (2H, m), 2.25 (2H, t, J=7.9Hz), 2.81 (6H, s), 3.15-3.3 (4H, m), 3.3-3.55 (4H, m), 4.5-4.65 (2H, m), 5.85 (1H, s), 6.66 (2H, d, J=8.3Hz), 6.8-6.9 (2H, m), 6.95 (2H, d, J=8.3Hz), 7.6-7.8 (6H, m), 7.67 (1H, d, J=8.2Hz), 8.2-8.3 (1H, m), 9.24 (1H, s), 10.69 (1H, br).

Example 124

N-Cyclooctyl-1-(2-dimethylaminoethyl)-3-(4-hydroxybenzhydryl)indole-2-carboxamide hydrochloride (Compound 123 hydrochloride)

$^1$H-NMR(DMSO-$d_6$) δ(ppm): 1.4-1.7 (12H, m), 1.7-1.9 (2H, m), 2.80 (6H, s), 3.3-3.45 (2H, m), 3.95-4.1 (1H, m), 4.55-4.65 (2H, m), 5.80 (1H, s), 6.66 (2H, d, J=8.6Hz), 6.8-6.9 (2H, s), 6.94 (2H, d, J=8.6Hz), 7.1-7.3 (6H, m), 7.67 (1H, d, J=8.2Hz), 8.01 (1H, d, J=7.9Hz), 9.26 (1H, s), 10.83 (1H, br).

Example 125

N-Propyl-3-(4-hydroxybenzhydryl)-1-(2-pyrrolidinylethyl)indole-2-carboxamide hydrochloride (Compound 124 hydrochloride)

$^1$H-NMR(DMSO-$d_6$) δ(ppm): 0.91 (3H, t, J=7.3Hz), 1.45-1.65 (2H, m), 1.75-2.05 (4H, m), 2.85-3.05 (2H, m), 3.05-3.4 (4H, m), 3.4-3.6 (2H, m), 4.45-4.6 (2H, m), 5.84 (1H, s), 6.66 (2H, d, J=8.3Hz), 6.85-6.9 (2H, m), 6.94 (2H, d, J=8.3Hz), 7.05-7.3 (6H, m), 7.62 (1H, d, J=6.3Hz), 8.2-8.3 (1H, m), 9.21 (1H, s), 10.65 (1H, br).

Example 126

N-Isopropyl-3-(4-hydroxybenzhydryl)-1-(2-pyrrolidinylethyl)indole-2-carboxamide hydrochloride (Compound 125 hydrochloride)

$^1$H-NMR(DMSO-$d_6$) δ(ppm): 1.17 (6H, d, J=6.6Hz), 1.75-2.05 (4H, m), 2.85-3.05 (2H, m), 3.4-3.6 (4H, m), 4.0-4.15 (1H, m), 4.5-4.65 (2H, m), 5.83 (1H, s), 6.67 (2H, d, J=8.6Hz), 6.85-6.9 (2H, m), 6.95 (2H, d, J=8.6Hz), 7.05-7.3 (6H, m), 7.62 (1H, d, J=8.3Hz), 7.84 (1H, br), 9.19 (1H, s), 10.64 (1H, br).

Example 127

N-(3-Morpholinopropyl)-3-(4-hydroxybenzhydryl)-1-(2-pyrrolidinylethyl)indole-2-carboxamide 2 hydrochloride (Compound 126 2 hydrochloride)

$^1$H-NMR(DMSO-$d_6$) δ(ppm): 1.75-2.05 (6H, m), 2.9-3.2 (6H, m), 3.2-3.6 (8H, m), 3.75-4.0 (4H, m), 4.5-4.65 (2H, m), 5.85 (1H, s), 6.67 (2H, d, J=8.4Hz), 6.85-6.9 (2H, m), 6.95 (2H, d, J=8.4Hz), 7.1-7.3 (6H, m), 7.65 (1H, d, J=8.2Hz), 8.5-8.6 (1H, m), 9.24 (1H, s), 11.05 (1H, br), 11.2 (1H, br).

Example 128

N-[3-(2-Oxopyrrolidinyl)propyl]-3-(4-hydroxybenzhydryl)-1-(2-pyrrolidinylethyl)indole-2-carboxamide hydrochloride (Compound 127 hydrochloride)

$^1$H-NMR($CDCl_3$) δ(ppm): 1.5-1.65 (2H, m), 1.95-2.2 (6H, m), 2.34 (2H, t, J=7.9Hz), 2.8-3.0 (2H, m), 3.1-3.35 (6H, m), 3.5-3.65 (2H, m), 3.65-3.8 (2H, m), 4.7-4.85 (2H, m), 5.86 (1H, s), 6.0-6.1 (1H, m), 6.7-6.85 (4H, m), 6.95 (2H, d, J=8.3Hz), 7.0-7.1 (2H, m), 7.1-7.3 (4H, m), 7.74 (1H, d, J=8.3Hz), 12.45 (1H, br).

In the following Examples 129 to 133, substantially the same procedure as in Example 7 was repeated using 3-[4-(2-dimethylaminoethoxy)benzhydryl]indole-2-carboxylic acid obtained in Reference Example 21 in place of Compound 6 and using corresponding amines in place of propylamine to give the desired compounds.

Example 129

N-Isopropyl-3-[4-(2-dimethylaminoethoxy)benzhydryl]indole-2-carboxamide (Compound 128)

$^1$H-NMR($CDCl_3$) δ(ppm): 1.01 (6H, d, J=6.6Hz), 2.34 (6H, s), 2.73 (2H, t, J=5.9Hz), 4.06 (2H, t, J=5.9Hz), 4.05-4.20 (1H, m), 5.72 (1H, d, J=7.9Hz), 6.01 (1H, s), 6.77-6.89 (4H, m), 7.07-7.45 (9H, m), 9.18 (1H, s).

Example 130

N-Propyl-3-[4-(2-dimethylaminoethoxy)benzhydryl]indole-2-carboxamide (Compound 129)

$^1$H-NMR($CDCl_3$) δ(ppm): 0.79 (3H, t, J=7.3Hz), 1.32-1.45 (2H, m), 2.35 (6H, s), 2.74 (2H, t, J=5.6Hz), 3.21-3.29 (2H, m), 4.06 (2H, t, J=5.9Hz), 5.82 (1H, t, J=5.6Hz), 6.03 (1H, s), 6.76-6.88 (4H, m), 7.06-7.64 (9H, m), 9.14 (1H, s).

Example 131

N-Cyclooctyl-3-[4-(2-dimethylaminoethoxy)benzhydryl]indole-2-carboxamide (Compound 130)

$^1$H-NMR($CDCl_3$) δ(ppm): 1.25-1.90 (14H, br), 2.35 (6H, s), 2.74 (2H, t, J=5.6Hz), 4.06 (2H, t, J=5.9Hz), 4.05-4.20 (1H, m), 5.84 (1H, d, J=8.2Hz), 6.01 (1H, s), 6.73-6.89 (4H, m), 7.06-7.65 (9H, m), 9.13 (1H, s).

Example 132

N-(4-Propylphenyl)-3-[4-(2-dimethylaminoethoxy)benzhydryl]indole-2-carboxamide (Compound 131)

$^1$H-NMR($CDCl_3$) δ(ppm): 0.92 (3H, t, J=7.3Hz), 1.54-1.65 (2H, m), 2.35 (6H, s), 2.54 (2H, t, J=7.3Hz), 2.74 (2H, t, J=5.6Hz), 4.07 (2H, t, J=5.6Hz), 6.15 (1H, s), 6.74-6.92 (4H, m), 7.08-7.42 (13H, m), 7.58 (1H, s), 9.23 (1H, s).

Example 133

N-[3-(2-Oxopyrrolidinyl)propyl]-3-[4-(2-dimethylaminoethoxy)benzhydryl]indole-2-carboxamide (Compound 132)

$^1$H-NMR($CDCl_3$) δ(ppm): 1.67-1.76 (2H, m), 1.98-2.09 (2H, m), 2.35 (6H, s), 2.42 (2H, t, J=7.9Hz), 2.75 (2H, t, J=5.6Hz), 3.30-3.40 (4H, m), 4.05 (2H, t, J=5.6Hz), 6.48 (1H, s), 6.81-6.93 (4H, m), 7.12-7.40 (9H, m), 9.54 (1H, s).

In the following Examples 134 to 138, substantially the same procedure as in Example 7 was repeated using 3-[4-(2-pyrrolidinylethoxy)benzhydryl]indole-2-carboxylic acid obtained in Reference Example 23 in place of Compound 6 and using corresponding amines in place of propylamine to give the desired compounds.

Example 134

N-Isopropyl-3-[4-(2-pyrrolidinylethoxy)benzhydryl]indole-2-carboxamide (Compound 133)

$^1$H-NMR($CDCl_3$) δ(ppm): 1.01 (6H, d, J=6.3Hz), 1.75-1.90 (4H, br), 2.60-2.70 (4H, br), 2.91 (2H, t, J=5.9Hz), 4.11 (2H, t, J=6.3Hz), 4.10-4.25 (1H, m), 5.72 (1H, d, J=7.6Hz), 6.01 (1H, s), 6.77-6.89 (4H, m), 7.07-7.40 (9H, m), 9.16 (1H, s).

Example 135

N-Propyl-3-[4-(2-pyrrolidinylethoxy)benzhydryl]indole-2-carboxamide (Compound 134)

$^1$H-NMR($CDCl_3$) δ(ppm): 0.79 (3H, t, J=7.3Hz), 1.32-1.45 (2H, m), 1.79-1.84 (4H, m), 2.55-2.70 (4H, br), 2.91 (2H, t, J=5.9Hz), 3.21-3.29 (2H, m), 4.10 (2H, t, J=5.9Hz), 5.82 (1H, t, J=5.6Hz), 6.03 (1H, s), 6.76-6.89 (4H, m), 7.05-7.40 (9H, m), 9.15 (1H, s).

Example 136

N-Cyclooctyl-3-[4-(2-pyrrolidinylethoxy)benzhydryl]indole-2-carboxamide (Compound 135)

$^1$H-NMR($CDCl_3$) δ(ppm): 1.25-1.80 (14H, br), 1.80-1.90 (4H, br), 2.55-2.75 (4H, br), 2.93 (2H, t, J=5.9Hz), 4.00-4.15 (1H, m), 4.12 (2H, t, J=5.9Hz), 5.84 (1H, d, J=7.9Hz), 6.01 (1H, s), 6.73-6.88 (4H, m), 7.06-7.64 (9H, m), 9.13 (1H, s).

Example 137

N-(4-Propylphenyl)-3-[4-(2-pyrrolidinylethoxy)benzhydryl]indole-2-carboxamide (Compound 136)

$^1$H-NMR($CDCl_3$) δ(ppm): 0.92 (3H, t, J=7.3Hz), 1.54-1.67 (2H, m), 1.75-1.90 (4H, br), 2.54 (2H, t, J=7.3Hz), 2.60-2.70 (4H, br), 2.93 (2H, t, J=5.9Hz), 4.12 (2H, t, J=5.9Hz), 6.15 (1H, s), 6.74-6.93 (4H, m), 7.08-7.42 (13H, m), 7.58 (1H, s), 9.24 (1H, s).

Example 138

N-[3-(2-Oxopyrrolidinyl)propyl]-3-[4-(2-pyrrolidinylethoxy)benzhydryl]indole-2-carboxamide (Compound 137)

$^1$H-NMR($CDCl_3$) δ(ppm): 1.67-1.76 (2H, m), 1.70-1.90 (4H, m), 1.97-2.08 (2H, m), 2.42 (2H, t, J=7.9Hz), 2.55-2.75 (4H, br), 2.91 (2H, t, J=5.9Hz), 3.30-3.39 (6H, m), 4.10 (2H, t, J=5.9Hz), 6.48 (1H, s), 6.80-6.93 (4H, m), 7.12-7.39 (9H, m), 9.62 (1H, s).

Example 139

Ethyl 3-[4-(2-dimethylaminoethoxy)benzhydryl]-1-(2-dimethylaminoethyl)indole-2-carboxylate (Compound 138)

Substantially the same procedure as in Example 73 was repeated using ethyl 3-[4-(2-chloroethoxy)-benzhydryl]-1-(2-dimethylaminoethyl)indole-2-carboxylate (26.49 g, 52.5 mmol) obtained in Reference Example 24 and an aqueous solution of dimethylamine (50%, 39.4 ml, 787 mmol) to give 24.3 g (yield: 90%) of the title compound.

$^1$H-NMR($CDCl_3$) δ(ppm): 1.36 (3H, t, J=7.3Hz), 2.32 (6H, s), 2.34 (6H, s), 2.65-2.73 (4H, m), 4.03 (2H, t, J=5.9Hz), 4.34 (2H, q, J=7.3Hz), 4.60 (2H, t, J=7.6Hz), 6.50 (1H, s), 6.79-6.95 (4H, m), 7.05-7.40 (9H, m).

Example 140

3-[4-(2-Dimethylaminoethoxy)benzhydryl]-1-(2-dimethylaminoethyl)indole-2-carboxylic acid (Compound 139)

Substantially the same procedure as in Example 6 was repeated using Compound 138 (23.8 g) obtained in Example 139 to give 21.7 g (yield: 96%) of the title compound.

$^1$H-NMR(DMSO-$d_6$) δ(ppm): 2.17 (6H, s), 2.19 (6H, s), 2.45-2.60 (4H, m), 3.95 (2H, t, J=5.9Hz), 4.63 (2H, t, J=7.3Hz), 6.69-6.78 (3H, m), 6.96-7.33 (11H, m).

In the following Examples 141 to 145, substantially the same procedure as in Example 7, and then, if necessary, substantially the same procedure as in Example 48 were repeated using Compound 139 in place of Compound 6 and using corresponding amines in place of propylamine to give the desired compounds.

Example 141

N-Isopropyl-3-[4-(2-dimethylaminoethoxy)benzhydryl]-1-(2-dimethylaminoethyl)indole-2-carboxamide (Compound 140)

$^1$H-NMR($CDCl_3$) δ(ppm): 1.10 (6H, d, J=6.6Hz), 2.21 (6H, s), 2.33 (6H, s), 2.69-2.76 (4H, m), 4.04 (2H, t, J=5.6Hz), 4.15-4.27 (1H, m), 4.44 (2H, t, J=6.9Hz), 5.98 (1H, s), 6.59 (1H, d, J=7.9Hz), 6.81-6.86 (4H, m), 7.07-7.35 (9H, m).

Example 142

N-Propyl-3-[4-(2-dimethylaminoethoxy)benzhydryl]-1-(2-dimethylaminoethyl)indole-2-carboxamide (Compound 141)

$^1$H-NMR($CDCl_3$) δ(ppm): 0.85 (3H, t, J=7.3Hz), 1.41-1.55 (2H, m), 2.21 (6H, s), 2.33 (6H, s), 2.69-2.78 (4H, m), 3.31 (2H, dt, J=6.6, 6.9Hz), 4.03 (2H, t, J=5.9Hz), 4.43 (2H, t, J=6.6Hz), 6.05 (1H, s), 6.79-6.92 (4H, m), 7.08-7.34 (9H, m).

Example 143

N-Cyclooctyl-3-[4-(2-dimethylaminoethoxy)benzhydryl]-1-(2-dimethylaminoethyl)indole-2-carboxamide hydrochloride (Compound 142 hydrochloride)

$^1$H-NMR(DMSO-$d_6$) δ(ppm): 1.35-1.90 (14H, m), 2.77 (6H, s), 2.81 (6H, s), 3.30-3.55 (4H, m), 3.85-4.05 (1H, br), 4.20-4.40 (2H, br), 4.50-4.70 (2H, br), 5.84 (1H, s), 6.89-6.93 (4H, m), 7.08-7.27 (8H, m), 7.71 (1H, d, J=8.3Hz), 8.36 (1H, d, J=7.6Hz).

Example 144

N-(4-Propylphenyl)-3-[4-(2-dimethylaminoethoxy)benzhydryl]-1-(2-dimethylaminoethyl)indole-2-carboxamide (Compound 143)

$^1$H-NMR($CDCl_3$) δ(ppm): 1.36 (3H, t, J=7.3Hz), 2.15-2.25 (2H, br), 2.37 (12H, s), 2.65-2.80 (4H, br), 4.07 (2H, t, J=5.6Hz), 4.30-4.38 (2H, m), 4.63 (2H, t, J=7.9Hz), 6.50 (1H, s), 6.79-6.91 (4H, m), 7.05-7.44 (13H, m).

Example 145

N-[3-(2-Oxopyrrolidinyl)propyl]-3-[4-(2-dimethylaminoethoxy)benzhydryl]-1-(2-dimethylaminoethyl)indole-2-carboxamide (Compound 144)

$^1$H-NMR($CDCl_3$) δ(ppm): 1.67-1.77 (2H, m), 1.95-2.07 (2H, m), 2.23 (6H, s), 2.33-2.39 (2H, m), 2.37 (6H, s), 2.76 (2H, t, J=5.6Hz), 2.75-2.90 (2H, br), 3.24 (2H, t, J=6.9Hz), 3.29-3.37 (4H, m), 4.06 (2H, t, J=5.6Hz), 4.43 (2H, t, J=6.3Hz), 6.10 (1H, s), 6.80-6.94 (4H, m), 7.09-7.35 (9H, m), 7.75 (1H, s).

Example 146

Ethyl 3-[4-(2-pyrrolidinylethoxy)benzhydryl]-1-(2-pyrrolidinylethyl)indole-2-carboxylate (Compound 145)

Substantially the same procedure as in Example 73 was repeated using ethyl 3-[4-(2-chloroethoxy)-benzhydryl]-1-(2-pyrrolidinylethyl)indole-2-carboxylate (36.7 g, 69.2 mmol) obtained in Reference Example 25 and pyrrolidine (32 ml, 380 mmol) to give 30.3 g (yield: 77%) of the title compound.

$^1$H-NMR($CDCl_3$) δ(ppm): 1.36 (3H, t, J=7.3Hz), 1.70-1.90 (8H, br), 2.50-2.80 (8H, br), 2.82-2.90 (4H, m), 4.07 (2H, t, J=5.9Hz), 4.34 (2H, q, J=7.3Hz), 4.63 (2H, t, J=7.9Hz), 6.50 (1H, s), 6.79-6.95 (4H, m), 7.05-7.43 (9H, m).

Example 147

3-[4-(2-Pyrrolidinylethoxy)benzhydryl]-1-(2-pyrrolidinylethyl)indole-2-carboxylic acid (Compound 146)

Substantially the same procedure as in Example 6 was repeated using Compound 145 (29.1 g) obtained in Example 146 to give 26.7 g (yield: 96%) of the title compound.

$^1$H-NMR($CDCl_3$) δ(ppm): 1.60-1.90 (8H, br), 2.50-2.75 (8H, br), 2.85 (2H, t, J=5.9Hz), 3.10-3.25 (2H, br), 4.00 (2H, t, J=5.9Hz), 4.60-4.80 (2H, br), 6.70-6.91 (5H, m), 7.06-7.24 (9H, m).

In the following Examples 148 to 152, substantially the same procedure as in Example 7 was repeated using Compound 146 in place of Compound 6 and using corresponding amines in place of propylamine to give the desired compounds.

Example 148

N-Isopropyl-3-[4-(2-pyrrolidinylethoxy)benzhydryl]-1-(2-pyrrolidinylethyl)indole-2-carboxamide (Compound 147)

$^1$H-NMR($CDCl_3$) δ(ppm): 1.11 (6H, d, J=6.9Hz), 1.55-1.90 (10H, br), 2.25-2.55 (2H, br), 2.55-2.80 (4H, br), 2.80-3.10 (4H, br), 4.11 (2H, t, J=5.6Hz), 4.10-4.30 (1H, m), 4.40-4.60 (2H, br), 6.05 (1H, s), 6.80-6.91 (4H, m), 7.09-7.36 (9H, m).

Example 149

N-Propyl-3-[4-(2-pyrrolidinylethoxy)benzhydryl]-1-(2-pyrrolidinylethyl)indole-2-carboxamide (Compound 148)

$^1$H-NMR($CDCl_3$) δ(ppm): 0.87 (3H, t, J=7.3Hz), 1.40-1.55 (2H, m), 1.65-1.95 (8H, br), 2.35-2.85 (8H, br), 2.85-3.15 (4H, br), 3.20-3.40 (2H, m), 4.05-4.20 (2H, br), 4.45-4.55 (2H, br), 6.08 (1H, s), 6.79-6.92 (4H, m), 7.09-7.26 (9H, m).

Example 150

N-Cyclooctyl-3-[4-(2-pyrrolidinylethoxy)benzhydryl]-1-(2-pyrrolidinylethyl)indole-2-carboxamide (Compound 149)

$^1$H-NMR($CDCl_3$) δ(ppm): 1.35-1.90 (24H, m), 2.30-2.55 (2H, br), 2.55-2.75 (4H, br), 2.92 (2H, t, J=5.9Hz), 2.90-3.10 (2H, br), 4.10 (2H, t, J=5.9Hz), 4.05-4.20 (1H, m), 4.50 (2H, t, J=6.6Hz), 6.05 (1H, s), 6.79-6.95 (4H, m), 7.05-7.46 (9H, m).

Example 151

N-(4-Propylphenyl)-3-[4-(2-pyrrolidinylethoxy)benzhydryl)-1-(2-pyrrolidinylethyl)indole-2-carboxamide (Compound 150)

$^1$H-NMR($CDCl_3$) δ(ppm): 0.94 (3H, t, J=7.3Hz), 1.55-1.70 (6H, m), 1.75-1.90 (6H, br), 2.25-2.45 (2H, br), 2.56 (2H, t, J=7.6Hz), 2.60-2.80 (4H, br), 2.85-3.15 (4H, br), 4.11 (2H, t, J=5.9Hz), 4.50-4.60 (2H, br), 6.21 (1H, s), 6.78-7.36 (18H, m).

Example 152

N-[3-(2-Oxopyrrolidinyl)propyl]-3-[4-(2-pyrrolidinylethoxy)benzhydryl]-1-(2-pyrrolidinylethyl)indole-2-carboxamide (Compound 151)

$^1$H-NMR($CDCl_3$) δ(ppm): 1.60-1.90 (10H, m), 1.97-2.08 (2H, m), 2.30-2.50 (6H, m), 2.60-2.75 (4H, br), 2.95-3.00 (2H, br), 3.00-3.10 (2H, br), 3.24-3.32 (4H, m), 3.36 (2H, t, J=7.3Hz), 4.10 (2H, t, J=5.9Hz), 4.40-4.55 (2H, br), 6.14 (1H, s), 6.79-6.97 (4H, m), 7.10-7.67 (9H, m), 8.43 (1H, br).

Example 153

N-[3-(2-Oxopyrrolidinyl)propyl]-3-(diphenylmethyl)-1-(2-methylaminoethyl)indole-2-carboxamide (Compound 152)

Substantially the same procedure as in Example 119 was repeated using N-[3-(2-oxopyrrolidinyl)propyl]-1-[2-(N-benzyloxycarbonyl-N-methylamino)ethyl]-3-(diphenylmethyl)indole-2-carboxamide (0.35 g, 0.54 mmol) obtained in Reference Example 27 to give 0.24 g (yield: 87%) of the title compound.

$^1$H-NMR($CDCl_3$) δ(ppm): 1.6-1.75 (2H, m), 1.95-2.1 (2H, m), 2.37 (2H, t, J=7.6 Hz), 2.46 (3H, s), 3.2-3.4 (8H, m), 4.57 (2H, t, J=5.7 Hz), 6.02(1H, s), 6.9-6.95 (2H, m), 7.1-7.3 (12H, m), 7.39 (1H, d, J=8.2 Hz).

Example 154

N-[3-(2-Oxopyrrolidinyl)propyl]-1-(2-aminoethyl)-3-(diphenylmethyl)indole-2-carboxamide (Compound 153)

Hydrazine hydrate (0.3 ml) was added to a solution of N-[3-(2-oxopyrrolidinyl)propyl]-3-(diphenylmethyl)-1-(2-phthalimideethyl)indole-2-carboxamide (0.553 g, 0.89 mmol) obtained in Reference Example 30 in 20 ml of ethanol, followed by stirring at 50°C for 3 hours. The resulting precipitate was filtered off and washed with chloroform. The combined organic layer was concentrated under reduced pressure to give a crude product. The obtained crude product was purified with silica gel column chromatography (chloroform/methanol = 10/1) to give 0.405 g (yield: 92%) of the title compound.

$^1$H-NMR($CDCl_3$) δ(ppm): 1.68 (2H, t, J=6.6 Hz), 2.00 (2H, t, J=7.9 Hz), 2.35 (2H, t, J=7.9 Hz), 2.80-3.4 (10H, m), 4.35-4.5 (2H, s), 6.00 (1H, s), 6.8-6.95 (2H, m), 7.15-7.35 (13H, m).

Reference Example 1

Ethyl 3-(diphenylmethyl)indole-2-carboxylate

Boron trifluoride-ether complex (0.65 ml, 5.29 mmol) was added to a solution of ethyl indole-2-carboxylate (10.0 g, 52.9 mmol) in 100 ml of methylene chloride, and a solution of benzhydryl acetate (11.4 g, 50.2 mmol) in 50 ml of methylene chloride was dropwise added thereto, followed by stirring at room temperature for 40 minutes. A saturated aqueous solution of sodium bicarbonate was added to the reaction solution for neutralization followed by extraction with chloroform. The resulting organic layer was washed with a saturated aqueous solution of sodium bicarbonate and a saturated aqueous solution of sodium chloride, and dried over magnesium sulfate, and the solvent was distilled off under reduced pressure to give a crude product. The obtained crude product was washed with diisopropyl ether to give 15.8 g (yield: 84%) of the title compound.

$^1$H-NMR($CDCl_3$) δ(ppm): 1.38 (3H, t, J=7.2Hz), 4.38 (2H, q, J=7.2Hz), 6.69 (1H, s), 6.8-6.9 (1H, m), 6.97 (1H, d, J=7.9Hz), 7.15-7.3 (11H, m), 8.35 (1H, d, J=8.2Hz), 8.85-8.95 (1H, br).

Reference Example 2

Ethyl 3-[bis(4-fluorophenyl)methyl]indole-2-carboxylate

To a solution of 4,4'-difluorobenzhydrol (12.8 g, 58.1 mmol) and ethyl indole-2-carboxylate (10.0 g, 52.9 mmol) in 100 ml of methylene chloride was added methanesulfonic acid (3.4 ml, 52.9 mmol), followed by stirring at room temperature for one hour. Then, 4,4'-difluorobenzhydrol (1.0 g, 4.54 mmol) was further added to the reaction mixture followed by stirring for 1.5 hours to complete the reaction. A 2N aqueous sodium hydroxide solution was added to the reaction solution, and a saturated aqueous solution of ammmonium chloride was further added thereto for neutralization followed by extraction with chloroform. The resulting organic layer was washed with a saturated aqueous solution of sodium bicarbonate and a saturated aqueous solution of sodium chloride, and dried over magnesium sulfate, and the solvent was distilled off under reduced pressure to give a crude product. The obtained crude product was recrystallized from ethanol to give 8.4 g (yield: 40%) of the title compound.

$^1$H-NMR($CDCl_3$) δ(ppm): 1.39 (3H, t, J=7.2Hz), 4.39 (2H, q, J=7.2Hz), 6.63 (1H, s), 6.85-7.0 (6H, m), 7.1-7.2 (4H, m), 7.2-7.3 (1H, m), 7.38 (1H, d, J=8.3Hz), 8.87 (1H, br s).

Reference Example 3

Ethyl 1-(3-chloropropyl)-3-(diphenylmethyl)indole-2-carboxylate

To a solution of ethyl 3-(diphenylmethyl)indole-2-carboxylate (38.0 g, 107 mmol) obtained in Reference Example 1 in 380 ml of N,N-dimethylformamide was added sodium hydride (60% in oil, 4.7 g, 118 mmol) under ice cooling, and 1-bromo-3-chloropropane (11.6 ml, 118 mmol) was added thereto, followed by stirring at room temperature for 21 hours. A saturated aqueous solution of ammonium chloride and water were added to the reaction solution followed by extraction with ethyl acetate. The resulting organic layer was washed with water and a saturated aqueous solution of sodium chloride and dried over magnesium sulfate, and the solvent was distilled off under reduced pressure to give 51.1 g of a crude product. The obtained crude product was recrystallized from ethanol to give 40.6 g (yield: 88%) of the title compound.

$^1$H-NMR($CDCl_3$) δ(ppm): 1.36 (3H, t, J=7.3Hz), 2.26-2.36 (2H, m), 3.57 (2H, t, J=6.3Hz), 4.34 (2H, q, J=7.3Hz), 4.66 (2H, t, J=7.3Hz), 6.58 (1H, s), 6.84-6.95 (2H, m), 7.15-7.30 (11H, m), 7.45 (1H, d, J=8.6Hz).

Reference Example 4

Ethyl 1-(4-chlorobutyl)-3-(diphenylmethyl)indole-2-carboxylate

Substantially the same procedure as in Reference Example 3 was repeated using ethyl 3-(diphenylmethyl)indole-2-carboxylate (75.76 g, 213 mmol) obtained in Reference Example 1 and 1-bromo-4-chlorobutane (27 ml, 234 mmol) to give 82.0 g (yield: 84%) of the title compound.

$^1$H-NMR($CDCl_3$) δ(ppm): 1.36 (3H, t, J=6.9Hz), 1.78-1.88 (2H, m), 1.93-2.05 (2H, m), 3.55 (2H, t, J=6.6Hz), 4.34 (2H, q, J=6.9Hz), 4.52 (2H, t, J=7.3Hz), 6.58 (1H, s), 6.83-6.94 (2H, m), 7.16-7.38 (12H, m).

Reference Example 5

Ethyl 3-[bis (4-chlorophenyl)methyl]indole-2-carboxylate

Substantially the same procedure as in Reference Example 2 was repeated using 4,4'-dichlorobenzhydrol (14.7 g, 58.1 mmol) and ethyl indole-2-carboxylate (10.0 g, 52.9 mmol) to give 21.0 g (yield: 94%) of the title compound.

$^1$H-NMR($CDCl_3$) δ(ppm): 1.39 (3H, t, J=7.3Hz), 4.39 (2H, q, J=7.3Hz), 6.62 (1H, s), 6.9-6.95 (2H, m), 7.13 (4H, d, J=8.6Hz), 7.2-7.35 (5H, m), 7.39 (1H, d, J=8.3Hz), 8.87 (1H, s).

Reference Example 6

Ethyl 3-[bis(4-methylphenyl)methyl]indole-2-carboxylate

Substantially the same procedure as in Reference Example 1 was repeated using 4,4'-dimethylbenzhydrol (11.8 g, 55.5 mmol) and ethyl indole-2-carboxylate (10.0 g, 52.9 mmol) to give 19.8 g (yield: 97%) of the title compound.

$^1$H-NMR($CDCl_3$) δ(ppm): 1.39 (3H, t, J=7.1Hz), 2.31 (6H, s), 4.38 (2H, q, J=7.1Hz), 6.60 (1H, s), 6.85-6.95 (1H, m), 7.0-7.15 (9H, m), 7.2-7.3 (1H, m), 7.35 (1H, d, J=8.6Hz), 8.82 (1H, s).

Reference Example 7

Ethyl 3-(4-benzyloxybenzhydryl)indole-2-carboxylate

Substantially the same procedure as in Reference Example 1 was repeated using 4-benzyloxybenzhydrol (30.0 g, 103 mmol) and ethyl indole-2-carboxylate (18.5 g, 97.9 mmol) to give 42.7 g (yield: 95%) of the title compound.

$^1$H-NMR($CDCl_3$) δ(ppm): 1.49 (3H, t, J=7.2Hz), 4.48 (2H, q, J=7.2Hz), 5.93 (2H, s), 6.72 (1H, s), 6.95-7.05 (3H, m), 7.08 (1H, d, J=7.9Hz), 7.23 (2H, d, J=8.6Hz), 7.25-7.55 (12H, m), 8.94 (1H, s).

Reference Example 8

3-(4-Benzyloxybenzhydryl)-1-(2-dimethylaminoethyl)indole-2-carboxylic acid

Substantially the same procedure as in Example 2 was repeated using ethyl 3-(4-benzyloxybenzhydryl)indole-2-carboxylate (20.0 g, 43.3 mmol) obtained in Reference Example 7 and 2-dimethylaminoethylchloride hydrochloride (6.55 g, 45.5 mmol) to give 26.3 g of ethyl 3-(4-benzyloxybenzhydryl)-1-(2-dimethylaminoethyl)indole-2-carboxylate. Then, substantially the same procedure as in Example 6 was repeated using this compound (26.3 g) to give 19.8 g (yield: 91%, 2 steps) of the title compound.

$^1$H-NMR($CDCl_3$) δ(ppm): 2.41 (6H, s), 3.18 (2H, t, J=6.1Hz), 4.77 (2H, t, J=6.1Hz), 5.00 (2H, s), 6.78 (1H, s), 6.84 (2H, d, J=8.9Hz), 6.85-6.95 (1H, m), 7.1-7.45 (15H, m).

Reference Example 9

3-(4-Benzyloxybenzhydryl)-1-(2-pyrrolidinylethyl)indole-2-carboxylic acid

Substantially the same procedure as in Example 2 was repeated using ethyl 3-(4-benzyloxybenzhydryl)indole-2-carboxylate (20.0 g, 43.33 mmol) obtained in Reference Example 7 and 2-pyrrolidinylethylchloride hydrochloride (8.1 g, 47.66 mmol) to give 24.9 g of ethyl 3-(4-benzyloxybenzhydryl)-1-(2-pyrrolidinylethyl)indole-2-carboxylate. Then, substantially the same procedure as in Example 6 was repeated using this compound (24.9 g) to give 21.2 g (yield: 92%, 2 steps) of the title compound.

$^1$H-NMR($CDCl_3$) δ(ppm): 1.65-1.85 (4H, m), 2.65-2.95 (4H, m), 3.35 (2H, t, J=6.1Hz), 4.75-4.9 (2H, m), 5.00 (2H, s), 6.76 (1H, s), 6.83 (2H, d, J=8.9Hz), 6.85-6.95 (1H, m), 7.05-7.45 (15H, m).

In the following Reference Examples 10 to 15, substantially the same procedure as in Example 7 was repeated using 3-(4-benzyloxybenzhydryl)-1-(2-dimethylaminoethyl)indole-2-carboxylic acid obtained in Reference Example 8 in place of Compound 6 and using corresponding amines in place of propylamine to give the desired compounds.

Reference Example 10

N-Propyl-3-(4-benzyloxybenzhydryl)-1-(2-dimethylaminoethyl)indole-2-carboxamide

$^1$H-NMR($CDCl_3$) δ(ppm): 0.85 (3H, t, J=7.4Hz), 1.4-1.55 (2H, m), 2.22 (6H, s), 2.77 (2H, t, J=6.6Hz), 3.31 (2H,

q, J=6.6Hz), 4.43 (2H, t, J=6.6Hz), 5.03 (2H, s), 6.05 (1H, s), 6.85-6.95 (4H, m), 7.0-7.1 (1H, m), 7.11 (2H, d, J=8.6Hz), 7.15-7.45 (12H, m).

Reference Example 11

N-Isopropyl-3-(4-benzyloxybenzhydryl)-1-(2-dimethylaminoethyl)indole-2-carboxamide

$^1$H-NMR($CDCl_3$) δ(ppm): 1.10 (6H, d, J=6.3Hz), 2.20 (6H, s), 2.74 (2H, t, J=6.9Hz), 4.15-4.3 (1H, m), 4.44 (2H, t, J=6.9Hz), 5.04 (2H, s), 5.99 (1H, s), 6.63 (1H, d, J=7.6Hz), 6.8-6.95 (4H, m), 7.10 (2H, d, J=8.6Hz), 7.15-7.45 (12H, m).

Reference Example 12

N-(4-propylphenyl)-3-(4-benzyloxybenzhydryl)-1-(2-dimethylaminoethyl)indole-2-carboxamide

$^1$H-NMR($CDCl_3$) δ(ppm): 0.94 (3H, t, J=7.3Hz), 1.55-1.7 (2H, m), 2.17 (6H, s), 2.5-2.6 (2H, m), 2.81 (2H, t, J=6.3Hz), 4.48 (2H, t, J=6.3Hz), 5.03 (2H, s), 6.17 (1H, s), 6.8-6.95 (3H, m), 7.00 (1H, d, J=8.3Hz), 7.1-7.45 (18H, m), 9.32 (1H, s).

Reference Example 13

N-(3-Morpholinopropyl)-3-(4-benzyloxybenzhydryl)-1-(2-dimethylaminoethyl)indole-2-carboxamide

$^1$H-NMR($CDCl_3$) δ(ppm): 1.6-1.75 (2H, m), 2.2-2.4 (6H, m), 2.22 (6H, s), 2.75 (2H, t, J=6.6Hz), 3.35-3.45 (2H, m), 3.6-3.7 (4H, m), 4.40 (2H, t, J=6.6Hz), 5.02 (2H, s), 6.05 (1H, s), 6.87 (2H, d, J=8.6Hz), 6.9-7.0 (2H, m), 7.12 (2H, d, J=8.6Hz), 7.15-7.5 (13H, m).

Reference Example 14

N-[3-(2-Oxopyrrolidinyl)propyl]-3-(4-benzyloxybenzhydryl)-1-(2-dimethylaminoethyl)indole-2-carboxamide

$^1$H-NMR($CDCl_3$) δ(ppm): 1.65-1.8 (2H, m), 1.9-2.1 (2H, m), 2.19 (6H, s), 2.35 (2H, t, J=8.1Hz), 2.79 (2H, t, J=6.4Hz), 3.24 (2H, t, J=7.1Hz), 3.3-3.4 (4H, m), 4.41 (2H, t, J=6.4Hz), 5.02 (2H, s), 6.12 (1H, s), 6.87 (2H, d, J=8.6Hz), 6.9-7.0 (1H, m), 7.13 (2H, d, J=8.6Hz), 7.2-7.5 (13H, m), 7.8-7.9 (1H, m).

Reference Example 15

N-Cyclooctyl-3-(4-benzyloxybenzhydryl)-1-(2-dimethylaminoethyl)indole-2-carboxamide

$^1$H-NMR($CDCl_3$) δ(ppm): 1.3-1.6 (12H, m), 1.65-1.85 (2H, m), 2.20 (6H, s), 2.73 (2H, t, J=6.9Hz), 4.05-4.2 (1H, m), 4.44 (2H, t, J=6.9Hz), 5.03 (2H, s), 6.00 (1H, s), 6.63 (1H, d, J=8.3Hz), 6.8-6.9 (4H, m), 7.10 (2H, d, J=8.5Hz), 7.15-7.45 (12H, m).

In the following Reference Examples 16 to 19, substantially the same procedure as in Example 7 was repeated using 3-(4-benzyloxybenzhydryl)-1-(2-pyrrolidinylethyl)indole-2-carboxylic acid obtained in Reference Example 9 in place of Compound 6 and using corresponding amines in place of propylamine to give the desired compounds.

Reference Example 16

N-Propyl-3-(4-benzyloxybenzhydryl)-1-(2-pyrrolidinylethyl)indole-2-carboxamide

$^1$H-NMR($CDCl_3$) δ(ppm): 0.87 (3H, t, J=7.4Hz), 1.6-1.75 (2H, m), 1.8-1.95 (4H, m), 2.3-2.45 (4H, m), 2.99 (2H, t, J=6.6Hz), 3.25-3.35 (2H, m), 4.46 (2H, t, J=6.6Hz), 5.03 (2H, s), 6.12 (1H, s), 6.85-6.95 (3H, m), 6.95 (1H, d, J=7.6Hz), 7.1-7.45 (14H, m), 7.75-7.85 (1H, m).

Reference Example 17

N-Isopropyl-3-(4-benzyloxybenzhydryl)-1-(2-pyrrolidinylethyl)-indole-2-carboxamide

$^1$H-NMR($CDCl_3$) δ(ppm): 1.10 (6H, d, J=6.6Hz), 1.55-1.65 (4H, m), 2.3-2.4 (4H, m), 2.97 (2H, t, J=6.6Hz), 4.15-4.25 (1H, m), 4.47 (2H, t, J=6.6Hz), 5.03 (2H, s), 6.08 (1H, s), 6.8-6.95 (4H, m), 7.1-7.45 (14H, m), 7.45-7.6 (1H, m).

Reference Example 18

N-(3-Morpholinopropyl)-3-(4-benzyloxybenzhydryl)-1-(2-pyrrolidinylethyl)indole-2-carboxamide

$^1H$-NMR($CDCl_3$) δ(ppm): 1.6-1.75 (6H, m), 2.25-2.45 (10H, m), 2.95-3.05 (2H, m), 3.3-3.45 (2H, m), 3.55-3.65 (4H, m), 4.45 (2H, t, J=6.6Hz), 5.02 (2H, s), 6.10 (1H, s), 6.8-6.95 (3H, m), 6.97 (1H, d, J=8.0Hz), 7.13 (2H, d, J=8.6Hz), 7.15-7.45 (12H, m), 7.9-8.0 (1H, m).

Reference Example 19

N-[3-(2-Oxopyrrolidinyl)propyl]-3-(4-benzyloxybenzhydryl)-1-(2-pyrrolidinylethyl)indole-2-carboxamide

$^1H$-NMR($CDCl_3$) δ(ppm): 1.6-1.8 (6H, m), 1.95-2.2 (2H, m), 2.3-2.45 (6H, m), 3.03 (2H, t, J=6.2Hz), 3.2-3.4 (6H, m), 4.46 (2H, t, J=6.2Hz), 5.02 (2H, s), 6.17 (1H, s), 6.8-6.95 (3H, m), 6.98 (1H, d, J=7.9Hz), 7.1-7.45 (14H, m), 8.4-8.6 (1H, m).

Reference Example 20

Ethyl 3-[4-(2-dimethylaminoethoxy)benzhydryl]indole-2-carboxylate

Substantially the same procedure as in Reference Example 2 was repeated using 4-(2-dimethylaminoethoxy)benzhydrol (14.78 g, 54.5 mmol) and ethyl indole-2-carboxylate (11.3 g, 59.9 mmol) to give 20.8 g (yield: 86%) of the title compound.

$^1H$-NMR($CDCl_3$) δ(ppm): 1.38 (3H, t, J=6.9Hz), 2.32 (6H, s), 2.71 (2H, t, J=5.9Hz), 4.03 (2H, t, J=5.9Hz), 4.38 (2H, q, J=7.3Hz), 6.61 (1H, s), 6.80-6.98 (4H, m), 7.09-7.36 (9H, m), 8.95 (1H, s).

Reference Example 21

3-[4-(2-Dimethylaminoethoxy)benzhydryl]indole-2-carboxylic acid

Substantially the same procedure as in Example 6 was repeated using ethyl 3-[4-(2-dimethylaminoethoxy)benzhydryl]indole-2-carboxylate (20.8 g) obtained in Reference Example 20 to give 18.2 g (yield: 94%) of the title compound.

$^1H$-NMR(DMSO-$d_6$) δ(ppm): 2.30 (6H, s), 2.75 (2H, t, J=5.6Hz), 4.03 (2H, t, J=5.9Hz), 6.77-7.39 (14H, m), 11.45 (1H, s).

Reference Example 22

Ethyl 3-[4-(2-pyrrolidinylethoxy)benzhydryl]indole-2-carboxylate

Substantially the same procedure as in Reference Example 2 was repeated using 4-(2-pyrrolidinylethoxy)benzhydrol (19.4 g, 65.1 mmol) and ethyl indole-2-carboxylate (13.55 g, 71.6 mmol) to give 31.2 g (quantitative) of the title compound.

$^1H$-NMR($CDCl_3$) δ(ppm): 1.39 (3H, t, J=7.3Hz), 1.60-1.85 (4H, m), 2.50-2.70 (4H, m), 2.88 (2H, t, J=6.3Hz), 4.08 (2H, t, J=6.3Hz), 4.38 (2H, q, J=6.9Hz), 6.61 (1H, s), 6.79-6.99 (4H, m), 7.10-7.40 (9H, m), 8.83 (1H, s).

Reference Example 23

3-[4-(2-Pyrrolidinylethoxy)benzhydryl]indole-2-carboxylic acid

Substantially the same procedure as in Example 6 was repeated using ethyl 3-[4-(2-pyrrolidinylethoxy)benzhydryl]indole-2-carboxylate (29.17 g, 62.2 mmol) obtained in Reference Example 22 to give 24.8 g (yield: 90%) of the title compound.

$^1H$-NMR(DMSO-$d_6$) δ(ppm): 1.60-1.80 (4H, br), 2.65-2.80 (4H, br), 2.97 (2H, t, J=5.6Hz), 4.08 (2H, t, J=5.9Hz), 6.72-7.37 (14H, m), 11.35 (1H, s).

Reference Example 24

Ethyl 3-[4-(2-chloroethoxy)benzhydryl]-1-(2-dimethylaminoethyl)indole-2-carboxylate

Substantially the same procedure as in Reference Example 2 was repeated using 4-(2-chloroethoxy)benzhydrol (17.66 g, 67.2 mmol) and ethyl 1-(2-dimethylaminoethyl)indole-2-carboxylate (19.2 g, 73.9 mmol) to give 27.6 g (yield: 81%) of the title compound.

$^1$H-NMR($CDCl_3$) δ(ppm): 1.36 (3H, t, J=7.3Hz), 2.34 (6H, s), 2.68 (2H, t, J=7.6Hz), 3.79 (2H, t, J=5.9Hz), 4.20 (2H, t, J=5.9Hz), 4.35 (2H, q, J=7.3Hz), 4.60 (2H, t, J=7.6Hz), 6.51 (1H, s), 6.79-6.95 (4H, m), 7.08-7.42 (9H, m).

Reference Example 25

3-[4-(2-Chloroethoxy)benzhydryl]-1-(2-pyrrolidinylethyl)indole-2-carboxylate

Substantially the same procedure as in Reference Example 2 was repeated using 4-(2-chloroethoxy)benzhydrol (17.6 g, 67 mmol) and ethyl 1-(2-pyrrolidinylethyl)indole-2-carboxylate (21.1 g, 73.6 mmol) to give 37.4 g (quantitative) of the title compound.

$^1$H-NMR($CDCl_3$) δ(ppm): 1.36 (3H, t, J=7.3Hz), 1.75-1.90 (4H, br), 2.55-2.75 (4H, br), 2.87 (2H, t, J=7.9Hz), 3.79 (2H, t, J=5.9Hz), 4.20 (2H, t, J=5.9Hz), 4.34 (2H, q, J=7.3Hz), 4.65 (2H, t, J=7.9Hz), 6.51 (1H, s), 6.79-6.95 (4H, m), 7.08-7.45 (9H, m).

Reference Example 26

1-[2-(N-Benzyloxycarbonyl-N-methylamino)ethyl]-3-(diphenylmethyl)indole-2-carboxylic acid

Substantially the same procedure as in Example 2 was repeated using ethyl 3-(diphenylmethyl)indole-2-carboxylate (1.23 g, 3.47 mmol) obtained in Reference Example 1 and 2-(N-benzyloxycarbonyl-N-methylamino)ethylchloride (0.79 g, 3.47 mmol) to give 0.93 g (yield: 49%) of ethyl 1-[2-(N-benzyloxycarbonyl-N-methylamino)ethyl]-3-(diphenylmethyl)-indole-2-carboxylate. Then, to a solution of this compound (0.93 g, 1.70 mmol) in a mixed solvent of ethanol (12 ml) and water (2 ml) was added lithium hydroxide (0.36 g, 8.51 mmol), followed by heating under reflux for 3 hours. The solvent was distilled off under reduced pressure and a saturated aqueous solution of ammmonium chloride was added thereto for neutralization, followed by extraction with ethyl acetate. The resulting organic layer was washed with a saturated aqueous solution of ammmonium chloride and a saturated aqueous solution of sodium chloride and dried over magnesium sulfate, and the solvent was distilled off under reduced pressure to give a crude product. The obtained crude product was crystallized from ethanol to give 0.65 g (yield: 74%) of the title compound.

$^1$H-NMR($CDCl_3$) δ(ppm): 2.61, 2.81 (3H, each s), 3.55-3.75 (2H, m), 4.55-4.65, 4.65-4.75 (2H, each m), 5.11 (2H, s), 6.69, 6.72 (1H, each s), 6.75-6.95 (2H, m), 7.02 (1H, br s), 7.1-7.4 (15H, m), 7.48 (1H, d, J=8.6 Hz).

Reference Example 27

N-[3-(2-Oxopyrrolidinyl)propyl]-1-[2-(N-benzyloxycarbonyl-N-methylamino)ethyl]-3-(diphenylmethyl)indole-2-carboxamide

Substantially the same procedure as in Example 7 was repeated using 1-[2-(N-benzyloxycarbonyl-N-methylamino)ethyl]-3-(diphenylmethyl)indole-2-carboxylic acid (0.57 g, 1.10 mmol) obtained in Reference Example 26 and 1-(3-aminopropyl)-2-pyrrolidinone (0.23 ml, 1.65 mmol) to give 0.46 g (yield: 65%) of the title compound.

$^1$H-NMR($CDCl_3$) δ(ppm): 1.6-1.75 (2H, m), 1.95-2.1 (2H, m), 2.35 (2H, t, J=7.9 Hz), 2.64, 2.75 (3H, each s), 3.15-3.4 (6H, m), 3.6-3.75 (2H, m), 4.4-4.55 (2H, m), 4.98, 5.05 (2H, each s), 5.94, 5.97 (1H, each s), 6.05-6.15, 6.15-6.25 (1H, each m), 6.8-6.9 (2H, m), 7.0-7.5(17H, m).

Reference Example 28

5-Diphenylmethyl-1,2-dihydro-4H-indolo[2,1-c][1,4]oxazin-4-one

Substantially the same procedure as in Example 2 was repeated using ethyl 3-(diphenylmethyl)indole-2-carboxylate (2.0 g, 5.6 mmol) obtained in Reference Example 1 and 2-bromoethyl acetate (0.8 ml, 7.2 mmol) to give 2.4 g (yield: 97%) of ethyl 1-(2-acetoxyethyl)-3-(diphenylmethyl)indole-2-carboxylate. Then, to a suspension of this compound (1.87 g, 4.25 mmol) in 15 ml of methanol was added sodium methoxide (0.98 ml, 5.10 mmol), followed by stirring at room temperature for 2 hours. A saturated aqueous solution of ammmonium chloride was added thereto for neutralization followed by extraction with chloroform. The resulting organic layer was washed with a saturated aqueous solution of sodium chloride and dried over magnesium sulfate, and the solvent was distilled off under reduced pressure to give 1.55 g (quantitative) of the title compound.

$^1$H-NMR($CDCl_3$) δ(ppm): 4.32 (2H, t, J=5.3 Hz), 4.72 (2H, t, J=5.3 Hz), 6.85 (1H, s), 6.93-6.99 (1H, m), 7.11 (1H, d, J=8.3 Hz), 7.19-7.37 (12H, m).

Reference Example 29

N-[3-(2-Oxopyrrolidinyl)propyl]-3-(diphenylmethyl)-1-(2-hydroxyethyl)indole-2-carboxamide

5-Diphenylmethyl-1,2-dihydro-4H-indolo[2,1-c][1,4]oxazin-4-one (1.12 g, 3.2 mmol) obtained in Reference Example 28 and 1-(3-aminopropyl)-2-pyrrolidinone (0.9 ml, 6.4 mmol) were mixed and the mixture was stirred at 100°C for 6 hours. Water was added to the reaction solution followed by extraction with chloroform. The resulting organic layer was washed with a saturated aqueous solution of sodium chloride and the solvent was distilled off under reduced pressure to give a crude product. The obtained crude product was purified with silica gel column chromatography (chloroform/methanol = 20/1) to give 1.12 g (yield: 71%) of the title compound.

$^1$H-NMR($CDCl_3$) δ(ppm): 1.71 (2H, q, J=6.9 Hz), 2.08 (2H, q, J=7.3 Hz), 2.39 (2H, t, J=7.9 Hz), 3.20-3.43 (6H, m), 4.06 (2H, t, J=5.3 Hz), 4.44 (2H, t, J=5.0 Hz), 6.08 (1H, s), 6.82-7.00 (3H, m), 7.11-7.38 (12H, m).

Reference Example 30

N-[3-(2-Oxopyrrolidinyl)propyl]-3-(diphenylmethyl)-1-(2-phthalimideethyl)indole-2-carboxamide

To a solution of N-[3-(2-oxopyrrolidinyl)propyl]-3-(diphenylmethyl)-1-(2-hydroxyethyl)indole-2-carboxamide (1.0 g, 2.02 mmol), triphenylphosphine (0.795 g, 3.03 mmol), and phthalimide (0.446 g, 3.03 mmol) in 30 ml of tetrahydrofuran was dropwise added diethyl azodicarboxylate (0.48 ml, 3.03 mmol), followed by stirring at room temperature for 2 hours and then being allowed to stand overnight. Water was added to the reaction mixture followed by extraction with ethyl acetate. The resulting organic layer was washed with a saturated aqueous solution of sodium chloride and dried over magnesium sulfate, and the solvent was distilled off under reduced pressure to give a crude product. The obtained crude product was purified with silica gel column chromatography (ethyl acetate) to give 0.70 g (yield: 56%) of the title compound.

$^1$H-NMR($CDCl_3$) δ(ppm): 1.56-1.7 (2H, m), 1.90-2.07 (2H, m), 2.37 (2H, t, J=7.9 Hz), 3.12-3.27 (4H, m), 3.35 (2H, t, J=6.9 Hz), 4.05 (2H, t, J=5.9 Hz), 4.68 (2H, t, J=5.9 Hz), 5.93 (1H, s), 6.10(1H, t, J=5.9 Hz), 6.62-6.78(2H, m), 6.96 (1H, dt, J=1.3, 6.6 Hz), 7.10-7.32 (11H, m), 7.55-7.65 (4H, m).

Industrial Applicability

According to the present invention, there can be provided indole derivatives which are useful as therapeutic agents for osteoporosis.

## Claims

1. An indole derivative represented by formula (I):

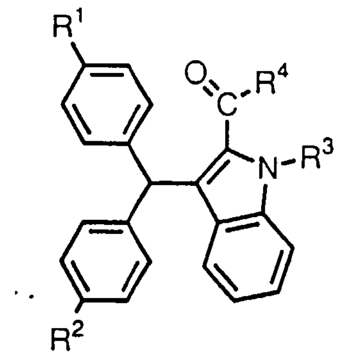

(I)

wherein $R^1$ represents hydrogen, lower alkyl, hydroxy, lower alkoxy, halogen, $-O-(CH_2)_n-OR^5$ (wherein $R^5$ represents hydrogen or lower alkyl, and n is an integer of 1 to 6), or

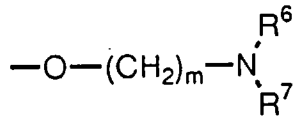

(wherein $R^6$ and $R^7$ independently represent hydrogen or lower alkyl, or $R^6$ and $R^7$ are combined together with the adjacent nitrogen atom to form a substituted or unsubstituted alicyclic heterocyclic group, and m represents an integer of 2 to 6), $R^2$ represents hydrogen, lower alkyl, or halogen, $R^3$ represents hydrogen, lower alkyl, or

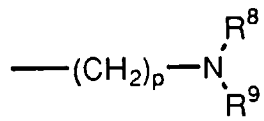

(wherein $R^8$ and $R^9$ independently represent hydrogen or lower alkyl, or $R^8$ and $R^9$ are combined together with the adjacent nitrogen atom to form a substituted or unsubstituted alicyclic heterocyclic group, and p represents an integer of 2 to 6), and $R^4$ represents hydroxy, lower alkoxy, substituted or unsubstituted aryloxy, or $-NR^{10}R^{11}$ {wherein $R^{10}$ and $R^{11}$ independently represent hydrogen, lower alkyl, alicyclic alkyl, lower alkenyl, substituted or unsubstituted aryl, a substituted or unsubstituted heterocyclic group,

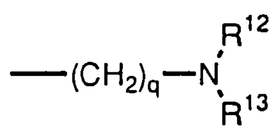

(wherein $R^{12}$ and $R^{13}$ independently represent hydrogen or lower alkyl, or $R^{12}$ and $R^{13}$ are combined together with the adjacent nitrogen atom to form a substituted or unsubstituted alicyclic heterocyclic group, and q represents an integer of 2 to 6), or $-(CH_2)_r-R^{14}$ (wherein $R^{14}$ represents substituted or unsubstituted aryl or a substituted or unsubstituted heterocyclic group, and r represents an integer of 1 to 6), or $R^{10}$ and $R^{11}$ are combined together with the adjacent nitrogen atom to form a substituted or unsubstituted alicyclic heterocyclic group), with the proviso that when $R^3$ is hydrogen or lower alkyl, $R^4$ is $-NR^{10}R^{11}$, or a pharmaceutically acceptable salt thereof.

**2.** An indole derivative according to claim 1, wherein $R^1$ and $R^2$ represent hydrogen, or a pharmaceutically acceptable salt thereof.

**3.** An indole derivative according to claim 2, wherein $R^3$ represents

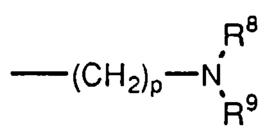

(wherein $R^8$, $R^9$, and p have the same meanings as defined above), or a pharmaceutically acceptable salt thereof.

**4.** A pharmaceutically acceptable composition comprising a pharmaceutically acceptable carrier and an effective amount of a compound according to any one of claims 1 to 3 as an active ingredient.

**INTERNATIONAL SEARCH REPORT**

International application No.
PCT/JP96/01980

**A. CLASSIFICATION OF SUBJECT MATTER**

Int. $Cl^6$ C07D209/42, 401/06, 403/06, 405/06, 409/06, A61K31/40, 31/41, 31/44, 31/445, 31/495, 31/535

According to International Patent Classification (IPC) or to both national classification and IPC

**B. FIELDS SEARCHED**

Minimum documentation searched (classification system followed by classification symbols)

Int. $Cl^6$ C07D209/42, 401/06, 403/06, 405/06, 409/06, A61K31/40, 31/41, 31/44, 31/445, 31/495, 31/535

Documentation searched other than minimum documentation to the extent that such documents are included in the fields searched

Electronic data base consulted during the international search (name of data base and, where practicable, search terms used)

CAS ONLINE

**C. DOCUMENTS CONSIDERED TO BE RELEVANT**

| Category* | Citation of document, with indication, where appropriate, of the relevant passages | Relevant to claim No. |
|---|---|---|
| P,X<br>P,A | WO, 95/19343, A1 (Kyowa Hakko Co., Ltd.), July 20, 1995 (20. 07. 95), Full descriptions & AU, 9514247, A | 1, 4<br>2, 3 |
| X<br>A | J. Med. Chem., 17(12), (1974), p. 1298-1304, Calvert W. Whitehead et al., "Effect of lipophilic substituents on some biological properties of indoles" | 1, 2, 4<br>3 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| October 2, 1996 (02. 10. 96) | October 15, 1996 (15. 10. 96) |
| Name and mailing address of the ISA/<br>Japanese Patent Office<br>Facsimile No. | Authorized officer<br><br>Telephone No. |

Form PCT/ISA/210 (second sheet) (July 1992)